# EUROPEAN PATENT APPLICATION

(11) **EP 4 190 789 A1**
(43) Date of publication of application: **07.06.2023**
(21) Application number: 21851201.0
(22) Date of filing: 29.07.2021
(51) Int. Cl.: C07D 495/04, A61K 31/4162, A61P 35/00

(54) **OXA-AZASPIRO DERIVATIVE, AND PREPARATION METHOD THEREFOR AND PHARMACEUTICAL USE THEREOF**

(30) Priority: 29.07.2020 CN 202010746651; 11.09.2020 CN 202010954059; 24.02.2021 CN 202110210203
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LU, Biao, Shanghai 200245 (CN); ZHANG, Caihua, Shanghai 200245 (CN); HE, Feng, Shanghai 200245 (CN); TAO, Weikang, Shanghai 200245 (CN)
(74) Representative: Potter Clarkson
(86) International application number: PCT/CN2021/109205
(87) International publication number: WO 2022/022630

(57) **Abstract**

Disclosed are an oxa-azasipro derivative, a preparation method therefor and a pharmaceutical use thereof. In particular, disclosed are an oxa-azasipro derivative as represented by general formula (I), a preparation method therefor, a pharmaceutical composition containing the derivative, and the use thereof as a therapeutic agent, especially the use thereof as a PI3Kδ inhibitor and the use thereof in the preparation of a drug for treating diseases or conditions improved by means of inhibiting PI3Kδ.

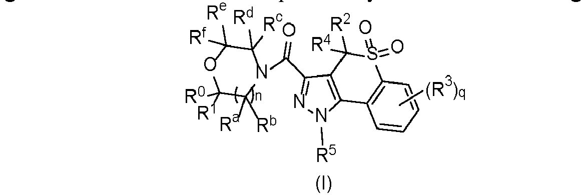

## Description

### TECHNICAL FIELD

The present disclosure belongs to the field of pharmaceutics, and relates to an oxa-azasipro derivative of general formula (I), a preparation method therefor, a pharmaceutical composition containing the derivative, and use thereof as a therapeutic agent, in particular use thereof as a PI3Kδ inhibitor and use thereof in preparing a medicament for the treatment of a disease or condition improved by inhibiting PI3Kδ.

### BACKGROUND

Phosphoinositide 3-kinase (PI3K) is a key regulatory kinase in the PI3K/AKT/mTOR signaling pathway and is involved in regulating cell proliferation, cell differentiation, apoptosis, angiogenesis and the like. Abnormal activation of PI3K is closely related to the development of various tumors, and different types of PI3Ks play different functions. PI3K has four subtypes: α, β, γ and δ, where PI3Kδ is mainly present in immune cells and blood cells and is closely related to the occurrence of immunity, hematological tumors and inflammation (Cell, 170(4), 605-635).

PI3Kδ is mainly expressed in immune cells and hematopoietic cells, and it is involved in BCR signaling in B cells and controls the development and maturation of B cells in the body. When an antigen stimulates the body, specific surface immunoglobulin Ig on the surface of the BCR can bind to the antigen, so that ITAM in an intracellular segment of a CD79A/B complex is phosphorylated, and the phosphorylated ITAM can recruit and activate SYK and further activate BTK and a downstream molecule PLCγ2 thereof. Activated SYK can bind to the P85 subunit of PI3Kδ to activate PI3Kδ, thus generating PIP3. The generated PIP3 can be recognized by the N-terminal domain of BTK and interacts with the N-terminal domain to mediate BTK recruitment to the membrane, so that BTK-mediated B cell signaling is activated, and thereby the expression of a plurality of related genes is induced. In addition, phosphorylated CD19 can also recruit PI3Kδ on cell membrane, activate PI3Kδ, catalyze PIP2 to generate PIP3, activate AKT, and promote cell proliferation, migration, apoptosis and other processes (N Engl J Med, 379, 2052-2062). In addition to regulating B cells, a recent study reported that PI3Kδ activation can promote the development, maturation and recruitment of Treg cells (Cancer Immunol Res, 2, 1080-1089). Inhibition of PI3Kδ can promote the proliferation and survival of CD8+ memory T cells (Cancer Res, 77, 4135-4145). Therefore, PI3Kδ is an ideal target for treating B cell lymphoma, and the development of a selective PI3Kδ inhibitor as a medicament for treating hematologic tumors is drawing more and more attention.

Idelalisib is the first approved PI3Kδ selective inhibitor, and it was approved for the treatment of chronic lymphocytic leukemia (CLL), follicular lymphoma (FL) and small lymphocytic lymphoma (SLL) in 2014. Duvelisib (acting on PI3K δ and γ) was subsequently approved for the treatment of chronic lymphocytic leukemia (CLL) and follicular lymphoma (FL) in 2018. Although the PI3Kδ inhibitors have shown very good results in treating these hematologic tumors, theses early inhibitors often show poor selectivity for PI3K kinase and thus clinically show much drug-related hepatotoxicity and gastrointestinal toxicity. To further reduce the potential side effects of PI3Kδ inhibitors, many companies have been actively developing the second-generation highly selective PI3Kδ inhibitors in recent years, typically parsaclisib, ME-401 and IOA-244, which are currently in different clinical stages. IOA-244 is a second-generation PI3Kδ inhibitor developed by the iOnctura (WO2011058149 and WO2014121901). Compared with traditional PI3Kδ inhibitors, it is an ATP non-competitive inhibitor, and this characteristic allows it to have high selectivity for inhibiting the PI3Kδ subtype.

Considering that the use of the first-generation PI3Kδ inhibitors currently on the market is limited in more patient populations due to their obvious side effects, there is a significant unmet medical need to develop a second-generation highly selective PI3Kδ inhibitor for the relevant patient populations.

Currently, relevant patent applications include WO2011058149A1, WO2015196759A1, WO2015196335A1, WO2014209980A1, WO2004069824A1 and the like.

### SUMMARY

The present disclosure is intended to provide a compound of general formula (I) or a tautomer, racemate, enantiomer or diastereomer thereof or a mixture thereof, or a pharmaceutically acceptable salt thereof: 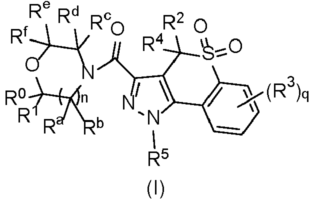 wherein:
R⁰, R¹, R^{a}, R^{b}, R^{c}, R^{d}, R^{e} and R^{f} are identical or different and are each independently selected from the group consisting of a hydrogen atom, alkyl, halogen, alkoxy, haloalkoxy, cyano, hydroxy, hydroxyalkyl, -(CH₂)ₛNR⁷R⁸, cycloalkyl, cycloalkylalkyl, cycloalkyloxy, heterocyclyl, heterocyclylalkyl, heterocyclyloxy, aryl and heteroaryl,
wherein the alkyl, cycloalkyl, heterocyclyl, aryl and heteroaryl are each independently optionally substituted with one or more substituents selected from the group consisting of halogen, alkyl, haloalkyl, cyano, nitro, -(CH₂)_{y}NR^{g}R^{h} and -OR⁹, wherein at least two of R⁰, R¹, R^{a}, R^{b}, R^{c}, R^{d}, R^{e} and R^{f}, together with the carbon atoms to which they are attached, form a spiro ring on the heterocycle to which they are attached, and the spiro ring is optionally substituted with one or more R';
R' are identical or different and are each independently selected from the group consisting of a hydrogen atom, alkyl, halogen, alkoxy, haloalkoxy, cyano, hydroxy, hydroxyalkyl, -(CH₂)ₛNR⁷R⁸ and nitro;
R⁵ is selected from the group consisting of a hydrogen atom, alkyl, cycloalkyl, heterocyclyl, aryl and heteroaryl, wherein the alkyl, cycloalkyl, heterocyclyl, aryl and heteroaryl are each independently optionally substituted with one or more substituents selected from the group consisting of halogen, alkyl, haloalkyl, nitro, cyano, hydroxyalkyl, -(CH₂)_{y}NR^{g}R^{h}, -OR⁹, -COR⁹, -COOR⁹, -OS(O)ₜR⁹, -S(O)ₜR⁹, -NR⁶COR⁹, -NR⁶SO₂R⁹ and R;
the R is selected from the group consisting of cycloalkyl, heterocyclyl, aryl, heteroaryl, cycloalkylalkyl, heterocyclylalkyl, arylalkyl and heteroarylalkyl, and the R are each independently optionally substituted with one or more substituents selected from the group consisting of halogen, alkyl, haloalkyl, nitro, cyano, hydroxyalkyl, -(CH₂)_{y}NR^{g}R^{h}, -OR⁹, cycloalkyl, cycloalkylalkyl, heterocyclyl, heterocyclylalkyl, aryl and heteroaryl;
R² and R⁴ are identical or different and are each independently selected from the group consisting of a hydrogen atom, halogen, alkyl, haloalkyl, alkoxy, cycloalkyl, aryl, heterocyclyl, heteroaryl, cycloalkylalkyl, arylalkyl, heterocyclylalkyl and heteroarylalkyl, wherein the alkyl, cycloalkyl, heterocyclyl, aryl and heteroaryl are each independently optionally substituted with one or more substituents selected from the group consisting of alkyl, haloalkyl, halogen, cyano, nitro, -(CH₂)_{y}NR^{g}R^{h}, -OR⁹, -COR⁹, -COOR⁹, -OS(O)ₜR⁹, -S(O)ₜR⁹, -NR⁶COR⁹ and -NR⁶SO₂R⁹;
R³ are identical or different and are each independently selected from the group consisting of a hydrogen atom, halogen, alkyl, haloalkyl, cyano, nitro, -(CH₂)ₛNR⁷R⁸, -ORⁱ, -CORⁱ, -COORⁱ, -OS(O)ₓRⁱ, -S(O)ₓRⁱ, -NR⁶CORⁱ, -NR⁶SO₂Rⁱ, cycloalkyl, heterocyclyl, aryl and heteroaryl, wherein the alkyl, cycloalkyl, heterocyclyl, aryl and heteroaryl are each independently optionally substituted with one or more substituents selected from the group consisting of alkyl, haloalkyl, halogen, cyano, nitro, -(CH₂)_{y}NR^{g}R^{h}, -OR⁹, -COR⁹, -COOR⁹, -OS(O)ₜR⁹, -S(O)ₜR⁹, -NR⁶COR⁹ and -NR⁶SO₂R⁹;
or two adjacent R³, together with the carbon atom to which they are attached, form cycloalkyl, heterocyclyl, aryl or heteroaryl, wherein the cycloalkyl, heterocyclyl, aryl and heteroaryl are each independently optionally substituted with one or more substituents selected from the group consisting of a hydrogen atom, alkyl, halogen, haloalkyl, alkoxy, haloalkoxy, cyano, amino, nitro, hydroxy, hydroxyalkyl, cycloalkyl, heterocyclyl, aryl and heteroaryl;
R⁶ is selected from the group consisting of a hydrogen atom, alkyl, cycloalkyl and aryl, wherein the alkyl, cycloalkyl and aryl are each independently optionally substituted with one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R⁷, R⁸, R^{g} and R^{h} are identical or different and are each independently selected from the group consisting of a hydrogen atom, alkyl, haloalkyl, hydroxyalkyl, cycloalkyl, heterocyclyl, aryl and heteroaryl;
or R⁷ and R⁸, or R^{g} and R^{h}, together with the nitrogen atom to which they are attached, form heterocyclyl, wherein the heterocyclyl is optionally substituted with one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R⁹ and Rⁱ are identical or different and are each independently selected from the group consisting of a hydrogen atom, halogen, alkyl, haloalkyl, hydroxyalkyl, alkoxy, haloalkoxy, -(CH₂)ₛNR⁷R⁸, cycloalkyl, heterocyclyl, aryl and heteroaryl; wherein the alkyl, cycloalkyl, heterocyclyl, aryl and heteroaryl are each independently optionally substituted with one or more substituents selected from the group consisting of halogen, alkyl, alkoxy, haloalkyl, hydroxy, hydroxyalkyl, cyano, amino, nitro, cycloalkyl and heterocyclyl;
n is 1 or 2;
q is 0, 1, 2, 3 or 4;
s and y are identical or different and are each independently selected from the group consisting of 0, 1, 2, 3, 4 and 5; and
t and x are identical or different and are each independently selected from the group consisting of 0, 1 and 2.

The present disclosure is also intended to provide a compound of general formula (I) or a tautomer, racemate, enantiomer or diastereomer thereof or a mixture thereof, or a pharmaceutically acceptable salt thereof: 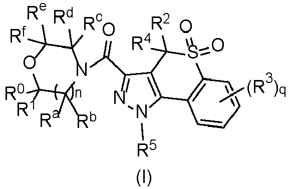 wherein:
R^{a}, R^{b}, R^{c}, R^{d}, R^{e} and R^{f} are identical or different and are each independently selected from the group consisting of a hydrogen atom, alkyl, halogen, alkoxy, haloalkoxy, cyano, hydroxy, hydroxyalkyl, -(CH₂)ₛNR⁷R⁸, cycloalkyl, cycloalkylalkyl, cycloalkyloxy, heterocyclyl, heterocyclylalkyl, heterocyclyloxy, aryl and heteroaryl,
wherein the alkyl, cycloalkyl, heterocyclyl, aryl and heteroaryl are each independently optionally substituted with one or more substituents selected from the group consisting of halogen, alkyl, haloalkyl, cyano, nitro, -(CH₂)_{y}NR^{g}R^{h} and -OR⁹;
R⁰ and R¹, together with the carbon atom to which they are attached, form a spiro ring on the heterocycle to which they are attached, the spiro ring being optionally substituted with one or more R';
R' are identical or different and are each independently selected from the group consisting of a hydrogen atom, alkyl, halogen, alkoxy, haloalkoxy, cyano, hydroxy, hydroxyalkyl, -(CH₂)ₛNR⁷R⁸ and nitro;
R⁵ is selected from the group consisting of a hydrogen atom, alkyl, cycloalkyl, heterocyclyl, aryl and heteroaryl, wherein the alkyl, cycloalkyl, heterocyclyl, aryl and
heteroaryl are each independently optionally substituted with one or more substituents selected from the group consisting of halogen, alkyl, haloalkyl, nitro, cyano, hydroxyalkyl, -(CH₂)_{y}NR^{g}R^{h}, -OR⁹, -COR⁹, -COOR⁹, -OS(O)ₜR⁹, -S(O)ₜR⁹, -NR⁶COR⁹, -NR⁶SO₂R⁹ and R;
the R is selected from the group consisting of cycloalkyl, heterocyclyl, aryl, heteroaryl, cycloalkylalkyl, heterocyclylalkyl, arylalkyl and heteroarylalkyl, and the R are each independently optionally substituted with one or more substituents selected from the group consisting of halogen, alkyl, haloalkyl, nitro, cyano, hydroxyalkyl, -(CH₂)_{y}NR^{g}R^{h}, -OR⁹, cycloalkyl, cycloalkylalkyl, heterocyclyl, heterocyclylalkyl, aryl and heteroaryl;
R² and R⁴ are identical or different and are each independently selected from the group consisting of a hydrogen atom, halogen, alkyl, haloalkyl, alkoxy, cycloalkyl, aryl, heterocyclyl, heteroaryl, cycloalkylalkyl, arylalkyl, heterocyclylalkyl and heteroarylalkyl, wherein the alkyl, cycloalkyl, heterocyclyl, aryl and heteroaryl are each independently optionally substituted with one or more substituents selected from the group consisting of alkyl, haloalkyl, halogen, cyano, nitro, -(CH₂)_{y}NR^{g}R^{h}, -OR⁹, -COR⁹, -COOR⁹, -OS(O)ₜR⁹, -S(O)ₜR⁹, -NR⁶COR⁹ and -NR⁶SO₂R⁹;
R³ are identical or different and are each independently selected from the group consisting of a hydrogen atom, halogen, alkyl, haloalkyl, cyano, nitro, -(CH₂)ₛNR⁷R⁸, -ORⁱ, -CORⁱ, -COORⁱ, -OS(O)ₓRⁱ, -S(O)ₓRⁱ, -NR⁶CORⁱ, -NR⁶SO₂Rⁱ, cycloalkyl, heterocyclyl, aryl and heteroaryl, wherein the alkyl, cycloalkyl, heterocyclyl, aryl and heteroaryl are each independently optionally substituted with one or more substituents selected from the group consisting of alkyl, haloalkyl, halogen, cyano, nitro, -(CH₂)_{y}NR^{g}R^{h}, -OR⁹, -COR⁹, -COOR⁹, -OS(O)ₜR⁹, -S(O)ₜR⁹, -NR⁶COR⁹ and -NR⁶SO₂R⁹;
or two adjacent R³, together with the carbon atom to which they are attached, form cycloalkyl, heterocyclyl, aryl or heteroaryl, wherein the cycloalkyl, heterocyclyl, aryl and heteroaryl are each independently optionally substituted with one or more substituents selected from the group consisting of a hydrogen atom, alkyl, halogen, haloalkyl, alkoxy, haloalkoxy, cyano, amino, nitro, hydroxy, hydroxyalkyl, cycloalkyl, heterocyclyl, aryl and heteroaryl;
R⁶ is selected from the group consisting of a hydrogen atom, alkyl, cycloalkyl and aryl, wherein the alkyl, cycloalkyl and aryl are each independently optionally substituted with one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R⁷, R⁸, R^{g} and R^{h} are identical or different and are each independently selected from the group consisting of a hydrogen atom, alkyl, haloalkyl, hydroxyalkyl, cycloalkyl, heterocyclyl, aryl and heteroaryl;
or R⁷ and R⁸, or R^{g} and R^{h}, together with the nitrogen atom to which they are attached, form heterocyclyl, wherein the heterocyclyl is optionally substituted with one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R⁹ and Rⁱ are identical or different and are each independently selected from the group consisting of a hydrogen atom, halogen, alkyl, haloalkyl, hydroxyalkyl, alkoxy, haloalkoxy, -(CH₂)ₛNR⁷R⁸, cycloalkyl, heterocyclyl, aryl and heteroaryl; wherein the alkyl, cycloalkyl, heterocyclyl, aryl and heteroaryl are each independently optionally substituted with one or more substituents selected from the group consisting of halogen, alkyl, alkoxy, haloalkyl, hydroxy, hydroxyalkyl, cyano, amino, nitro, cycloalkyl and heterocyclyl;
n is 1 or 2;
q is 0, 1, 2, 3 or 4;
s and y are identical or different and are each independently selected from the group consisting of 0, 1, 2, 3, 4 and 5; and
t and x are identical or different and are each independently selected from the group consisting of 0, 1 and 2.

In some preferred embodiments of the present disclosure, provided is a compound of general formula (I) or a tautomer, racemate, enantiomer or diastereomer thereof or a mixture thereof, or a pharmaceutically acceptable salt thereof, wherein R⁰ and R¹, together with the carbon to which they are attached, form a spiro ring on the heterocycle to which they are attached, preferably form a spiro 3-6 membered ring, more preferably form a spiro 3-6 membered carbocycle, and further preferably form a spirocyclopropyl. In some preferred embodiments of the present disclosure, provided is a compound of general formula (I) or a tautomer, racemate, enantiomer or diastereomer thereof or a mixture thereof, or a pharmaceutically acceptable salt thereof, which is a compound of general formula (II) or a tautomer, racemate, enantiomer or diastereomer thereof or a mixture thereof, or a pharmaceutically acceptable salt thereof: 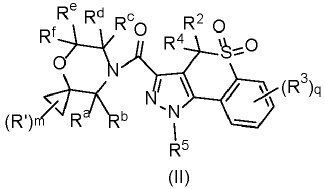
wherein: m is 0, 1, 2 or 3;
R', R^{a}-R^{f}, R²-R⁵ and q are as defined in general formula (I).

In some preferred embodiments of the present disclosure, provided is a compound of general formula (I) or (II) or a tautomer, racemate, enantiomer or diastereomer thereof or a mixture thereof, or a pharmaceutically acceptable salt thereof, wherein R⁵ is aryl or heteroaryl, and the aryl and heteroaryl are each independently optionally substituted with one or more substituents selected from the group consisting of halogen, alkyl, haloalkyl, nitro, cyano, hydroxyalkyl, -(CH₂)_{y}NR^{g}R^{h}, -OR⁹, -COR⁹, -COOR⁹, -OS(O)ₜR⁹, -S(O)ₜR⁹, -NR⁶COR⁹, -NR⁶SO₂R⁹ and R;
the R is selected from the group consisting of cycloalkyl, heterocyclyl, aryl, heteroaryl, cycloalkylalkyl, heterocyclylalkyl, arylalkyl and heteroarylalkyl, and the R are each independently optionally substituted with one or more substituents selected from the group consisting of halogen, alkyl, haloalkyl and-OR⁹;
R⁶, R⁹, R^{g}, R^{h}, y and t are as defined in general formula (I) or (II).

In some preferred embodiments of the present disclosure, provided is a compound of general formula (I) or (II) or a tautomer, racemate, enantiomer or diastereomer thereof or a mixture thereof, or a pharmaceutically acceptable salt thereof, wherein R⁵ is aryl or heteroaryl, and the aryl and heteroaryl are each independently optionally substituted with R, wherein the R is selected from the group consisting of cycloalkylalkyl, heterocyclylalkyl, arylalkyl and heteroarylalkyl, and the R are each independently optionally substituted with one or more substituents selected from the group consisting of halogen, alkyl and haloalkyl;
preferably, R⁵ is aryl, wherein the aryl is optionally substituted with heterocyclylalkyl, and the heterocyclylalkyl is optionally substituted with one or more substituents selected from the group consisting of halogen, alkyl and haloalkyl;
further preferably, R⁵ is 6-10 membered aryl, wherein the 6-10 membered aryl is optionally substituted with 3-8 membered heterocyclyl C₁₋₆ alkyl, and the 3-8 membered heterocyclyl C₁₋₆ alkyl is optionally substituted with one or more substituents selected from the group consisting of halogen, C₁₋₆ alkyl and C₁₋₆ haloalkyl;
more preferably, R⁵ is phenyl, wherein the phenyl is substituted with morpholinomethyl;
even more preferably, R⁵ is

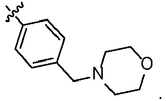

In some preferred embodiments of the present disclosure, provided is a compound of general formula (I) or (II) or a tautomer, racemate, enantiomer or diastereomer thereof or a mixture thereof, or a pharmaceutically acceptable salt thereof, which is a compound of general formula (III) or a tautomer, racemate, enantiomer or diastereomer thereof or a mixture thereof, or a pharmaceutically acceptable salt thereof: 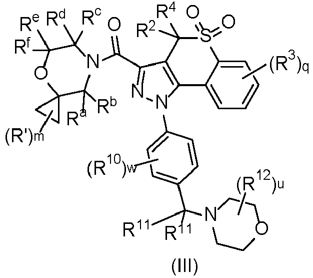 wherein:
R¹⁰ are identical or different and are each independently selected from the group consisting of a hydrogen atom, halogen, alkyl, haloalkyl, nitro, cyano, hydroxyalkyl, -(CH₂)ₛNR⁷R⁸, -OR⁹, cycloalkyl, heterocyclyl, aryl and heteroaryl, wherein the cycloalkyl, heterocyclyl, aryl and heteroaryl are each independently optionally substituted with one or more substituents selected from the group consisting of alkyl, haloalkyl, alkoxy, haloalkoxy, halogen, cyano, nitro and -(CH₂)_{y}NR^{g}R^{h};
R¹¹ are identical or different and are each independently selected from the group consisting of a hydrogen atom, halogen, alkyl, haloalkyl, alkoxy, haloalkoxy, hydroxy, hydroxyalkyl, cyano, nitro, -(CH₂)_{y}NR^{g}R^{h}, cycloalkyl, cycloalkyloxy and cycloalkylalkyl;
each R¹² is identical or different and is each independently selected from the group consisting of a hydrogen atom, halogen, alkyl, haloalkyl, nitro, cyano, hydroxyalkyl, -(CH₂)_{y}NR^{g}R^{h}, -OR⁹, cycloalkyl, cycloalkylalkyl, heterocyclyl, heterocyclylalkyl, aryl and heteroaryl; when u is greater than or equal to 2, two R¹² can form a spiro or bridged ring system on a morpholine ring;
w is 0, 1, 2, 3 or 4;
u is 0, 1, 2, 3, 4, 5 or 6;
R', R^{a}-R^{h}, R²-R⁴, R⁷-R⁹, s, m, y and q are as defined in general formula (I) or (II).

In some preferred embodiments of the present disclosure, provided is a compound of general formula (I), (II) or (III) or a tautomer, racemate, enantiomer or diastereomer thereof or a mixture thereof, or a pharmaceutically acceptable salt thereof, wherein R' are identical or different and are each independently selected from the group consisting of a hydrogen atom, alkyl and halogen; preferably, R' are identical or different and are each independently selected from the group consisting of a hydrogen atom, C₁₋₆ alkyl and halogen; more preferably, R' is a hydrogen atom.

In some preferred embodiments of the present disclosure, provided is a compound of general formula (I), (II) or (III) or a tautomer, racemate, enantiomer or diastereomer thereof or a mixture thereof, or a pharmaceutically acceptable salt thereof, wherein R² and R⁴ are identical or different and are each independently selected from the group consisting of a hydrogen atom, halogen, alkyl, haloalkyl, alkoxy, cycloalkyl and cycloalkylalkyl, wherein the alkyl and cycloalkyl are each independently optionally substituted with one or more substituents selected from the group consisting of alkyl, haloalkyl, halogen, cyano and -OR⁹, and R⁹ is as defined in general formula (I); preferably, R² and R⁴ are identical or different and are each independently selected from the group consisting of a hydrogen atom, halogen and C₁₋₆ alkyl; more preferably, both R² and R⁴ are hydrogen atoms.

In some preferred embodiments of the present disclosure, provided is a compound of general formula (I), (II) or (III) or a tautomer, racemate, enantiomer or diastereomer thereof or a mixture thereof, or a pharmaceutically acceptable salt thereof, wherein R³ are identical or different and are each independently selected from the group consisting of a hydrogen atom, halogen, alkyl, haloalkyl, cyano, nitro, -(CH₂)ₛNR⁷R⁸ and -ORⁱ, and R⁷, R⁸, Rⁱ and s are as defined in general formula (I);
preferably, R³ are identical or different and are each independently selected from the group consisting of a hydrogen atom, halogen, C₁₋₆ haloalkyl, C₁₋₆ alkoxy and C₁₋₆ alkyl;
more preferably, R³ are identical or different and are each independently selected from the group consisting of a hydrogen atom, halogen and C₁₋₆ alkyl; further preferably, R³ is fluorine.

In some preferred embodiments of the present disclosure, provided is a compound of general formula (I), (II) or (III) or a tautomer, racemate, enantiomer or diastereomer thereof or a mixture thereof, or a pharmaceutically acceptable salt thereof, wherein R⁶ is selected from the group consisting of a hydrogen atom, alkyl and cycloalkyl, wherein the alkyl and cycloalkyl are each independently optionally substituted with one or more substituents selected from the group consisting of alkyl, alkoxy, halogen, hydroxy and hydroxyalkyl; preferably, R⁶ is a hydrogen atom or C₁₋₆ alkyl.

In some preferred embodiments of the present disclosure, provided is a compound of general formula (I), (II) or (III) or a tautomer, racemate, enantiomer or diastereomer thereof or a mixture thereof, or a pharmaceutically acceptable salt thereof, wherein R⁷ and R⁸ are identical or different and are each independently selected from the group consisting of a hydrogen atom, alkyl, haloalkyl and cycloalkyl; or R⁷ and R⁸, together with the nitrogen atom to which they are attached, form a heterocyclyl, and the heterocyclyl is optionally substituted with one or more substituents selected from the group consisting of alkyl, alkoxy, halogen, hydroxyalkyl and cycloalkyl;
preferably, R⁷ and R⁸ are identical or different and are each independently selected from the group consisting of a hydrogen atom, C₁₋₆ alkyl and C₁₋₆ haloalkyl; or R⁷ and R⁸, together with the nitrogen atom to which they are attached, form a heterocyclyl, and the heterocyclyl is optionally substituted with one or more substituents selected from the group consisting of C₁₋₆ alkyl, C₁₋₆ alkoxy and halogen.

In some preferred embodiments of the present disclosure, provided is a compound of general formula (I), (II) or (III) or a tautomer, racemate, enantiomer or diastereomer thereof or a mixture thereof, or a pharmaceutically acceptable salt thereof, wherein R⁹ and R' are identical or different and are each independently selected from the group consisting of a hydrogen atom, alkyl, haloalkyl, hydroxyalkyl, cycloalkyl, heterocyclyl, aryl and heteroaryl, wherein the alkyl, cycloalkyl, heterocyclyl, aryl and heteroaryl are each independently optionally substituted with one or more substituents selected from the group consisting of halogen, alkyl, alkoxy, haloalkyl, cyano and amino;
preferably, R⁹ and Rⁱ are identical or different and are each independently selected from the group consisting of a hydrogen atom, alkyl, haloalkyl and cycloalkyl, wherein the alkyl and cycloalkyl are each independently optionally substituted with one or more substituents selected from the group consisting of halogen, alkyl, alkoxy and haloalkyl;
more preferably, R⁹ and Rⁱ are identical or different and are each independently selected from the group consisting of a hydrogen atom, C₁₋₆ alkyl and C₁₋₆ haloalkyl.

In some preferred embodiments of the present disclosure, provided is a compound of general formula (III) or a tautomer, racemate, enantiomer or diastereomer thereof or a mixture thereof, or a pharmaceutically acceptable salt thereof, wherein R¹⁰ are identical or different and are each independently selected from the group consisting of a hydrogen atom, halogen, alkyl, haloalkyl, nitro, cyano, hydroxyalkyl, -(CH₂)ₛNR⁷R⁸ and -OR⁹, and R⁷, R⁸, R⁹ and s are as defined in general formula (III);
preferably, R¹⁰ are identical or different and are each independently selected from the group consisting of a hydrogen atom, halogen and C₁₋₆ alkyl; more preferably, R¹⁰ is a hydrogen atom.

In some preferred embodiments of the present disclosure, provided is a compound of general formula (III) or a tautomer, racemate, enantiomer or diastereomer thereof or a mixture thereof, or a pharmaceutically acceptable salt thereof, wherein R¹¹ are identical or different and are each independently selected from the group consisting of a hydrogen atom, halogen, alkyl, haloalkyl, alkoxy, haloalkoxy, hydroxy, hydroxyalkyl, cyano and -(CH₂)_{y}NR^{g}R^{h}, and R^{g}, R^{h} and y are as defined in general formula (III); preferably, R¹¹ are identical or different and are each independently selected from the group consisting of a hydrogen atom, halogen and C₁₋₆ alkyl; more preferably, R¹¹ is a hydrogen atom.

In some preferred embodiments of the present disclosure, provided is a compound of general formula (III) or a tautomer, racemate, enantiomer or diastereomer thereof or a mixture thereof, or a pharmaceutically acceptable salt thereof, wherein R¹² are identical or different and are each independently selected from the group consisting of a hydrogen atom, halogen, alkyl, haloalkyl, nitro, cyano, hydroxyalkyl, -(CH₂)_{y}NR^{g}R^{h} and -OR⁹, and R⁹, R^{g}, R^{h} and y are as defined in general formula (III);
preferably, R¹² are identical or different and are each independently selected from the group consisting of a hydrogen atom, halogen and C₁₋₆ alkyl; more preferably, R¹² is a hydrogen atom.

In some preferred embodiments of the present disclosure, provided is a compound of general formula (I), (II) or (III) or a tautomer, racemate, enantiomer or diastereomer thereof or a mixture thereof, or a pharmaceutically acceptable salt thereof, wherein R^{a}-R^{f} are identical or different and are each independently selected from the group consisting of a hydrogen atom, halogen, alkyl, cyano, hydroxy and hydroxyalkyl; preferably, R^{a}-R^{f} are identical or different and are each independently selected from the group consisting of a hydrogen atom, halogen and C₁₋₆ alkyl; more preferably, R^{a}-R^{f} are each independently a hydrogen atom.

In some preferred embodiments of the present disclosure, provided is a compound of general formula (I) or a tautomer, racemate, enantiomer or diastereomer thereof or a mixture thereof, or a pharmaceutically acceptable salt thereof, wherein n is 1.

In some preferred embodiments of the present disclosure, provided is a compound of general formula (I), (II) or (III) or a tautomer, racemate, enantiomer or diastereomer thereof or a mixture thereof, or a pharmaceutically acceptable salt thereof, wherein q is 0, 1, 2 or 3, and is preferably 1.

In some preferred embodiments of the present disclosure, provided is a compound of general formula (I), (II) or (III) or a tautomer, racemate, enantiomer or diastereomer thereof or a mixture thereof, or a pharmaceutically acceptable salt thereof, wherein s and y are identical or different and are each independently selected from the group consisting of 0 and 1, and are preferably 0.

In some preferred embodiments of the present disclosure, provided is a compound of general formula (I), (II) or (III) or a tautomer, racemate, enantiomer or diastereomer thereof or a mixture thereof, or a pharmaceutically acceptable salt thereof, wherein t and x are identical or different and are each independently selected from the group consisting of 0 and 2, and are preferably 2.

In some preferred embodiments of the present disclosure, provided is a compound of general formula (II) or (III) or a tautomer, racemate, enantiomer or diastereomer thereof or a mixture thereof, or a pharmaceutically acceptable salt thereof, wherein m is 0, 1 or 2, preferably 0 or 1, and more preferably 0.

In some preferred embodiments of the present disclosure, provided is a compound of general formula (III) or a tautomer, racemate, enantiomer or diastereomer thereof or a mixture thereof, or a pharmaceutically acceptable salt thereof, wherein u is 0, 1, 2 or 3, preferably 0 or 1, and more preferably 0.

In some preferred embodiments of the present disclosure, provided is a compound of general formula (III) or a tautomer, racemate, enantiomer or diastereomer thereof or a mixture thereof, or a pharmaceutically acceptable salt thereof, wherein w is 0, 1 or 2, preferably 0 or 1, and more preferably 0.

In some preferred embodiments of the present disclosure, provided is a compound of general formula (III) or a tautomer, racemate, enantiomer or diastereomer thereof or a mixture thereof, or a pharmaceutically acceptable salt thereof, wherein R', R¹⁰, R¹², R^{a}, R^{b}, R^{c}, R^{d}, R^{e} and R^{f} are identical or different and are each independently selected from the group consisting of a hydrogen atom, halogen and C₁₋₆ alkyl; m is 0 or 1; w is 0 or 1; u is 0 or 1; R¹¹, R² and R⁴ are identical or different and are each independently selected from the group consisting of a hydrogen atom, halogen and C₁₋₆ alkyl; R³ are identical or different and are each independently selected from the group consisting of a hydrogen atom, halogen, C₁₋₆ haloalkyl, C₁₋₆ alkoxy and C₁₋₆ alkyl; and q is 1.

**Table A. Typical compounds disclosed herein include, but are not limited to:**

| Example No. | Structure and name of compound |
|---|---|
| 1 | 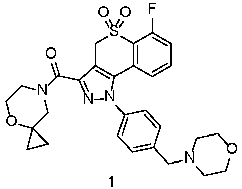 |
| | (6-fluoro-1-(4-(morpholinomethyl)phenyl)-5, 5-dioxido-1,4-dihydrothioc hromeno[4,3-c]pyrazol-3-yl)(4-oxa-7-azaspiro[2.5]octan-7-yl)methanon e 1 |
| 2 | 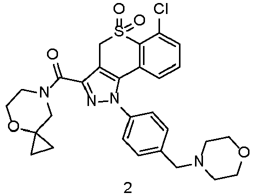 |
| | (6-chloro-1-(4-(morpholinomethyl)phenyl)-5,5-dioxido-1,4-dihydrothioc hromeno[4,3-c]pyrazol-3-yl)(4-oxa-7-azaspiro[2.5]octan-7-yl)methanon e2 |
| 3 | 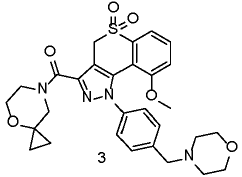 |
| | (9-methoxy-1-(4-(morpholinomethyl)phenyl)-5,5-dioxido-1,4-dihydrothi ochromeno[4,3-c]pyrazol-3-yl)(4-oxa-7-azaspiro[2.5]octan-7-yl)methan one 3 |
| 4 | 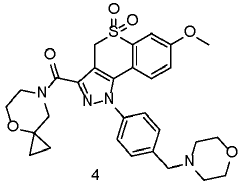 |
| | (7-methoxy-1-(4-(morpholinomethyl)phenyl)-5,5-dioxido-1,4-dihydrothi ochromeno[4,3-c]pyrazol-3-yl)(4-oxa-7-azaspiro[2.5]octan-7-yl)methan one **4** |
| 5 | 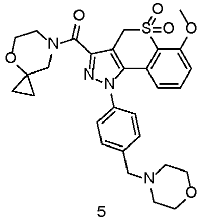 |
| | (6-methoxy-1-(4-(morpholinomethyl)phenyl)-5, 5-dioxido-1,4-dihydrothi ochromeno[4,3-*c*]pyrazol-3-yl)(4-oxa-7-azaspiro[2.5]octan-7-yl)methan one **5** |
| 6 | 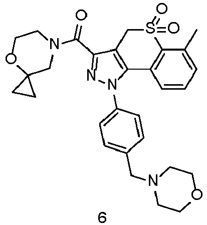 |
| | (6-methyl-1-(4-(morpholinomethyl)phenyl)-5,5-dioxido-1,4-dihydrothio chromeno[4,3-*c*]pyrazol-3-yl)(4-oxa-7-azaspiro[2.5]octan-7-yl)methano ne **6** |
| 7 | 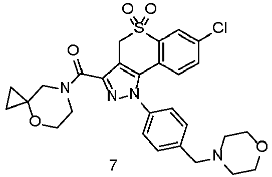 |
| | (7-chloro-1-(4-(morpholinomethyl)phenyl)-5,5-dioxido-1,4-dihydrothioc hromeno[4,3-*c*]pyrazol-3-yl)(4-oxa-7-azaspiro[2.5]octan-7-yl)methanon e 7 |
| 8 | 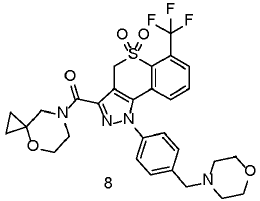 |
| | (1-(4-(morpholinomethyl)phenyl)-5,5-dioxido-6-(trifluoromethyl)-1,4-dihydrothiochromeno[4,3-*c*]pyrazol-3-yl)(4-oxa-7-azaspiro[2.5]octan-7-yl )methanone **8** |
| 9 | 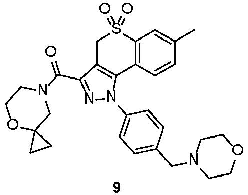 |
| | (7-methyl-1-(4-(morpholinomethyl)phenyl)-5,5-dioxido-1,4-dihydrothio chromeno[4,3-*c*]pyrazol-3-yl)(4-oxa-7-azaspiro[2.5]octan-7-yl)methano ne **9** |
| 11 | 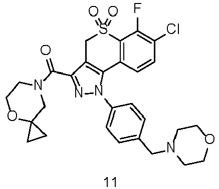 |
| | (7-chloro-6-fluoro-1-(4-(morpholinomethyl)phenyl)-5, 5-dioxido-1,4-dih ydrothiochromeno[4,3-*c*]pyrazol-3-yl)(4-oxa-7-azaspiro[2.5]octan-7-yl) methanone **11** |
| 12 | 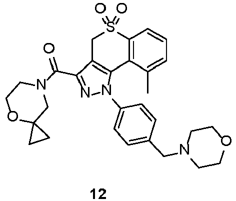 |
| | (9-methyl-1-(4-(morpholinomethyl)phenyl)-5,5-dioxido-1,4-dihydrothio chromeno[4,3-*c*]pyrazol-3-yl)(4-oxa-7-azaspiro[2.5]octan-7-yl)methano ne **12** |

Another aspect of the present disclosure relates to a method for preparing a compound of general formula (I) or a tautomer, racemate, enantiomer or diastereomer thereof or a mixture thereof, or a pharmaceutically acceptable salt thereof, which comprises the following step: 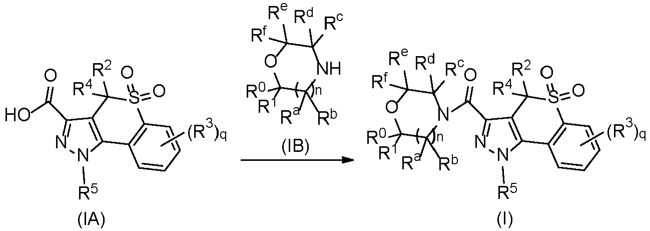
subjecting a compound of general formula (IA) or a tautomer, racemate, enantiomer or diastereomer thereof or a mixture thereof, or a pharmaceutically acceptable salt thereof to a reaction with a compound of general formula (IB) or a pharmaceutically acceptable salt thereof to give the compound of general formula (I) or the tautomer, racemate, enantiomer or diastereomer thereof or the mixture thereof, or the pharmaceutically acceptable salt thereof,
wherein R⁰-R⁵, R^{a}-R^{f}, n and q are as defined in general formula (I).

Another aspect of the present disclosure relates to a method for preparing a compound of general formula (II) or a tautomer, racemate, enantiomer or diastereomer thereof or a mixture thereof, or a pharmaceutically acceptable salt thereof, which comprises the following step: 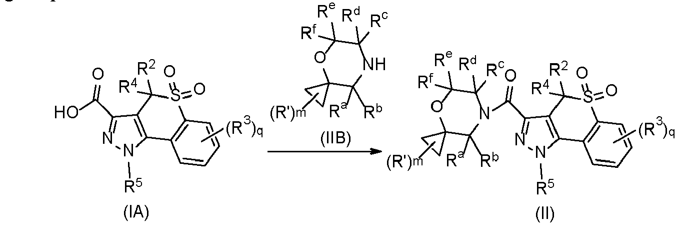
subjecting a compound of general formula (IA) or a tautomer, racemate, enantiomer or diastereomer thereof or a mixture thereof, or a pharmaceutically acceptable salt thereof to a reaction with a compound of general formula (IIB) or a pharmaceutically acceptable salt thereof to give the compound of general formula (II) or the tautomer, racemate, enantiomer or diastereomer thereof or the mixture thereof, or the pharmaceutically acceptable salt thereof,
wherein R', R²-R⁵, R^{a}-R^{f}, m and q are as defined in general formula (II).

Another aspect of the present disclosure relates to a method for preparing a compound of general formula (III) or a tautomer, racemate, enantiomer or diastereomer thereof or a mixture thereof, or a pharmaceutically acceptable salt thereof, which comprises the following step: 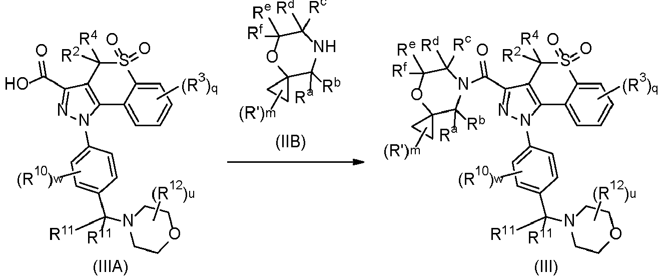
subjecting a compound of general formula (IIIA) or a tautomer, racemate, enantiomer or diastereomer thereof or a mixture thereof, or a pharmaceutically acceptable salt thereof to a reaction with general formula (IIB) or a pharmaceutically acceptable salt thereof to give the compound of general formula (III) or the tautomer, racemate, enantiomer or diastereomer thereof or the mixture thereof, or the pharmaceutically acceptable salt thereof,
wherein R', R²-R⁴, R^{a}-R^{f}, R¹⁰-R¹², q, u, w and m are as defined in general formula (III).

Another aspect of the present disclosure relates to a pharmaceutical composition comprising the compound of general formula (I), (II) or (III) or the compounds shown in Table A or the tautomer, racemate, enantiomer or diastereomer thereof or the mixture thereof, or the pharmaceutically acceptable salt thereof disclosed herein, and one or more pharmaceutically acceptable carriers, diluents or excipients.

The present disclosure further relates to use of the compound of general formula (I), (II) or (III) or the compounds shown in Table A or the tautomer, racemate, enantiomer or diastereomer thereof or the mixture thereof, or the pharmaceutically acceptable salt thereof, or the pharmaceutical composition comprising the same, in preparing a medicament for inhibiting PI3Kδ.

The present disclosure further relates to use of the compound of general formula (I), (II) or (III) or the compounds shown in Table A or the tautomer, racemate, enantiomer or diastereomer thereof or the mixture thereof, or the pharmaceutically acceptable salt thereof, or the pharmaceutical composition comprising the same, in preparing a medicament for treating and/or preventing inflammatory diseases, autoimmune diseases, cancer and related diseases, wherein: the cancer is preferably selected from the group consisting of melanoma, skin cancer, liver cancer, kidney cancer, lung cancer, nasopharyngeal cancer, gastric cancer, esophageal cancer, colorectal cancer, gallbladder cancer, bile duct cancer, chorionic epithelioma, pancreatic cancer, polycythemia vera, pediatric tumors, cervical cancer, ovarian cancer, breast cancer, bladder cancer, urothelial cancer, ureteral tumor, prostate cancer, seminoma, testicular tumor, leukemia, head and neck tumor, endometrial cancer, thyroid cancer, lymphoma, sarcoma, osteoma, neuroturbo chargeoma, neuroblastoma, neuroendocrine carcinoma, brain tumor, CNS cancer, myeloma, astrocytoma, glioblastoma and glioma (the tumors described above are all corresponding malignancies), the leukemia is preferably selected from the group consisting of chronic lymphocytic leukemia, acute lymphocytic leukemia (ALL), acute myeloid leukemia (AML), chronic myeloid leukemia (CML) and hairy cell leukemia, the lymphoma is preferably selected from the group consisting of small lymphocytic lymphoma, marginal zone lymphoma, follicular lymphoma, mantle cell lymphoma, non-Hodgkin's lymphoma (NHL), lymphoplasmacytic lymphoma, nodal marginal zone lymphoma, T-cell lymphoma, B-cell lymphoma and diffuse large B-cell lymphoma, the lung cancer is preferably non-small cell lung cancer or small cell lung cancer, the myeloma is preferably multiple myeloma (MM), the autoimmune disease is preferably selected from the group consisting of asthma, rheumatoid arthritis, acute disseminated encephalomyelitis (ADEM), Addison's disease, alopecia areata, ankylosing spondylitis, antiphospholipid antibody syndrome (APS), autoimmune hemolytic anemia, autoimmune hepatitis, autoimmune inner ear disease, pemphigus, pemphigoid, Behcet's disease, celiac disease, anti-glutamine transaminase, Chagas' disease, chronic obstructive pulmonary disease, Crohn's disease, dermatomyositis, type 1 diabetes, endometriosis, Goodpasture's syndrome, Graves' disease, Guillain-Barre's syndrome (GBS), Hashimoto's disease, hidradenitis suppurativa, Kawasaki disease, IgA nephropathy, immune thrombocytopenic purpura, idiopathic thrombocytopenic purpura (ITP), interstitial cystitis, lupus, lupus nephritis, membranous nephropathy, mixed connective tissue disease, morphea, multiple sclerosis (MS), myasthenia gravis, narcolepsy, neuromyotonia, pernicious anemia, psoriasis, psoriatic arthritis, polymyositis, primary biliary cirrhosis, schizophrenia, scleroderma, dry eye and mouth syndrome, Sjogren's syndrome, stiffman syndrome, temporal arteritis, ulcerative colitis, vasculitis, vitiligo and Wegener's granulomatosis, the lupus is preferably lupus erythematosus or systemic lupus erythematosus, the pemphigus is preferably pemphigus vulgaris, the liver cancer is preferably hepatocellular carcinoma, the head and neck tumor is preferably head and neck squamous cell carcinoma, the sarcoma is preferably osteosarcoma or soft tissue sarcoma, and the colorectal cancer is preferably colon cancer or rectal cancer.

The present disclosure further relates to a method for inhibiting PI3Kδ, which comprises administering to a patient in need an inhibiting effective amount of the compound of general formula (I), (II) or (III) or the compounds shown in Table A or the tautomer, racemate, enantiomer or diastereomer thereof or the mixture thereof, or the pharmaceutically acceptable salt thereof, or the pharmaceutical composition comprising the same.

The present disclosure further relates to a method for treating and/or preventing a PI3Kδ-mediated disease, which comprises administering to a patient in need a therapeutically effective amount of the compound of general formula (I), (II) or (III) or the compounds shown in Table A or the tautomer, racemate, enantiomer or diastereomer thereof or the mixture thereof, or the pharmaceutically acceptable salt thereof, or the pharmaceutical composition comprising the same.

The present disclosure further relates to a method for treating and/or preventing inflammatory diseases, autoimmune diseases, cancer and related diseases, which comprises administering to a patient in need a therapeutically or prophylactically effective amount of the compound of general formula (I), (II) or (III) or the compounds shown in Table A or the tautomer, racemate, enantiomer or diastereomer thereof or the mixture thereof, or the pharmaceutically acceptable salt thereof, or the pharmaceutical composition comprising the same, wherein: the cancer is preferably selected from the group consisting of melanoma, skin cancer, liver cancer, kidney cancer, lung cancer, nasopharyngeal cancer, gastric cancer, esophageal cancer, colorectal cancer, gallbladder cancer, bile duct cancer, chorionic epithelioma, pancreatic cancer, polycythemia vera, pediatric tumors, cervical cancer, ovarian cancer, breast cancer, bladder cancer, urothelial cancer, ureteral tumor, prostate cancer, seminoma, testicular tumor, leukemia, head and neck tumor, endometrial cancer, thyroid cancer, lymphoma, sarcoma, osteoma, neuroturbo chargeoma, neuroblastoma, neuroendocrine carcinoma, brain tumor, CNS cancer, myeloma, astrocytoma, glioblastoma and glioma, the leukemia is preferably selected from the group consisting of chronic lymphocytic leukemia, acute lymphocytic leukemia (ALL), acute myeloid leukemia (AML), chronic myeloid leukemia (CML) and hairy cell leukemia, the lymphoma is preferably selected from the group consisting of small lymphocytic lymphoma, marginal zone lymphoma, follicular lymphoma, mantle cell lymphoma, non-Hodgkin's lymphoma (NHL), lymphoplasmacytic lymphoma, nodal marginal zone lymphoma, T-cell lymphoma, B-cell lymphoma and diffuse large B-cell lymphoma, the lung cancer is preferably non-small cell lung cancer or small cell lung cancer, the myeloma is preferably multiple myeloma (MM), the autoimmune disease is preferably selected from the group consisting of asthma, rheumatoid arthritis, acute disseminated encephalomyelitis (ADEM), Addison's disease, alopecia areata, ankylosing spondylitis, antiphospholipid antibody syndrome (APS), autoimmune hemolytic anemia, autoimmune hepatitis, autoimmune inner ear disease, pemphigus, pemphigoid, Behcet's disease, celiac disease, anti-glutamine transaminase, Chagas' disease, chronic obstructive pulmonary disease, Crohn's disease, dermatomyositis, type 1 diabetes, endometriosis, Goodpasture's syndrome, Graves' disease, Guillain-Barre's syndrome (GBS), Hashimoto's disease, hidradenitis suppurativa, Kawasaki disease, IgA nephropathy, immune thrombocytopenic purpura, idiopathic thrombocytopenic purpura (ITP), interstitial cystitis, lupus, lupus nephritis, membranous nephropathy, mixed connective tissue disease, morphea, multiple sclerosis (MS), myasthenia gravis, narcolepsy, neuromyotonia, pernicious anemia, psoriasis, psoriatic arthritis, polymyositis, primary biliary cirrhosis, schizophrenia, scleroderma, dry eye and mouth syndrome, Sjogren's syndrome, stiffman syndrome, temporal arteritis, ulcerative colitis, vasculitis, vitiligo and Wegener's granulomatosis, the lupus is preferably lupus erythematosus or systemic lupus erythematosus, the pemphigus is preferably pemphigus vulgaris, the liver cancer is preferably hepatocellular carcinoma, the head and neck tumor is preferably head and neck squamous cell carcinoma, the sarcoma is preferably osteosarcoma or soft tissue sarcoma, and the colorectal cancer is preferably colon cancer or rectal cancer.

The present disclosure further relates to the compound of general formula (I), (II) or (III) or the compounds shown in Table A or the tautomer, racemate, enantiomer or diastereomer thereof or the mixture thereof, or the pharmaceutically acceptable salt thereof, or the pharmaceutical composition comprising the same, for use as a medicament.

The present disclosure further relates to the compound of general formula (I), (II) or (III) or the compounds shown in Table A or the tautomer, racemate, enantiomer or diastereomer thereof or the mixture thereof, or the pharmaceutically acceptable salt thereof, or the pharmaceutical composition comprising the same, for use as a PI3Kδ inhibitor.

The present disclosure further relates to the compound of general formula (I), (II) or (III) or the compounds shown in Table A or the tautomer, racemate, enantiomer or diastereomer thereof or the mixture thereof, or the pharmaceutically acceptable salt thereof, or the pharmaceutical composition comprising the same, for use in treating and/or preventing a PI3Kδ-mediated disease.

The present disclosure further relates to the compound of general formula (I), (II) or (III) or the compounds shown in Table A or the tautomer, racemate, enantiomer or diastereomer thereof or the mixture thereof, or the pharmaceutically acceptable salt thereof, or the pharmaceutical composition comprising the same, for use in treating and/or preventing inflammatory diseases, autoimmune diseases, cancer and related diseases, wherein: the cancer is preferably selected from the group consisting of melanoma, skin cancer, liver cancer, kidney cancer, lung cancer, nasopharyngeal cancer, gastric cancer, esophageal cancer, colorectal cancer, gallbladder cancer, bile duct cancer, chorionic epithelioma, pancreatic cancer, polycythemia vera, pediatric tumors, cervical cancer, ovarian cancer, breast cancer, bladder cancer, urothelial cancer, ureteral tumor, prostate cancer, seminoma, testicular tumor, leukemia, head and neck tumor, endometrial cancer, thyroid cancer, lymphoma, sarcoma, osteoma, neuroturbo chargeoma, neuroblastoma, neuroendocrine carcinoma, brain tumor, CNS cancer, myeloma, astrocytoma, glioblastoma and glioma, the leukemia is preferably selected from the group consisting of chronic lymphocytic leukemia, acute lymphocytic leukemia (ALL), acute myeloid leukemia (AML), chronic myeloid leukemia (CML) and hairy cell leukemia, the lymphoma is preferably selected from the group consisting of small lymphocytic lymphoma, marginal zone lymphoma, follicular lymphoma, mantle cell lymphoma, non-Hodgkin's lymphoma (NHL), lymphoplasmacytic lymphoma, nodal marginal zone lymphoma, T-cell lymphoma, B-cell lymphoma and diffuse large B-cell lymphoma, the lung cancer is preferably non-small cell lung cancer or small cell lung cancer, the myeloma is preferably multiple myeloma (MM), the autoimmune disease is preferably selected from the group consisting of asthma, rheumatoid arthritis, acute disseminated encephalomyelitis (ADEM), Addison's disease, alopecia areata, ankylosing spondylitis, antiphospholipid antibody syndrome (APS), autoimmune hemolytic anemia, autoimmune hepatitis, autoimmune inner ear disease, pemphigus, pemphigoid, Behcet's disease, celiac disease, anti-glutamine transaminase, Chagas' disease, chronic obstructive pulmonary disease, Crohn's disease, dermatomyositis, type 1 diabetes, endometriosis, Goodpasture's syndrome, Graves' disease, Guillain-Barre's syndrome (GBS), Hashimoto's disease, hidradenitis suppurativa, Kawasaki disease, IgA nephropathy, immune thrombocytopenic purpura, idiopathic thrombocytopenic purpura (ITP), interstitial cystitis, lupus, lupus nephritis, membranous nephropathy, mixed connective tissue disease, morphea, multiple sclerosis (MS), myasthenia gravis, narcolepsy, neuromyotonia, pernicious anemia, psoriasis, psoriatic arthritis, polymyositis, primary biliary cirrhosis, schizophrenia, scleroderma, dry eye and mouth syndrome, Sjogren's syndrome, stiffman syndrome, temporal arteritis, ulcerative colitis, vasculitis, vitiligo and Wegener's granulomatosis, the lupus is preferably lupus erythematosus or systemic lupus erythematosus, the pemphigus is preferably pemphigus vulgaris, the liver cancer is preferably hepatocellular carcinoma, the head and neck tumor is preferably head and neck squamous cell carcinoma, the sarcoma is preferably osteosarcoma or soft tissue sarcoma, and the colorectal cancer is preferably colon cancer or rectal cancer.

The active compound may be formulated into a form suitable for administration by any suitable route, and one or more pharmaceutically acceptable carriers are used to formulate the composition of the present disclosure by conventional methods. Thus, the active compound of the present disclosure may be formulated into a variety of dosage forms for oral administration, administration by injection (e.g., intravenous, intramuscular or subcutaneous), or administration by inhalation or insufflation. The compounds of the present disclosure may also be formulated into a dosage form, such as tablets, hard or soft capsules, aqueous or oily suspensions, emulsions, injections, dispersible powders or granules, suppositories, lozenges or syrups.

As a general guide, the active compound of the present disclosure is preferably in a form of a unit dose, or in a form of a single dose that can be self-administered by a patient. The unit dose of the compound or composition of the present disclosure may be in a tablet, capsule, cachet, vial, powder, granule, lozenge, suppository, regenerating powder or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the composition may comprise 0.1 wt.% to 99 wt.% of the active compound.

The tablet comprises the active ingredient and a non-toxic pharmaceutically acceptable excipient that is used for mixing and is suitable for the preparation of the tablet. Such an excipient may be an inert excipient, a granulating agent, a disintegrant, a binder and a lubricant. Such a tablet may be uncoated or may be coated by known techniques for masking the taste of the drug or delaying the disintegration and absorption of the drug in the gastrointestinal tract and thus enabling sustained release of the drug over a longer period.

An oral formulation in a soft gelatin capsule where the active ingredient is mixed with an inert solid diluent or with a water-soluble carrier or oil vehicle may also be provided. An aqueous suspension comprises the active substance and an excipient that is used for mixing and is suitable for the preparation of the aqueous suspension. Such an excipient is a suspending agent, a dispersant or a wetting agent. The aqueous suspension may also comprise one or more preservatives, one or more colorants, one or more corrigents and one or more sweeteners.

An oil suspension may be formulated by suspending the active ingredient in a vegetable oil, or in a mineral oil. The oil suspension may comprise a thickening agent. The sweeteners and corrigents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative and an antioxidant. Such a formulation may also comprise a palliative, a preservative, a colorant and an antioxidant.

The pharmaceutical composition of the present disclosure may be in the form of a sterile injectable aqueous solution. Acceptable vehicles or solvents that can be used include water, Ringer's solution and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections.

The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compound of the present disclosure can be administered in the form of dispersible powders and granules that are formulated into aqueous suspensions by adding water. Such a pharmaceutical composition can be prepared by mixing the active ingredient with a dispersant or a wetting agent, a suspending agent, or one or more preservatives. As is well known to those skilled in the art, the dose of the drug administered depends on a variety of factors, including but not limited to, the activity of the particular compound employed, the severity of the disease, the age of the patient, the weight of the patient, the health condition of the patient, the behavior of the patient, the diet of the patient, the time of administration, the route of administration, the rate of excretion, the combination of drugs, and the like. In addition, the optimal treatment regimen, such as the mode of administration, the daily dose of the compound or the type of pharmaceutically acceptable salts, can be verified according to conventional treatment regimens.

### Description of the terms

Unless otherwise stated, the terms used in the specification and claims have the following meanings.

The term "alkyl" refers to a saturated aliphatic hydrocarbon group, which is a linear or branched group containing 1 to 20 carbon atoms, preferably alkyl containing 1 to 12 (e.g., 1, 2, 3, 4, 5, 6, 7, 8, 9, 10, 11 and 12) carbon atoms, and more preferably alkyl containing 1 to 6 (e.g., 1, 2, 3, 4, 5 and 6) carbon atoms. Non-limiting examples include methyl, ethyl, *n*-propyl, isopropyl, *n*-butyl, isobutyl, *tert-*butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof, and the like. More preferred is a lower alkyl having 1 to 6 carbon atoms, and non-limiting examples include methyl, ethyl, *n*-propyl, isopropyl, *n*-butyl, isobutyl, *tert-*butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl may be substituted or unsubstituted. When substituted, it may be substituted at any accessible connection site, wherein the substituent is preferably one or more substituents independently and optionally selected from the group consisting of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkylene" refers to a saturated linear or branched aliphatic hydrocarbon group, which is a residue derived from the parent alkane by removal of two hydrogen atoms from the same carbon atom or two different carbon atoms. It is a linear or branched group containing 1 to 20 carbon atoms, preferably alkylene containing 1 to 12 (e.g., 1, 2, 3, 4, 5, 6, 7, 8, 9, 10, 11 and 12) carbon atoms, and more preferably alkylene containing 1 to 6 carbon atoms. Non-limiting examples of alkylene include, but are not limited to, methylene (-CH₂-), 1,1-ethylene (-CH(CH₃)-), 1,2-ethylene (-CH₂CH₂-), 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), and the like. The alkylene may be substituted or unsubstituted. When substituted, it may be substituted at any accessible connection site, wherein the substituent is preferably one or more substituents independently and optionally selected from the group consisting of alkenyl, alkynyl, alkoxy, haloalkoxy, cycloalkyloxy, heterocyclyloxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio and oxo.

The term "alkenyl" refers to an alkyl compound containing at least one carbon-carbon double bond in the molecule, wherein the alkyl is as defined above. The alkenyl may be substituted or unsubstituted. When substituted, the substituent is preferably one or more groups independently selected from the group consisting of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkynyl" refers to an alkyl compound containing at least one carbon-carbon triple bond in the molecule, wherein the alkyl is as defined above. The alkynyl may be substituted or unsubstituted. When substituted, the substituent is preferably one or more groups independently selected from the group consisting of alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, more preferably 3 to 8 (e.g., 3, 4, 5, 6, 7 and 8) carbon atoms, and most preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "spiro cycloalkyl" refers to a 5- to 20-membered polycyclic group in which monocyclic rings share one carbon atom (referred to as the spiro atom), wherein the spiro cycloalkyl may contain one or more double bonds. The spiro cycloalkyl is preferably 6- to 14-membered, and more preferably 7- to 10-membered (e.g., 7-membered, 8-membered, 9-membered or 10-membered). According to the number of the spiro atoms shared among the rings, the spiro cycloalkyl may be monospiro cycloalkyl, bispiro cycloalkyl or polyspiro cycloalkyl, preferably monospiro cycloalkyl and bispiro cycloalkyl, and more preferably 3-membered/4-membered, 3-membered/5-membered, 3-membered/6-membered, 4-membered/4-membered, 4-membered/5-membered, 4-membered/6-membered, 5-membered/5-membered, 5-membered/6-membered or 6-membered/6-membered monospiro cycloalkyl. Non-limiting examples of spiro cycloalkyl include:

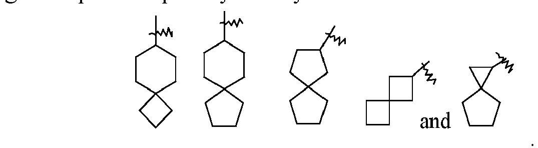

The term "fused cycloalkyl" refers to a 5- to 20-membered carbon polycyclic group in which each ring shares a pair of adjacent carbon atoms with the other rings in the system, wherein one or more of the rings may contain one or more double bonds. The fused cycloalkyl is preferably 6- to 14-membered, and more preferably 7- to 10-membered (e.g., 7-membered, 8-membered, 9-membered or 10-membered).

According to the number of the formed rings, the fused cycloalkyl may be bicyclic, tricyclic, tetracyclic or polycyclic fused cycloalkyl, preferably bicyclic or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3 -membered, 6-membered/4-membered, 6-membered/5-membered and 6-membered/6-membered bicyclic fused cycloalkyl. Non-limiting examples of fused cycloalkyl include:

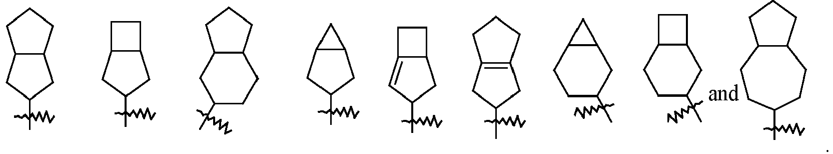

The term "bridged cycloalkyl" refers to a 5- to 20-membered carbon polycyclic group in which any two rings share two carbon atoms that are not directly connected to each other, wherein the bridged cycloalkyl may contain one or more double bonds. The bridged cycloalkyl is preferably 6- to 14-membered, and more preferably 7- to 10-membered (e.g., 7-membered, 8-membered, 9-membered or 10-membered). According to the number of the formed rings, the bridged cycloalkyl may be bicyclic, tricyclic, tetracyclic or polycyclic, preferably bicyclic, tricyclic or tetracyclic, and more preferably bicyclic or tricyclic. Non-limiting examples of bridged cycloalkyl include:

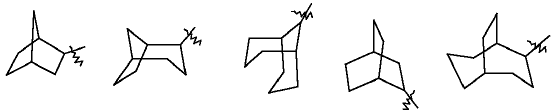

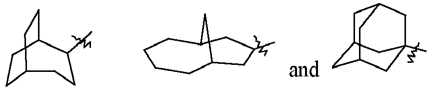

The cycloalkyl ring includes those in which the cycloalkyl described above (including monocyclic, spiro, fused and bridged rings) is fused to an aryl, heteroaryl or heterocycloalkyl ring, wherein the ring connected to the parent structure is cycloalkyl. Non-limiting examples include 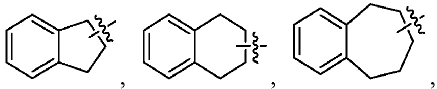 and the like, and it is preferably

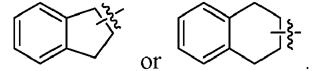

The cycloalkyl may be substituted or unsubstituted. When substituted, it may be substituted at any accessible connection site, wherein the substituent is preferably one or more substituents independently and optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "alkoxy" refers to -O-(alkyl), wherein the alkyl is as defined above. Non-limiting examples of alkoxy include methoxy, ethoxy, propoxy and butoxy. Alkoxy may be optionally substituted or unsubstituted. When substituted, the substituent is preferably one or more groups independently selected from the group consisting of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heterocyclyl" refers to a saturated or partially unsaturated monocyclic or polycyclic substituent containing 3 to 20 ring atoms, wherein one or more of the ring atoms are a heteroatom selected from the group consisting of nitrogen, oxygen and sulfur, the sulfur optionally being substituted with oxo (i.e., form sulfoxide or sulfone), but excluding a cyclic portion of -O-O-, -O-S- or -S-S-; and the remaining ring atoms are carbon. The heterocyclyl preferably contains 3 to 12 (e.g., 3, 4, 5, 6, 7, 8, 9, 10, 11 and 12) ring atoms, of which 1 to 4 (e.g., 1, 2, 3 and 4) are heteroatoms; more preferably 3 to 8 (e.g., 3, 4, 5, 6, 7 and 8) ring atoms, of which 1 to 3 (e.g., 1, 2 and 3) are heteroatoms; more preferably 3 to 6 ring atoms, of which 1 to 3 are heteroatoms; most preferably 5 or 6 ring atoms, of which 1 to 3 are heteroatoms. Non-limiting examples of monocyclic heterocyclyl include pyrrolidinyl, tetrahydropyranyl, 1,2.3.6-tetrahydropyridinyl, piperidinyl, piperazinyl, morpholinyl, thiomorpholinyl, homopiperazinyl, and the like. Polycyclic heterocyclyl includes spiro heterocyclyl, fused heterocyclyl, and bridged heterocyclyl.

The term "spiro heterocyclyl" refers to a 5-20 membered polycyclic heterocyclyl group in which monocyclic rings share one atom (referred to as the spiro atom), wherein one or more of the ring atoms is a heteroatom selected from the group consisting of nitrogen, oxygen and sulfur, the sulfur optionally being substituted with oxo (i.e., form sulfoxide or sulfone); and the remaining ring atoms are carbon. The spiro heterocyclyl may contain one or more double bonds. The spiro heterocyclyl is preferably 6- to 14-membered, and more preferably 7- to 10-membered (e.g., 7-membered, 8-membered, 9-membered or 10-membered). According to the number of spiro atoms shared among the rings, the spiro heterocyclyl may be monospiro heterocyclyl, bispiro heterocyclyl or polyspiro heterocyclyl, preferably monospiro heterocyclyl and bispiro heterocyclyl, and more preferably 3-membered/4-membered, 3-membered/5-membered, 3-membered/6-membered, 4-membered/4-membered, 4-membered/5-membered, 4-membered/6-membered, 5-membered/5-membered, 5-membered/6-membered or 6-membered/6-membered monospiro heterocyclyl. Non-limiting examples of spiro heterocyclyl include:

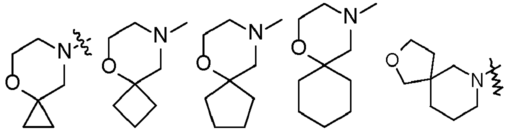

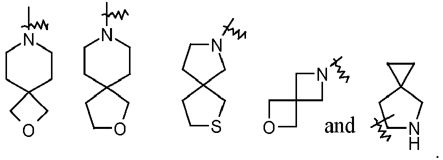

The term "fused heterocyclyl" refers to a 5- to 20-membered polycyclic heterocyclyl group in which each ring shares a pair of adjacent atoms with the other rings in the system, wherein one or more of the rings may contain one or more double bonds, wherein one or more of the ring atoms is a heteroatom selected from the group consisting of nitrogen, oxygen and sulfur, the sulfur optionally being substituted with oxo (i.e., form sulfoxide or sulfone); and the remaining ring atoms are carbon. The fused heterocyclyl is preferably 6- to 14-membered, and more preferably 7- to 10-membered (e.g., 7-membered, 8-membered, 9-membered or 10-membered). According to the number of the formed rings, the fused heterocyclyl may be bicyclic, tricyclic, tetracyclic or polycyclic fused heterocyclyl, preferably bicyclic or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3 -membered, 6-membered/4-membered, 6-membered/5-membered and 6-membered/6-membered bicyclic fused heterocyclyl. Non-limiting examples of fused heterocyclyl include:

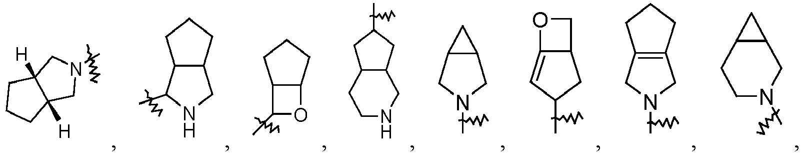

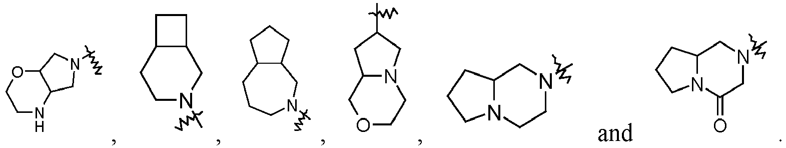

The term "bridged heterocyclyl" refers to a 5- to 14-membered polycyclic heterocyclyl group in which any two rings share two atoms that are not directly connected, wherein the bridged heterocyclyl may contain one or more double bonds, wherein one or more of the ring atoms is a heteroatom selected from the group consisting of nitrogen, oxygen and sulfur, the sulfur optionally being substituted with oxo (i.e., form sulfoxide or sulfone); and the remaining ring atoms are carbon. The bridged heterocyclyl is preferably 6- to 14-membered, and more preferably 7- to 10-membered (e.g., 7-membered, 8-membered, 9-membered or 10-membered). According to the number of the formed rings, the bridged heterocyclyl may be bicyclic, tricyclic, tetracyclic or polycyclic, preferably bicyclic, tricyclic or tetracyclic, and more preferably bicyclic or tricyclic. Non-limiting examples of bridged heterocyclyl include:

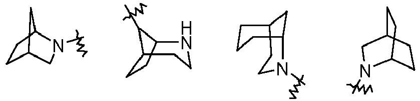

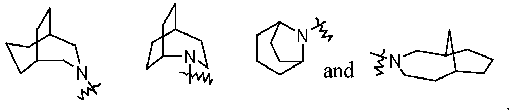

The heterocyclyl ring includes those in which the heterocyclyl described above (including monocyclic, spiro heterocyclic, fused heterocyclic and bridged heterocyclic rings) is fused to an aryl, heteroaryl or cycloalkyl ring, wherein the ring connected to the parent structure is heterocyclyl. Non-limiting examples include: 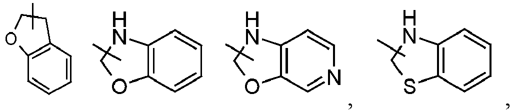 etc.

The heterocyclyl may be substituted or unsubstituted. When substituted, it may be substituted at any accessible connection site, wherein the substituent is preferably one or more substituents independently and optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "aryl" refers to a 6- to 14-membered, preferably 6- to 10-membered carbon monocyclic or fused polycyclic (in which the rings share a pair of adjacent carbon atoms) group having a conjugated π-electron system, such as phenyl and naphthyl. The aryl ring includes those in which the aryl ring described above is fused to a heteroaryl, heterocyclyl or cycloalkyl ring, wherein the ring connected to the parent structure is an aryl ring. Non-limiting examples include:

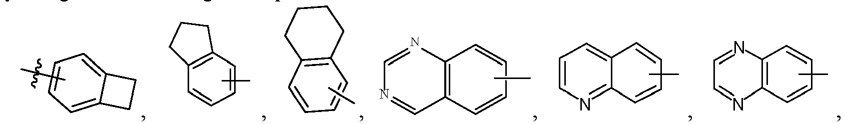

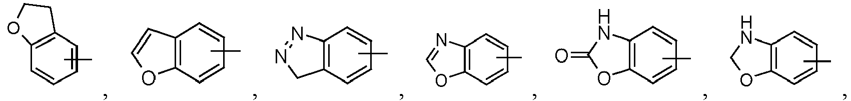

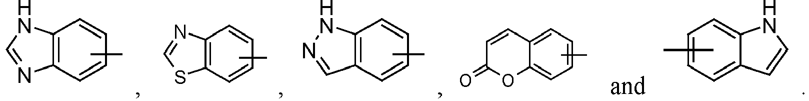

The aryl may be substituted or unsubstituted. When substituted, it may be substituted at any accessible connection site, wherein the substituent is preferably one or more substituents independently and optionally selected from the group consisting of a hydrogen atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The term "heteroaryl" refers to a heteroaromatic system containing 1 to 4 (e.g., 1, 2, 3 and 4) heteroatoms and 5 to 14 ring atoms, wherein the heteroatoms are selected from the group consisting of oxygen, sulfur and nitrogen. The heteroaryl is preferably 5- to 10-membered (e.g., 5-membered, 6-membered, 7-membered, 8-membered, 9-membered or 10-membered) and more preferably 5-membered or 6-membered, e.g., furyl, thienyl, pyridinyl, pyrrolyl, *N-*alkylpyrrolyl, pyrimidinyl, pyrazinyl, pyridazinyl, imidazolyl, pyrazolyl, triazolyl and tetrazolyl. The heteroaryl ring includes those in which the heteroaryl ring described above is fused to an aryl, heterocyclyl or cycloalkyl ring, wherein the ring connected to the parent structure is a heteroaryl ring. Non-limiting examples include:

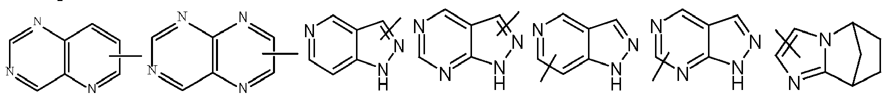

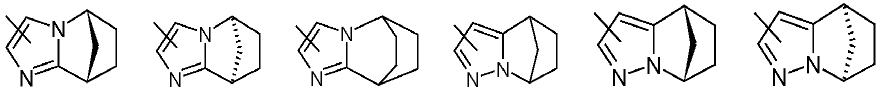

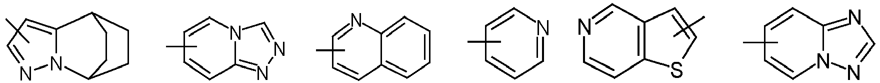

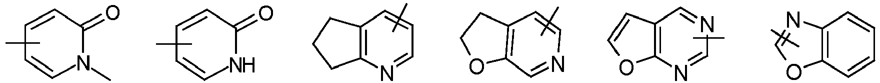

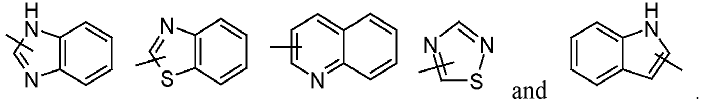

The heteroaryl may be substituted or unsubstituted. When substituted, it may be substituted at any accessible connection site, wherein the substituent is preferably one or more substituents independently and optionally selected from the group consisting of halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl and heteroaryl.

The cycloalkyl, heterocyclyl, aryl and heteroaryl described above include residues derived from the parent ring by removal of one hydrogen atom from a ring atom, or residues derived from the parent ring by removal of two hydrogen atoms from the same ring atom or two different ring atoms, i.e., "divalent cycloalkyl", "divalent heterocyclyl", "arylene" or "heteroarylene".

The term "amino protecting group" refers to a group that can be easily removed and is intended to protect an amino group from being changed when a reaction is conducted elsewhere in the molecule. Non-limiting examples include (trimethylsilyl)ethoxymethyl, tetrahydropyranyl, *tert*-butoxycarbonyl, acetyl, benzyl, allyl, *p*-methoxybenzyl, and the like. These groups may be optionally substituted with 1 to 3 substituents selected from the group consisting of halogen, alkoxy and nitro.

The term "hydroxy protecting group" is a suitable group known in the art for protecting hydroxy. See the hydroxy protecting groups in the literature (*"*Protective Groups in Organic Synthesis", 5th Ed. T.W.Greene & P.G.M.Wuts). As an example, preferably, the hydroxy protecting group may be (C₁₋₁₀ alkyl or aryl)₃silyl, e.g., triethylsilyl, triisopropylsilyl, *tert*-butyldimethylsilyl, *tert*-butyldiphenylsilyl or the like; C₁₋₁₀ alkyl or substituted alkyl, preferably alkoxy or aryl-substituted alkyl, more preferably C₁₋₆ alkoxy-substituted C₁₋₆ alkyl or phenyl-substituted C₁₋₆ alkyl, and most preferably C₁₋₄ alkoxy-substituted C₁₋₄ alkyl, e.g., methyl, *tert*-butyl, allyl, benzyl, methoxymethyl (MOM), ethoxyethyl, 2-tetrahydropyranyl (THP) or the like; (C₁₋₁₀ alkyl or aryl)acyl, e.g., formyl, acetyl, benzoyl, *p*-nitrobenzoyl or the like; (C₁₋₆ alkyl or C₆₋₁₀ aryl)sulfonyl; or (C₁₋₆ alkoxy or C₆₋₁₀ aryloxy)carbonyl.

The term "arylalkyl" refers to aryl-alkylene-, wherein the aryl and alkylene are as defined above.

The term "heteroarylalkyl" refers to heteroaryl-alkylene-, wherein the heteroaryl and alkylene are as defined above.

The term "cycloalkylalkyl" refers to cycloalkyl-alkylene-, wherein the cycloalkyl and alkylene are as defined above.

The term "heterocyclylalkyl" refers to heterocyclyl-alkylene-, wherein the heterocyclyl and alkylene are as defined above.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to alkoxy substituted with one or more halogens, wherein the alkoxy is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as defined above.

The term "halogen" refers to fluorine, chlorine, bromine or iodine.

The term "hydroxy" refers to -OH.

The term "mercapto" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O- or (cycloalkyl)C(O)O-, wherein the alkyl and cycloalkyl are as defined above.

In the chemical structure of the compound of the present disclosure, a bond " 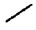 " represents an unspecified configuration, namely if chiral isomers exist in the chemical structure, the bond " 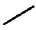 " may be " 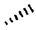 " or " 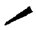 ", or contains both the configurations of " 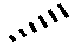 " and " 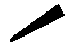 ".

The compound of the present disclosure may also include an isotopic derivative thereof. The term "isotopic derivative" refers to compounds that differ in structure only by having one or more enriched isotopic atoms. For example, compounds with the structure of the present disclosure having "deuterium" or "tritium" in place of hydrogen, or ¹⁸F-fluorine labeling (¹⁸F isotope) in place of fluorine, or ¹¹C-, ¹³C- or ¹⁴C-enriched carbon (¹¹C-, ¹³C- or ¹⁴C-carbon labeling; ¹¹C-, ¹³C- or ¹⁴C-isotope) in place of a carbon atom are within the scope of the present disclosure. Such a compound can be used as an analytical tool or a probe in, for example, a biological assay, or may be used as a tracer for *in vivo* diagnostic imaging of disease, or as a tracer in a pharmacodynamic, pharmacokinetic or receptor study.

The present disclosure also comprises various deuterated forms of the compound of formula (I), (II) or (III) or the compounds in Table A. Each available hydrogen atom connected to a carbon atom may be independently replaced with a deuterium atom. Those skilled in the art are able to synthesize the deuterated forms of the compound of formula (I), (II) or (III) or the compounds in Table A with reference to the relevant literature. Commercially available deuterated starting materials can be used in preparing the deuterated forms of the compound of formula (I), (II) or (III) or the compounds in Table A, or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like. Deuterides can generally retain comparable activity to non-deuterated compounds and can achieve better metabolic stability when deuterated at certain specific sites, thereby achieving certain therapeutic advantages. The term "optional" or "optionally" means that the event or circumstance subsequently described may, but not necessarily, occur, and that the description includes instances where the event or circumstance occurs or does not occur. For example, the expression "a heterocyclyl group optionally substituted with alkyl" means that the alkyl may be, but not necessarily, present, and includes instances where the heterocyclyl group is or is not substituted with the alkyl.

The term "substituted" means that one or more, preferably 1-5, more preferably 1-3 hydrogen atoms in the group are independently substituted with a corresponding number of substituents. Those skilled in the art are able to determine (experimentally or theoretically) possible or impossible substitution without undue efforts. For example, it may be unstable when an amino or hydroxy group having a free hydrogen is bound to a carbon atom having an unsaturated (e.g., olefinic) bond.

The term "pharmaceutical composition" refers to a mixture containing one or more of the compounds described herein or a physiologically/pharmaceutically acceptable salt or pro-drug thereof, and other chemical components, and other components, for example, physiologically/pharmaceutically acceptable carriers and excipients. The pharmaceutical composition is intended to promote the administration to an organism, so as to facilitate the absorption of the active ingredient, thereby exerting biological activities.

The term "pharmaceutically acceptable salt" refers to the salts of the compound of the present disclosure, which are safe and effective for use in the body of a mammal and possess the requisite biological activities. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacological active agents, the term "therapeutically effective amount" refers to an amount of a medicament or an agent that is sufficient to provide the desired effect but is non-toxic. The determination of the effective amount varies from person to person. It depends on the age and general condition of a subject, as well as the particular active substance used. The appropriate effective amount in a case may be determined by those skilled in the art in the light of routine tests.

The term "pharmaceutically acceptable" used herein means that those compounds, materials, compositions and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration and temperature, it means that the parameter may vary by ±10%, and sometimes more preferably within ±5%. As will be appreciated by those skilled in the art, when the parameters are not critical, the numbers are generally given for illustrative purposes only and are not intended to be limiting.

### Synthesis Method of Compounds of the Present Disclosure

To achieve the purpose of the present disclosure, the following technical schemes are adopted in the present disclosure: 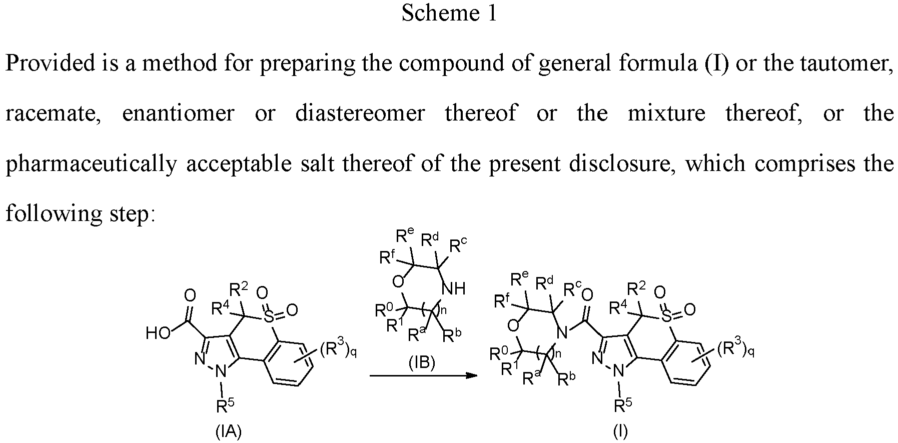 subjecting a compound of general formula (IA) or a tautomer, racemate, enantiomer or diastereomer thereof or a mixture thereof, or a pharmaceutically acceptable salt thereof to a reaction with a compound of general formula (IB) or a pharmaceutically acceptable salt thereof (preferably hydrochloride) under alkaline conditions, optionally in the presence of a condensing agent, to give the compound of general formula (I) or the tautomer, racemate, enantiomer or diastereomer thereof or the mixture thereof, or the pharmaceutically acceptable salt thereof,
wherein R⁰-R⁵, R^{a}-R^{f}, n and q are as defined in general formula (I). 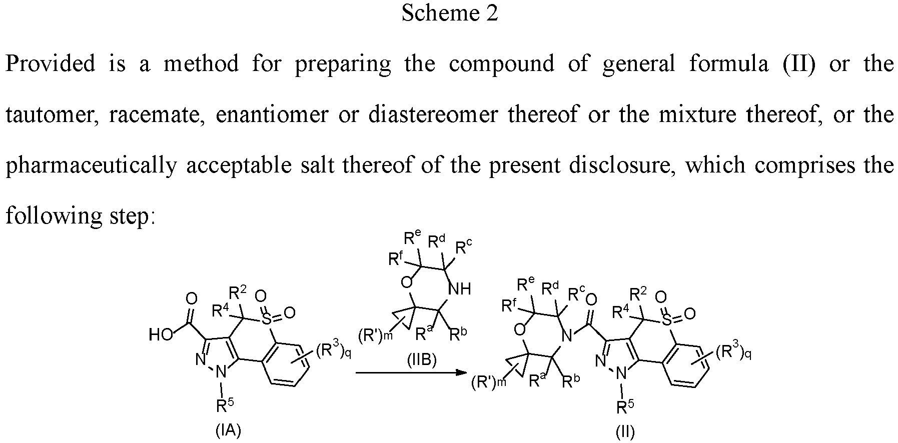
subjecting a compound of general formula (IA) or a tautomer, racemate, enantiomer or diastereomer thereof or a mixture thereof, or a pharmaceutically acceptable salt thereof to a reaction with a compound of general formula (IIB) or a pharmaceutically acceptable salt thereof (preferably hydrochloride) under alkaline conditions, optionally in the presence of a condensing agent, to give the compound of general formula (II) or the tautomer, racemate, enantiomer or diastereomer thereof or the mixture thereof, or the pharmaceutically acceptable salt thereof,
wherein R', R²-R⁵, R^{a}-R^{f}, m and q are as defined in general formula (II). 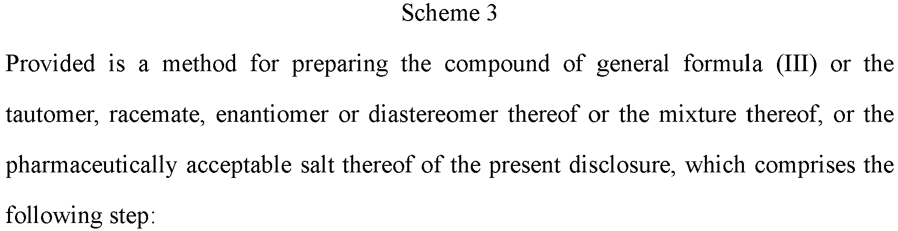 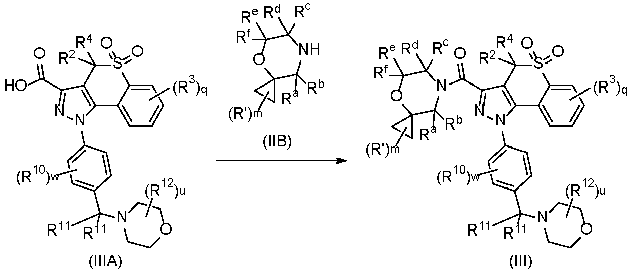
subjecting a compound of general formula (IIIA) or a tautomer, racemate, enantiomer or diastereomer thereof or a mixture thereof, or a pharmaceutically acceptable salt thereof to a reaction with general formula (IIB) or a pharmaceutically acceptable salt thereof (preferably hydrochloride) under alkaline conditions, optionally in the presence of a condensing agent, to give the compound of general formula (III) or the tautomer, racemate, enantiomer or diastereomer thereof or the mixture thereof, or the pharmaceutically acceptable salt thereof,
wherein R', R²-R⁴, R^{a}-R^{f}, R¹⁰-R¹², q, u, w and m are as defined in general formula (III). The reagents that provide alkaline conditions in the above reactions include organic and inorganic bases, wherein the organic bases include, but are not limited to, triethylamine, 4-dimethylaminopyridine, *N,N-*diisopropylethylamine, *n*-butyllithium, lithium diisopropylamide, potassium acetate, sodium *tert*-butoxide, potassium *tert*-butoxide or 1,8-diazabicycloundec-7-ene; and the inorganic bases include, but are not limited to, sodium hydride, potassium phosphate, sodium carbonate, sodium acetate, potassium acetate, potassium carbonate, cesium carbonate, sodium hydroxide, lithium hydroxide and potassium hydroxide. The regents are preferably *N,N-*diisopropylethylamine and/or 4-dimethylaminopyridine, and more preferably a combination of *N,N-*diisopropylethylamine and 4-dimethylaminopyridine.

The condensing agent described in the above reactions includes, but is not limited to, 1-(3-dimethylaminopropyl)-3-ethylcarbodiimide hydrochloride, *N,N*'-dicyclohexylcarbodiimide, *N,N*'-diisopropylcarbodiimide, *O*-benzotriazol-*N*,*N*,*N*',*N*'-tetramethyluronium tetrafluorob orate, 1 -hydroxybenzotriazole, 1-hydroxy-7-azobenzotriazol, *O*-benzotriazol-*N,N,N',N*'-tetramethyluronium hexafluorophosphate, 2-(7-azabenzotriazol)-*N,N,N',N*'-tetramethyluronium hexafluorophosphate (HATU, also known as *N,N,N',N*'-tetramethyl-*O*-(7-azabenzotriazol-1-yl)uronium hexafluorophosphate or *O*-(7-azabenzotriazol)-*N,N,N',N*'-tetramethyluronium hexafluorophosphate), 2-(7-benzotriazol oxide)-*N,N,N',N*'-tetramethyluronium hexafluorophosphate, benzotriazol-1-yloxytris(dimethylamino)phosphonium hexafluorophosphate or benzotriazol-1-yl-oxytripyrrolidinylphosphine hexafluorophosphate; it is preferably 1-(3-dimethylaminopropyl)-3-ethylcarbodiimide hydrochloride and/or 1-hydroxybenzotriazole, or HATU alone, and more preferably a combination of 1-(3-dimethylaminopropyl)-3-ethylcarbodiimide hydrochloride and 1-hydroxybenzotriazole.

The above reactions are preferably performed in solvents including, but not limited to acetic acid, methanol, ethanol, acetonitrile, *n*-butanol, toluene, tetrahydrofuran, dichloromethane, petroleum ether, ethyl acetate, *n*-hexane, dimethyl sulfoxide, 1,4-dioxane, ethylene glycol dimethyl ether, water, toluene, xylene, pyridine, dioxan, N,N-dimethylacetamide, *N,N*-dimethylformamide and mixtures thereof.

### DETAILED DESCRIPTION

The following examples further illustrate the present disclosure, but the present disclosure is not limited thereto.

### Examples

The structure of the compound is determined by nuclear magnetic resonance (NMR) spectroscopy and/or mass spectrometry (MS). NMR shift (δ) is given in a unit of 10⁻⁶ (ppm). NMR spectra were measured using a Bruker AVANCE NEO 500M nuclear magnetic resonance instrument, with deuterated dimethyl sulfoxide (DMSO-*d₆*), deuterated chloroform (CDCl₃) and deuterated methanol (CD₃OD) as determination solvents and tetramethylsilane (TMS) as internal standard.

Mass spectra were measured using Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters, MS model: Waters ACQuity Qda Detector/Waters SQ Detector) and THERMO Ultimate 3000-Q Exactive (manufacturer: THERMO, MS model: THERMO Q Exactive).

High performance liquid chromatography (HPLC) analysis is performed using Agilent HPLC 1200DAD, Agilent HPLC 1200VWD and Waters HPLC e2695-2489 high performance liquid chromatographs.

Chiral HPLC analysis is performed using an Agilent 1260 DAD high performance liquid chromatograph.

High performance liquid preparative chromatography is performed using Waters 2545-2767, Waters 2767-SQ Detecor2, Shimadzu LC-20AP and Gilson GX-281 preparative chromatographs.

Chiral preparative HPLC is performed using a Shimadzu LC-20AP preparative chromatograph.

CombiFlash rapid preparation instrument used is CombiFlash Rf200 (TELEDYNE ISCO).

Huanghai HSGF254 or Qingdao GF254 silica gel plates of specifications 0.15 mm to 0.2 mm are adopted for thin layer chromatography (TLC) analysis and 0.4 mm to 0.5 mm for TLC separation and purification.

The silica gel column chromatography generally used 200 to 300-mesh silica gel (Huanghai, Yantai) as the carrier.

The mean inhibition of kinase and the IC₅₀ value are determined using a NovoStar microplate reader (BMG, Germany).

Known starting materials described herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can be performed in an argon atmosphere or a nitrogen atmosphere unless otherwise specified.

The argon atmosphere or nitrogen atmosphere means that the reaction flask is connected to a balloon containing about 1 L of argon or nitrogen.

The hydrogen atmosphere means that the reaction flask is connected to a balloon containing about 1 L of hydrogen.

Parr 3916EKX hydrogenator, Qinglan QL-500 hydrogenator or HC2-SS hydrogenator was used in the pressurized hydrogenation reactions.

The hydrogenation reactions usually involve 3 cycles of vacuumization and hydrogen purge.

A CEM Discover-S 908860 microwave reactor is used in the microwave reactions.

In the examples, a solution refers to an aqueous solution unless otherwise specified.

In the examples, the reaction temperature is room temperature, i.e., 20 °C to 30 °C, unless otherwise specified.

The monitoring of the reaction progress in the examples was conducted by thin layer chromatography (TLC). The developing solvent for reactions, the eluent system for column chromatography purification and the developing solvent system for thin layer chromatography included: A: dichloromethane/methanol system. The volume ratio of the solvents was adjusted according to the polarity of the compound, or by adding a small amount of basic or acidic reagents such as triethylamine and acetic acid.

### Example 1

### (6-fluoro-1-(4-(morpholinomethyl)phenyl)-5, 5-dioxido-1,4-dihydrothiochromeno[4,3-c] pyrazol-3-yl)(4-oxa-7-azaspiro[2.5]octan-7-yl)methanone 1

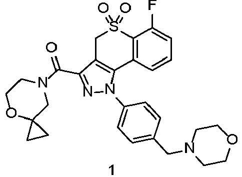

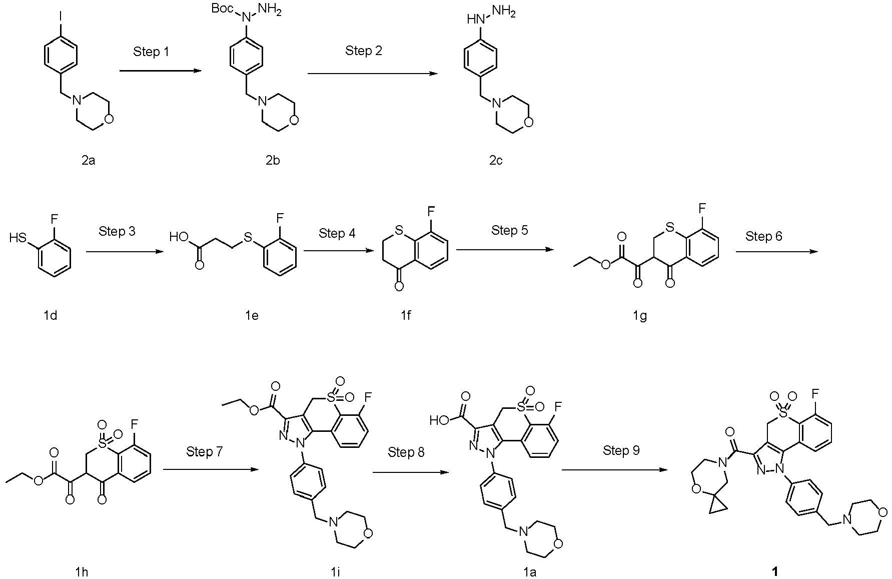

### Step 1

### Tert-butyl 1-(4-(morpholinomethyl)phenyl)hydrazine-1-carboxylate 2b

Compound 4-(4-iodobenzyl)morpholine **2a** (51 g, 168.24 mmol, prepared according to the method disclosed in Example 17.1 (page 59) in the specification of the patent application WO200832191A2) and *tert*-butyl carbazate (23.347 g, 176.66 mmol, Accela ChemBio) were dissolved in dimethyl sulfoxide (400 mL) under argon atmosphere, and the mixture was stirred for 10 min, followed by addition of cuprous iodide (1.603 g, 8.42 mmol). The resulting mixture was warmed to 50 °C and stirred for 17 h. Water (400 mL) was added, and the aqueous phase was extracted with ethyl acetate (300 mL × 6). The organic phases were combined and concentrated under reduced pressure, and the residue was purified by a CombiFlash rapid preparation instrument with eluent system A to give the title compound **2b** (51 g, yield: 98.6%).

MS m/z (ESI): 308.1 [M+1].

### Step 2

### Hydrochloride of 4-(4-hydrazinobenzyl)morpholine 2c

Compound **2b** (51 g, 165.91 mmol) was dissolved in methanol (80 mL) at 0 °C, and a solution of hydrogen chloride in 1,4-dioxane (350 mL, 4.0 M, Infinity Scientific) was added dropwise. The mixture was naturally warmed to room temperature and stirred for 17 h. Then the mixture was concentrated under reduced pressure to give crude hydrochloride of the title compound **2c** (45.4 g), which was directly used in the next step without purification.

### Step 3

### 3-((2-fluorophenyl)thio)propanoic acid 1e

2-fluorobenzenethiol **1d** (50 g, 390.11 mmol, Accela ChemBio) and potassium carbonate (70 g, 507.14 mmol, Sinopharm) were dissolved in *N,N*-dimethylformamide (500 mL), and the mixture was stirred at 60 °C for 1 h, followed by addition of 3-bromopropionic acid (65.6 g, 429.15 mmol, Accela ChemBio). The resulting mixture was reacted at 60 °C for 3 h. After the reaction was completed, water (1000 mL) was added to the reaction solution, and ethyl acetate (300 mL × 2) was added for extraction. The aqueous phase was adjusted to pH of about 3 with concentrated hydrochloric acid and extracted with ethyl acetate (400 mL × 3). The organic phases were combined, washed successively with water (400 mL × 3) and saturated brine (400 mL × 2), dried over anhydrous sodium sulfate for 15 min, filtered and concentrated under reduced pressure to give crude title compound **1e** (70 g), which was directly used in the next step without purification.

MS m/z (ESI): 198.9 [M-1].

### Step 4

### 8-fluorothiochroman-4-one 1f

Compound **1e** (70 g, 356.65 mmol) was dissolved in concentrated sulfuric acid (200 mL), and the mixture was stirred at 0 °C for 3 h. After the reaction was completed, the reaction solution was poured into ice water (1000 mL), and ethyl acetate (400 mL × 3) was added for extraction. The organic phases were combined, washed with saturated brine (400 mL × 2), dried over anhydrous sodium sulfate for 15 min and filtered, and the filtrate was concentrated under reduced pressure to give crude title compound **1f** (30 g), which was directly used in the next step without purification.

### Step 5

### Ethyl 2-(8-fluoro-4-oxothiochroman-3-yl)-2-oxoacetate 1g

Sodium ethoxide (52 g, 152.59 mmol, 20% w/w ethanol solution, Adamas) was added to a 500 mL three-neck flask, and diethyl oxalate (16.7 g, 114.47 mmol, dissolved in 100 mL of toluene, Accela ChemBio) was slowly added dropwise at 0 °C, followed by addition of compound **1f** (13.9 g, 76.28 mmol, dissolved in 100 mL of toluene). The reaction solution was concentrated under reduced pressure, water (400 mL) was added to the residue, and dichloromethane (200 mL × 2) was added for extraction. The aqueous phase was adjusted to pH of about 2 with 5 M hydrochloric acid solution and extracted with ethyl acetate (250 mL × 3). The organic phases were combined, washed with saturated brine (200 mL × 2), dried over anhydrous sodium sulfate for 15 min and filtered, and the filtrate was concentrated under reduced pressure to give crude title compound **1g** (22 g), which was directly used in the next step without purification.

MS m/z (ESI): 280.9 [M-1].

### Step 6

### Ethyl 2-(8-fluoro-1,1-dioxido-4-oxothiochroman-3-yl)-2-oxoacetate 1h

Compound **1g** (22 g, 77.93 mmol) was dissolved in dichloromethane (250 mL), and 3-chloroperoxybenzoic acid (34.8 g, 171.46 mmol, Ourchem) was added in portions under an ice bath. The mixture was stirred at room temperature for 17 h. Then the mixture was filtered, the filtrate was concentrated under reduced pressure, and the residue was purified by a CombiFlash rapid preparation instrument with eluent system A to give the title compound **1h** (24.5 g, yield: 100%).

MS m/z (ESI): 313.0 [M-1].

### Step 7

### Ethyl 6-fluoro-1-(4-(morpholinomethyl)phenyl)-1,4-dihydrothiochromeno[4,3-c]pyrazole-3-c arboxylate 5,5-dioxide 1i

Compound **1h** (9.4 g, 29.90 mmol), the hydrochloride of compound **2c** (6.8 g, 75%) and glacial acetic acid (3.6 g, 59.94 mmol, Hushi) were dissolved in absolute ethanol (200 mL), and the mixture was heated to reflux and stirred for 3 h. Saturated sodium bicarbonate solution (300 mL) was added, and the mixed solution was extracted with ethyl acetate (250 mL × 3). The organic phases were combined and concentrated under reduced pressure, and the residue was purified by a CombiFlash rapid preparation instrument with eluent system A to give the title compound **1i** (7.5 g, yield: 51.6%).

MS m/z (ESI): 486.1 [M+1].

### Step 8

### 6-fluoro-1-(4-(morpholinomethyl)phenyl)-1,4-dihydrothiochromeno[4,3-c]pyrazole-3-c arboxylic acid 5,5-dioxide 1a

Compound **1i** (14 g, 29.07 mmol) was dissolved in tetrahydrofuran (150 mL), and aqueous sodium hydroxide solution (58.2 mL, 2 M) was added. The mixture was stirred for 4 h. Concentrated hydrochloric acid solution was added to adjust the pH to about 3, and the resulting mixture was concentrated under reduced pressure to give crude title compound **1a** (21.2 g), which was directly used in the next step without purification.

MS m/z (ESI): 458.0 [M+1].

### Step 9

### (6-fluoro-1-(4-(morpholinomethyl)phenyl)-5,5-dioxido-1,4-dihydrothiochromeno[4,3-c] pyrazol-3-yl)(4-oxa-7-azaspiro[2.5]octan-7-yl)methanone 1

Compound **1a** (100 mg, 218.59 mmol), 4-oxa-7-azaspiro[2.5]octane hydrochloride (33 mg, 220.77 mmol, Jiangsu Aikon), 1-(3-dimethylaminopropyl)-3-ethylcarbodiimide hydrochloride (251 mg, 651.77 mmol), 1-hydroxybenzotriazole (99 mg, 655.77 mmol), *N*,*N*-diisopropylethylamine (110 mg, 1.09 mmol) and 4-dimethylaminopyridine (53 mg, 437.18 mmol) were dissolved in dichloromethane (30 mL), and the mixture was stirred at room temperature for 16 h. Water (50 mL) was added, and a mixed solvent (60 mL) of dichloromethane and methanol (v:v = 10:1) was added for extraction. The organic phases were combined, washed successively with water (60 mL) and saturated brine (60 mL), dried over anhydrous sodium sulfate and filtered, the filtrate was concentrated under reduced pressure, and the resulting residue was purified by a CombiFlash rapid preparation instrument with eluent system A to give the title product **1** (60 mg, yield: 49.6%).

MS m/z (ESI): 553.3 [M+1].

¹H NMR (500 MHz, DMSO-*d*₆): *δ* 7.62-7.44 (m, 6H), 6.66 (d, 1H), 4.99 (d, 2H), 4.07 (t, 1H), 3.90 (s, 1H), 3.76-3.59 (m, 9H), 3.32 (s, 1H), 2.42 (s, 4H), 0.73-0.60 (m, 4H).

### Example 2

### (6-chloro-1-(4-(morpholinomethyl)phenyl)-5,5-dioxido-1,4-dihydrothiochromeno[4,3-c ]pyrazol-3-yl)(4-oxa-7-azaspiro[2.5]octan-7-yl)methanone 2

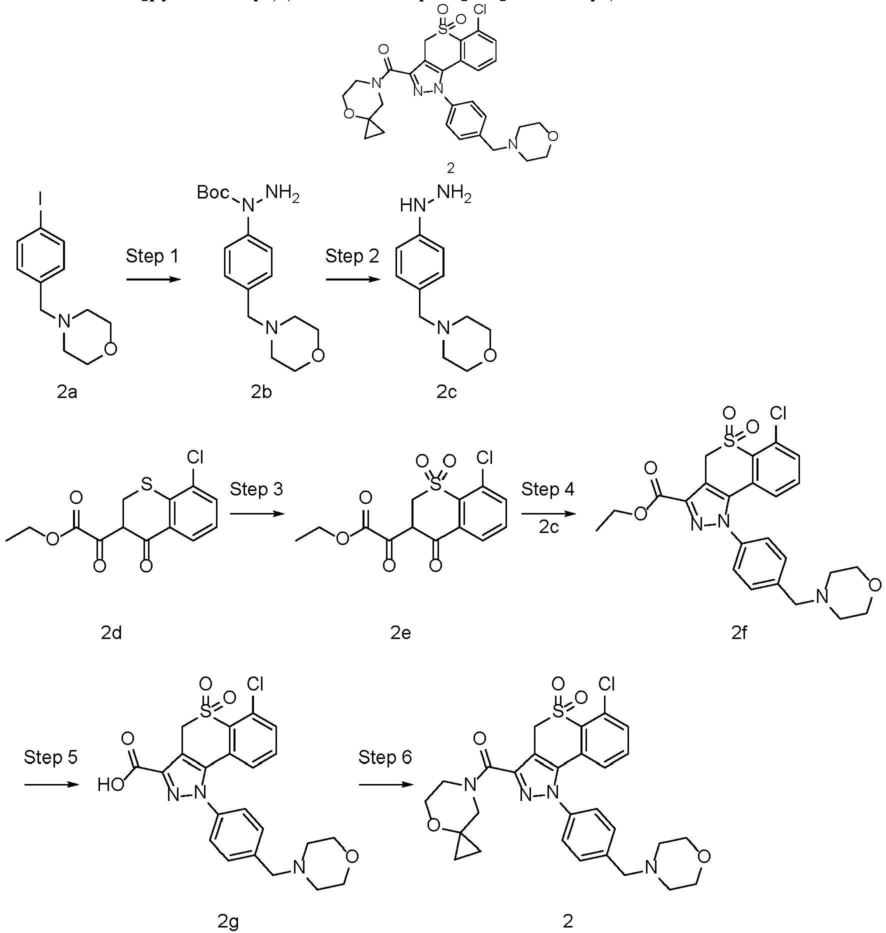

### Step 1

### Tert-butyl 1-(4-(morpholinomethyl)phenyl)hydrazine-1-carboxylate 2b

Compound 4-(4-iodobenzyl)morpholine **2a** (51 g, 168.24 mmol, prepared according to the method disclosed in Example 17.1 (page 59) in the specification of the patent application WO200832191A2) and *tert*-butyl carbazate (23.347 g, 176.66 mmol, Accela ChemBio) were dissolved in dimethyl sulfoxide (400 mL) under argon atmosphere, and the mixture was stirred for 10 min, followed by addition of cuprous iodide (1.603 g, 8.42 mmol). The resulting mixture was warmed to 50 °C and stirred for 17 h. Water (400 mL) was added, and the aqueous phase was extracted with ethyl acetate (300 mL × 6). The organic phases were combined and concentrated under reduced pressure, and the residue was purified by a CombiFlash rapid preparation instrument with eluent system A to give the title compound **2b** (51 g, yield: 98.6%).

MS m/z (ESI): 308.1 [M+1].

### Step 2

### Hydrochloride of 4-(4-hydrazinobenzyl)morpholine 2c

Compound **2b** (51 g, 165.91 mmol) was dissolved in methanol (80 mL) at 0 °C, and a solution of hydrogen chloride in 1,4-dioxane (350 mL, 4.0 M, Infinity Scientific) was added dropwise. The mixture was naturally warmed to room temperature and stirred for 17 h. Then the mixture was concentrated under reduced pressure to give crude hydrochloride of the title compound **2c** (45.4 g), which was directly used in the next step without purification.

### Step 3

### Ethyl 2-(8-chloro-1,1-dioxido-4-oxothiochroman-3-yl)-2-oxoacetate 2e

Compound ethyl 2-(8-chloro-4-oxothiochroman-3-yl)-2-oxoacetate **2d** (25.7 g, 86.03 mmol, prepared according to the disclosed method for intermediate E4 (page 169) in the specification of the patent "CN102695710B") was dissolved in dichloromethane (250 mL), and 3-chloroperoxybenzoic acid (43.664 g, 215.07 mmol) was added in portions under an ice bath. The mixture was stirred at room temperature for 17 h. Then the mixture was filtered, the filtrate was concentrated under reduced pressure, and the residue was purified by a CombiFlash rapid preparation instrument with eluent system A to give the title compound **2e** (28.323 g, yield: 99.5%).

MS m/z (ESI): 330.9 [M+1].

### Step 4

### Ethyl 6-chloro-1-(4-(morpholinomethyl)phenyl)-1,4-dihydrothiochromeno[4,3-c]pyrazole-3-c arboxylate 5,5-dioxide 2f

Compound **2e** (10 g, 30.24 mmol), the hydrochloride of compound **2c** (9.192 g, 75%) and glacial acetic acid (3.632 g, 60.48 mmol, Hushi) were dissolved in absolute ethanol (300 mL), and the mixture was heated to reflux and stirred for 3 h. Saturated sodium bicarbonate solution (300 mL) was added, and the mixed solution was extracted with ethyl acetate (250 mL × 3). The organic phases were combined and concentrated under reduced pressure, and the residue was purified by a CombiFlash rapid preparation instrument with eluent system A to give the title compound **2f** (10 g, yield: 65.9%).

MS m/z (ESI): 502.0 [M+1].

### Step 5

### 6-chloro-1-(4-(morpholinomethyl)phenyl)-1,4-dihydrothiochromeno[4,3-c]pyrazole-3-c arboxylic acid 5,5-dioxide 2g

Compound **2f** (10 g, 19.92 mmol) was dissolved in tetrahydrofuran (150 mL), and aqueous sodium hydroxide solution (39.8 mL, 2.5 M) was added. The mixture was stirred for 4 h. Concentrated hydrochloric acid solution was added to adjust the pH to about 3, and the resulting mixture was concentrated under reduced pressure to give crude title compound **2g** (17.028 g), which was directly used in the next step without purification.

MS m/z (ESI): 474.0 [M+1].

### Step 6

### (6-chloro-1-(4-(morpholinomethyl)phenyl)-5,5-dioxido-1,4-dihydrothiochromeno[4,3-c ]pyrazol-3-yl)(4-oxa-7-azaspiro[2.5]octan-7-yl)methanone 2

Compound **2g** (544 mg, 633.61 µmol, 55.2%), 4-oxa-7-azaspiro[2.5]octane hydrochloride (97 mg, 648.32 µmol, PharmaBlock), HATU (290 mg, 762.70 µmol) and *N*,*N*-diisopropylethylamine (411 mg, 3.18 mmol) were dissolved in *N*,*N*-dimethylformamide (60 mL), and the mixture was stirred at room temperature for 17 h. Saturated sodium bicarbonate solution (50 mL) was added, and the aqueous phase was extracted with ethyl acetate (50 mL × 3). The organic phases were combined and concentrated under reduced pressure, and the resulting residue was purified by a CombiFlash rapid preparation instrument with eluent system A to give the title product **2** (108 mg, yield: 30.0%).

MS m/z (ESI): 569.0 [M+1].

¹H NMR (500 MHz, DMSO-*d*₆): *δ* 7.66-7.64 (m, 1H), 7.54-7.40 (m, 5H), 6.83-6.81 (m, 1H), 5.02-5.00 (m, 2H), 4.08 (s, 1H), 3.92 (s, 1H), 3.75-3.58 (m, 10H), 2.46-2.41 (m, 4H), 0.73-0.61 (m, 4H).

### Example 3

### (9-methoxy-1-(4-(morpholinomethyl)phenyl)-5, 5-dioxido-1,4-dihydrothiochromeno[4,3 -c]pyrazol-3-yl)(4-oxa-7-azaspiro[2.5]octan-7-yl)methanone 3

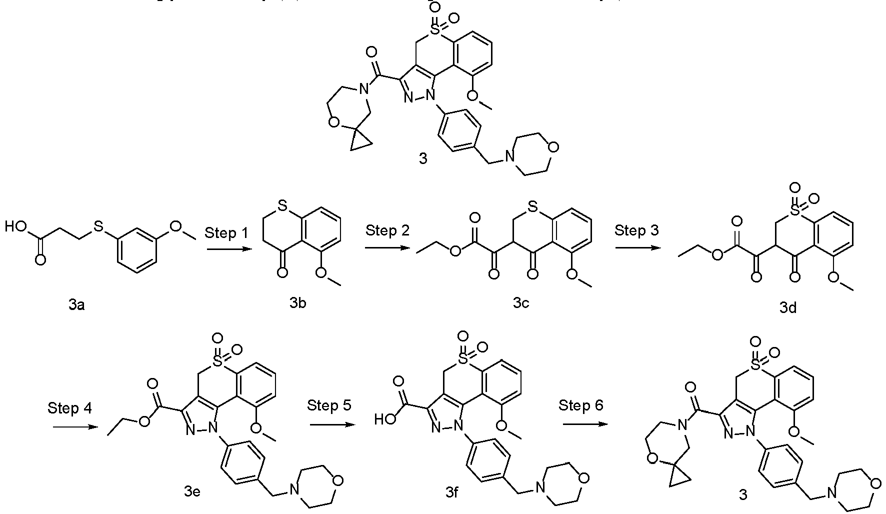

### Step 1

### 5-methoxythiochroman-4-one 3b

3-(3-methoxyphenylthio)formic acid **3a** (12 g, 56.53 mmol, prepared according to the method disclosed in "Organic Letters, 2020, 22(3), 1155-1159") and sulfuric acid (40 mL) were added into a 100 mL one-neck flask, and the mixture was stirred at room temperature for 3 h. The reaction solution was poured into ice water (100 mL), and ethyl acetate (100 mL × 3) was added for extraction. The organic phase was washed with saturated brine (100 mL × 2), dried over anhydrous sodium sulfate and filtered, and the filtrate was concentrated under reduced pressure to give the target product **3b** (300 mg, yield: 2.73%).

### Step 2

### Ethyl 2-(5-methoxy-4-oxothiochroman-3-yl)-2-oxoacetate 3c

Sodium ethoxide (1.44 g, 4.23 mmol, 20% w/w ethanol solution) was dissolved in toluene (20 mL), and the mixture was cooled to 0 °C. A solution of diethyl oxalate (463 mg, 3.166 mmol) in toluene (20 mL) was added dropwise, followed by the addition of compound **3b** (410 mg, 2.11 mmol). The resulting mixture was reacted at room temperature for 17 h. The reaction solution was concentrated under reduced pressure, water (100 mL) was added to the residue, and dichloromethane (50 mL) was added for extraction. The aqueous phase was adjusted to pH of about 2 with 5 M hydrochloric acid solution and extracted with ethyl acetate (50 mL × 3). The organic phases were combined, washed with saturated brine (20 mL × 2), dried over anhydrous sodium sulfate and filtered, and the filtrate was concentrated under reduced pressure to give the title compound 3c (900 mg), which was directly used in the next step without purification.

### Step 3

### Ethyl 2-(5-methoxy-1,1-dioxido-4-oxothiochroman-3-yl)-2-oxoacetate 3d

Crude compound **3c** (900 mg, 3.058 mmol) was dissolved in dichloromethane (30 mL), and 3-chloroperoxybenzoic acid (1.2 g, 6.95 mmol) was added. The mixture was stirred at room temperature for 17 h. Then the mixture was concentrated under reduced pressure, and the residue was purified by a CombiFlash rapid preparation instrument with eluent system B to give the title compound **3d** (550 mg, yield: 55.1%).

### Step 4

### Ethyl 9-methoxy-1-(4-(morpholinomethyl)phenyl)-1,4-dihydrothiochromeno[4,3-c]pyrazole-3 -carboxylate 5,5-dioxide 3e

Compound **3d** (550 mg, 1.68 mmol) was dissolved in ethanol (30 mL), and the hydrochloride of compound **2c** (384 mg) and glacial acetic acid (203 mg, 3.3804 mmol) were added. The mixture was stirred at 90 °C for 2 h. Then the mixture was concentrated under reduced pressure, slurried with ethanol and filtered, and the filter cake was dried to give the title product **3e** (700 mg, yield: 83.4%).

### Step 5

### 9-methoxy-1-(4-(morpholinomethyl)phenyl)-1,4-dihydrothiochromeno[4,3-c]pyrazole-3 -carboxylic acid 5,5-dioxide 3f

Compound **3e** (10.2 g, 20.5 mmol) was dissolved in tetrahydrofuran (50 mL), and aqueous sodium hydroxide solution (3 M, 2.8 mL) was added. The mixture was stirred at room temperature for 4 h. The reaction solution was adjusted to pH of about 2 with 5.0 M hydrochloric acid solution and concentrated under reduced pressure to give crude title product **3f** (1 g, purity: 60%), which was directly used in the next step without purification.

### Step 6

### (9-methoxy-1-(4-(morpholinomethyl)phenyl)-5,5-dioxido-1,4-dihydrothiochromeno[4,3 -c]pyrazol-3-yl)(4-oxa-7-azaspiro[2.5]octan-7-yl)methanone 3

Crude compound **3f** (300 mg, 332.26 µmol, purity: 60%), 4-oxa-7-azaspiro[2.5]octane hydrochloride (50 mg, 334.18 µmol, PharmaBlock), HATU (152 mg, 399.76 µmol) and *N,N-*diisopropylethylamine (215 mg, 1.66 mmol) were dissolved in *N*,*N*-dimethylformamide (60 mL), and the mixture was stirred at room temperature for 17 h. Saturated sodium bicarbonate solution (50 mL) was added, and the aqueous phase was extracted with ethyl acetate (50 mL × 3). The organic phases were combined and concentrated under reduced pressure, and the resulting residue was purified by a CombiFlash rapid preparation instrument with eluent system A to give the title product **3** (40 mg, yield: 21.3%).

MS m/z (ESI): 565.0 [M+1].

¹H NMR (500 MHz, CDCl₃) *δ* 7.77-7.75 (m, 1H), 7.59-7.56 (m, 1H), 7.42-7.39(m, 2H), 7.35-7.33 (m, 1H), 7.31-7.30 (m, 1H), 7.03-7.01 (m, 1H), 4.73-4.71 (m, 2H), 4.33-4.32 (m, 1H), 4.19 (s, 1H), 3.89-3.82 (m, 3H), 3.79-3.74 (m, 5H), 3.57 (s, 2H), 3.13 (s, 3H), 2.50-2.48 (m, 4H), 0.87-0.85 (m, 2H), 0.74-0.72 (m, 2H).

### Example 4

### (7-methoxy-1-(4-(morpholinomethyl)phenyl)-5,5-dioxido-1,4-dihydrothiochromeno[4,3 -c]pyrazol-3-yl)(4-oxa-7-azaspiro[2.5]octan-7-yl)methanone 4

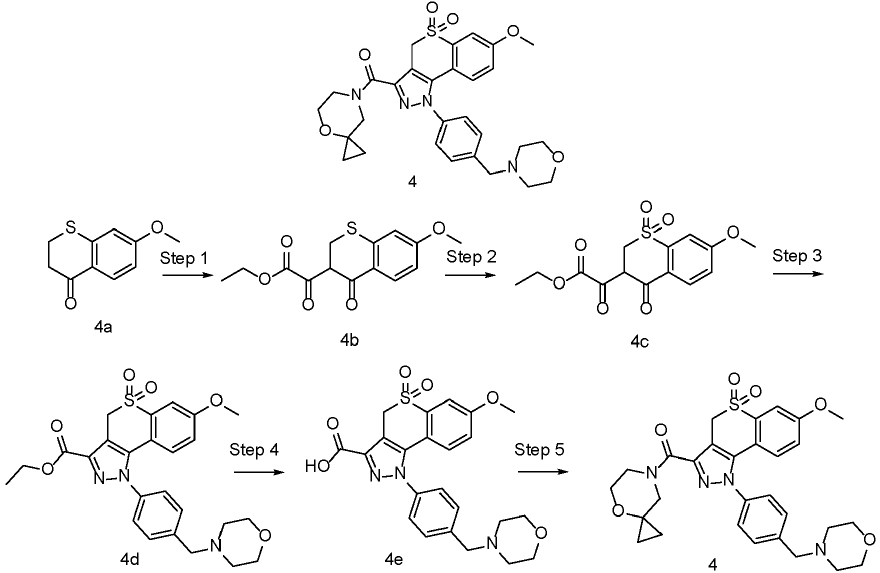

### Step 1

### Ethyl 2-(7-methoxy-4-oxothiochroman-3-yl)-2-oxoacetate 4b

Sodium ethoxide (19.267 g, 56.62 mmol, 20% w/w ethanol solution) was added into a 500 mL single-neck flask, and a solution of diethyl oxalate (6.207 g, 42.47 mmol) in toluene (300 mL) was added at 0 °C, followed by addition of 7-methoxythiochroman-4-one **4a** (5.5 g, 28.31 mmol, prepared according to the method disclosed in "Organic Letters, 2020, 22(3), 1155-1159"). The mixture was stirred at room temperature for 17 h. The reaction solution was concentrated under reduced pressure, water (400 mL) was added to the residue, and dichloromethane (200 mL × 2) was added for extraction. The aqueous phase was adjusted to pH of about 2 with 5 M hydrochloric acid solution and extracted with ethyl acetate (200 mL × 3). The organic phases were combined, washed with saturated brine (200 mL × 2), dried over anhydrous sodium sulfate and filtered, and the filtrate was concentrated under reduced pressure to give crude title compound **4b** (8.3 g), which was directly used in the next step without purification.

### Step 2

### Ethyl 2-(7-methoxy-1,1-dioxido-4-oxothiochroman-3-yl)-2-oxoacetate 4c

Crude compound **4b** (8.3 g, 28.20 mmol) was dissolved in dichloromethane (200 mL), and 3-chloroperoxybenzoic acid (12.166 g, 70.50 mmol) was added in portions under an ice bath. The mixture was stirred at room temperature for 17 h. Then the mixture was filtered, the filtrate was concentrated under reduced pressure, and the residue was purified by a CombiFlash rapid preparation instrument with eluent system B to give the title compound **4c** (8.8 g, yield: 95.6%).

### Step 3

### Ethyl 7-methoxy-1-(4-(morpholinomethyl)phenyl)-1,4-dihydrothiochromeno[4,3-c]pyrazole-3 -carboxylate 5,5-dioxide 4d

Compound **4c** (8.8 g, 26.96 mmol) was dissolved in ethanol (200 mL), and the hydrochloride of **2c** (6.7 g) and glacial acetic acid (3.239 g, 53.93 mmol) were added. The mixture was stirred at 90 °C for 2 h. Then the mixture was concentrated under reduced pressure, slurried with ethanol and filtered, and the filter cake was dried to give the title product **4d** (10.2 g, yield: 76.0%).

### Step 4

### 7-methoxy-1-(4-(morpholinomethyl)phenyl)-1,4-dihydrothiochromeno[4,3-c]pyrazole-3 -carboxylic acid 5,5-dioxide 4e

Compound **4d** (10.2 g, 20.5 mmol) was dissolved in tetrahydrofuran (100 mL), and aqueous sodium hydroxide solution (1.0 M, 102.5 mL) was added. The mixture was stirred at room temperature for 4 h. The reaction solution was adjusted to pH of about 2 with 5.0 M hydrochloric acid solution and concentrated under reduced pressure to give the title product **4e** (16.3 g, 5 purity: 8.8%).

### Step 5

### (7-methoxy-1-(4-(morpholinomethyl)phenyl)-5,5-dioxido-1,4-dihydrothiochromeno[4,3 -c]pyrazol-3-yl)(4-oxa-7-azaspiro[2.5]octan-7-yl)methanone 4

Compound **4e** (300 mg, 332.26 µmol, 58.8%), 4-oxa-7-azaspiro[2.5]octane hydrochloride (50 mg, 334.18 µmol, PharmaBlock), HATU (151 mg, 397.13 µmol) and *N,N*-diisopropylethylamine (215 mg, 1.66 mmol) were dissolved in *N*,*N*-dimethylformamide (5 mL), and the mixture was stirred at room temperature for 17 h. Saturated sodium bicarbonate solution (10 mL) was added, and the aqueous phase was extracted with ethyl acetate (20 mL × 3). The organic phases were combined and concentrated under reduced pressure, and the resulting residue was purified by a CombiFlash rapid preparation instrument with eluent system A to give the title product **4** (47 mg, yield: 25.1%).

MS m/z (ESI): 565.1 [M+1].

¹H NMR (500 MHz, CDCl₃) *δ* 7.62-7.60 (m, 1H), 7.53-7.49 (m, 2H), 7.46-7.40 (m, 2H), 7.07-6.89 (m, 1H), 6.83-6.81 (m, 1H), 4.76-4.75 (m, 2H), 4.31-4.29 (m, 1H), 4.18-4.16 (m, 1H), 3.91-3.88 (m, 5H), 3.83-3.78 (m, 6H), 3.63-3.60 (m, 2H), 2.54-2.51 (m, 4H), 0.87-0.84 (m, 2H), 0.72-0.68 (m, 2H).

### Example 5

### (6-methoxy-1-(4-(morpholinomethyl)phenyl)-5,5-dioxido-1,4-dihydrothiochromeno[4,3 -c]pyrazol-3-yl)(4-oxa-7-azaspiro[2.5]octan-7-yl)methanone 5

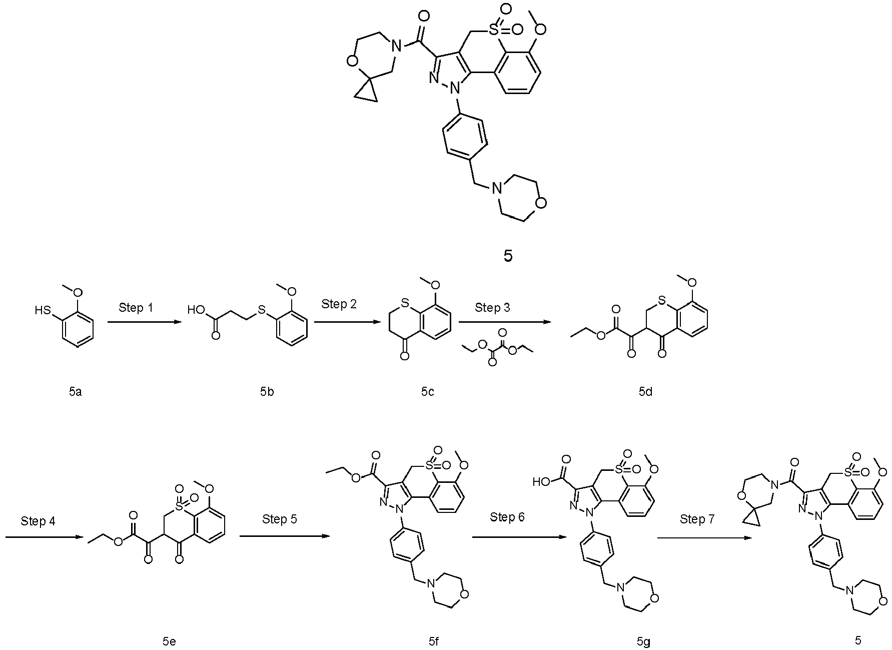

### Step 1

### 3-((2-methoxyphenyl)thio)propanoic acid 5b

2-methoxybenzenethiol **5a** (25.0 g, 178.31 mmol, Accela ChemBio) and potassium carbonate (36.9 g, 267.50 mmol, Sinopharm) were dissolved in *N,N*-dimethylformamide (200 mL). The mixture was stirred at 60 °C for 30 min under nitrogen atmosphere and cooled to room temperature, and 3-bromopropionic acid (28.6 g, 187.28 mmol, Adamas) was added. The resulting mixture was stirred at 60 °C for 3 h under nitrogen atmosphere. Water (1000 mL) was added to the reaction solution, and ethyl acetate (300 mL × 2) was added for extraction. The aqueous phase was adjusted to pH of about 3 with concentrated hydrochloric acid and extracted with ethyl acetate (400 mL × 2). The organic phases were combined, washed successively with water (400 mL × 2) and saturated brine (400 mL × 2), dried over anhydrous sodium sulfate and filtered, and the filtrate was concentrated under reduced pressure to give the title product **5b** (37 g, yield: 98%).

MS m/z (ESI): 213.1 [M-1].

### Step 2

### 8-methoxythiochroman-4-one 5c

Compound **5b** (37 g, 174.3 mmol) was dissolved in concentrated sulfuric acid (200 mL), and the mixture was stirred at 0 °C for 2 h. The reaction solution was poured into ice water (1000 mL), and ethyl acetate (300 mL × 3) was added for extraction. The organic phase was washed with saturated brine (300 mL × 2), dried over anhydrous sodium sulfate and filtered, the filtrate was concentrated under reduced pressure, and the residue was purified by a CombiFlash rapid preparation instrument with eluent system B to give the title product **5c** (1.74 g, yield: 4.3%).

MS m/z (ESI): 194.9 [M+1].

### Step 3

### Ethyl 2-(8-methoxy-4-oxothiochroman-3-yl)-2-oxoacetate 5d

Sodium ethoxide (6.10 g, 17.92 mmol, 20% w/w ethanol solution) was added to a 100 mL three-neck flask, and diethyl oxalate (1.97 g, 13.49 mmol, dissolved in 30 mL of toluene) and compound **5c** (1.74 g, 8.95 mmol, dissolved in 30 mL of toluene) were added at 0 °C. The mixture was reacted at room temperature for 16 h. The reaction solution was concentrated under reduced pressure, water (80 mL) was added to the residue, and dichloromethane (80 mL × 2) was added for extraction. The aqueous phase was adjusted to pH of about 2 with 5 M hydrochloric acid solution and extracted with ethyl acetate (70 mL × 3). The organic phases were combined, washed with saturated brine (60 mL × 2), dried over anhydrous sodium sulfate for 15 min and filtered, and the filtrate was concentrated under reduced pressure to give the title product **5d** (2.6 g, yield: 98.6%).

MS m/z (ESI): 295.0 [M+1].

### Step 4

### Ethyl 2-(8-methoxy-1,1-dioxido-4-oxothiochroman-3-yl)-2-oxoacetate 5e

Compound **5d** (2.6 g, 8.83 mmol) was dissolved in dichloromethane (250 mL), and 3-chloroperoxybenzoic acid (3.9 g, 19.47 mmol) was added in portions under an ice bath. The mixture was stirred at room temperature for 17 h. Then the mixture was filtered, the filtrate was concentrated under reduced pressure, and the residue was purified by a CombiFlash rapid preparation instrument with eluent system A to give the title compound **5e** (2.8 g, yield: 97.1%).

MS m/z (ESI): 326.9 [M+1].

### Step 5

### Ethyl

### 6-methoxy-1-(4-(morpholinomethyl)phenyl)-1,4-dihydrothiochromeno[4,3-c]pyrazole-3 -carboxylate 5,5-dioxide 5f

Compound **5e** (1.3 g, 3.98 mmol), the hydrochloride of compound **2c** (825.7 mg) and glacial acetic acid (478.4 mg, 7.96 mmol) were dissolved absolute ethanol (60 mL), and the mixture was heated to reflux and stirred for 3 h. Saturated sodium bicarbonate solution (60 mL) was added, and the mixed solution was extracted with ethyl acetate (80 mL × 3). The organic phases were combined and concentrated under reduced pressure, and the residue was purified by a CombiFlash rapid preparation instrument with eluent system A to give the title compound **5f** (1.63 g, yield: 82.2%).

MS m/z (ESI): 498.0 [M+1].

### Step 6

### 6-methoxy-1-(4-(morpholinomethyl)phenyl)-1,4-dihydrothiochromeno[4,3-c]pyrazole-3 -carboxylic acid 5,5-dioxide 5g

Compound **5f** (1.63 g, 3.27 mmol) was dissolved in tetrahydrofuran (30 mL), and aqueous sodium hydroxide solution (6.5 mL, 2.5 M) was added. The mixture was stirred for 4 h. Concentrated hydrochloric acid solution was added to adjust the pH to about 3, and the resulting mixture was concentrated under reduced pressure to give crude title compound **5g** (2.4 g, yield: 156.0%), which was directly used in the next step without purification.

MS m/z (ESI): 470.0 [M+1].

### Step 7

### (6-methoxy-1-(4-(morpholinomethyl)phenyl)-5,5-dioxido-1,4-dihydrothiochromeno[4,3 -c]pyrazol-3-yl)(4-oxa-7-azaspiro[2.5]octan-7-yl)methanone 5

Crude compound **5g** (330 mg, 421.71 µmol, purity: 60%), 4-oxa-7-azaspiro[2.5]octane hydrochloride (63 mg, 421.74 µmol, Jiangsu Aikon) and HATU (297.6 mg, 1.26 mmol) were dissolved in *N,N*-dimethylformamide (30 mL), and *N*,*N*-diisopropylethylamine (326 mg, 2.53 mmol) was added. The mixture was stirred for 2 h. Water (60 mL) was added to quench the reaction, and the aqueous phase was extracted with ethyl acetate (80 mL × 3). The organic phases were combined, washed with saturated brine (80 mL × 3), dried over anhydrous sodium sulfate and filtered, the filtrate was concentrated under reduced pressure, and the residue was purified by a CombiFlash rapid preparation instrument with eluent system A to give the title compound **5** (102 mg, yield: 42.8%).

MS m/z (ESI): 565.1 [M+1].

¹H NMR (500 MHz, DMSO-*d*₆): *δ* 7.53-7.50(m, 2H), 7.45-7.37 (m, 3H), 7.25(d, 1H), 6.39-6.37(m, 1H), 4.80 (d, 2H), 4.07 (m, 2H), 3.91 (s, 3H), 3.74-3.57 (m, 9H), 3.32 (s, 1H), 2.41 (t, 4H),0.74-0.59 (m, 4H).

### Example 6

### (6-methyl-1-(4-(morpholinomethyl)phenyl)-5,5-dioxido-1,4-dihydrothiochromeno[4,3-c ]pyrazol-3-yl)(4-oxa-7-azaspiro[2.5]octan-7-yl)methanone 6

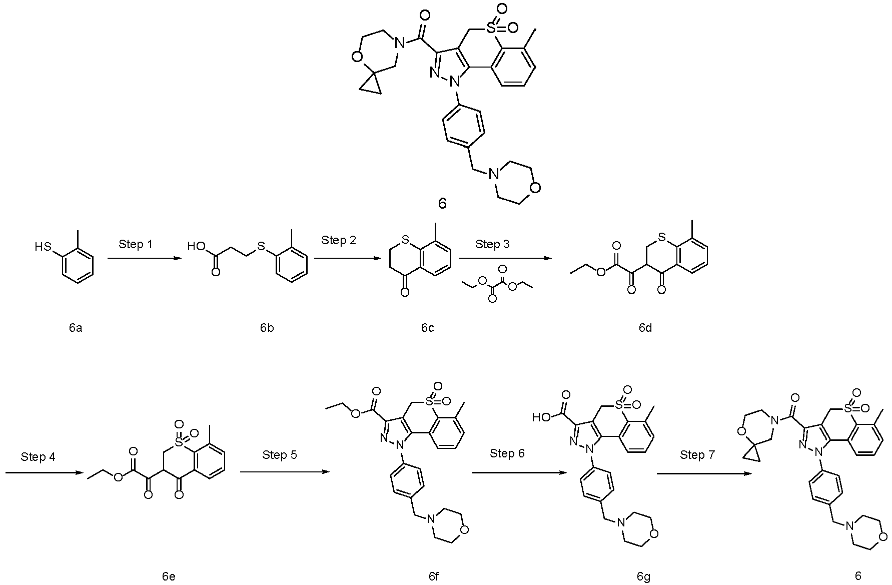

### Step 1

### 3-(o-tolylthio)propionic acid 6b

2-methylbenzenethiol **6a** (25.0g, 201.2 mmol, Accela ChemBio) and potassium carbonate (41.7 g, 301.9 mmol) were dissolved in N,N-dimethylformamide (200 mL), and the mixture was stirred at 60 °C for 30 min under nitrogen atmosphere. Then the mixture was cooled to room temperature, followed by addition of 3-bromopropionic acid (32.3 g, 211.4 mmol, Adamas). The resulting mixture was stirred at 60 °C for 3 h under nitrogen atmosphere. Water (1000 mL) was added to the reaction solution, and ethyl acetate (300 mL × 2) was added for extraction. The aqueous phase was adjusted to pH of about 3 with concentrated hydrochloric acid and extracted with ethyl acetate (400 mL × 2). The organic phases were combined, washed successively with water (400 mL × 2) and saturated brine (400 mL × 2), dried over anhydrous sodium sulfate and filtered, and the filtrate was concentrated under reduced pressure to give the title product **6b** (39 g, yield: 98%).

MS m/z (ESI): 195.2 [M-1].

### Step 2

### 8-methylthiochroman-4-one 6c

Compound **6b** (39 g, 198.6 mmol) was dissolved in concentrated sulfuric acid (200 mL), and the mixture was stirred at 0 °C for 2 h. The reaction solution was poured into ice water (1000 mL), and ethyl acetate (300 mL × 3) was added for extraction. The organic phase was washed with saturated brine (300 mL × 2) and dried over anhydrous sodium sulfate, the filtrate was concentrated under reduced pressure, and the residue was purified by a CombiFlash rapid preparation instrument with eluent system B to give the title product **6c** (15.5 g, yield: 43%).

MS m/z (ESI): 178.9 [M+1].

### Step 3

### Ethyl 2-(8-methyl-4-oxothiochroman-3-yl)-2-oxoacetate 6d

Sodium ethoxide (59 g, 173.93 mmol, 20% w/w ethanol solution) was added to a 500 mL three-neck flask, and diethyl oxalate (19 g, 130.49 mmol, dissolved in 100 mL of toluene) and compound **6c** (15.5 g, 86.9 mmol, dissolved in 100 mL of toluene) were added at 0 °C. The mixture was reacted at room temperature for 16 h. The reaction solution was concentrated under reduced pressure, water (400 mL) was added to the residue, and dichloromethane (200 mL × 2) was added for extraction. The aqueous phase was adjusted to pH of about 2 with 5 M hydrochloric acid solution and extracted with ethyl acetate (200 mL × 3). The organic phases were combined, washed with saturated brine (200 mL × 2), dried over anhydrous sodium sulfate for 15 min and filtered, and the filtrate was concentrated under reduced pressure to give the title product **6d** (24 g, yield: 99.0%).

MS m/z (ESI): 279.0 [M+1].

### Step 4

### Ethyl 2-(8-methyl-1,1-dioxido-4-oxothiochroman-3-yl)-2-oxoacetate 6e

Compound **6d** (24 g, 86.23 mmol) was dissolved in dichloromethane (250 mL), and 3-chloroperoxybenzoic acid (29 g, 172.5 mmol) was added in portions under an ice bath. The mixture was stirred at room temperature for 17 h. Then the mixture was filtered, the filtrate was concentrated under reduced pressure, and the residue was purified by a CombiFlash rapid preparation instrument with eluent system A to give the title compound **6e** (26 g, yield: 97.1%).

MS m/z (ESI): 310.9 [M+1].

### Step 5

### Ethyl 6-methyl-1-(4-(morpholinomethyl)phenyl)-1,4-dihydrothiochromeno[4,3-c]pyrazole-3-c arboxylate 5,5-dioxide 6f

Compound **6e** (15 g, 49.01 mmol), the hydrochloride of compound **2c** (10 g) and glacial acetic acid (5.9 g, 98.11 mmol) were dissolved in absolute ethanol (300 mL), and the mixture was heated to reflux and stirred for 3 h. Saturated sodium bicarbonate solution (300 mL) was added, and the mixed solution was extracted with ethyl acetate (250 mL × 3). The organic phases were combined and concentrated under reduced pressure, and the residue was purified by a CombiFlash rapid preparation instrument with eluent system A to give the title compound **6f** (20 g, yield: 86.9%).

MS m/z (ESI): 482.0 [M+1].

### Step 6

### 6-methyl-1-(4-(morpholinomethyl)phenyl)-1,4-dihydrothiochromeno[4,3-c]pyrazole-3-c arboxylic acid 5,5-dioxide 6g

Compound **6f** (14 g, 29.07 mmol) was dissolved in tetrahydrofuran (150 mL), and aqueous sodium hydroxide solution (58.2 mL, 2.5 M) was added. The mixture was stirred for 4 h. Concentrated hydrochloric acid solution was added to adjust the pH to about 3, and the resulting mixture was concentrated under reduced pressure to give crude title compound **6g** (21.2 g), which was directly used in the next step without purification.

MS m/z (ESI): 454.0 [M+1].

### Step 7

### (6-methyl-1-(4-(morpholinomethyl)phenyl)-5,5-dioxido-1,4-dihydrothiochromeno[4,3-c ]pyrazol-3-yl)(4-oxa-7-azaspiro[2.5]octan-7-yl)methanone 6

Compound **6g** (250 mg, 330.75 µmol), 4-oxa-7-azaspiro[2.5]octane hydrochloride (49.5 mg, 330.77 µmol) and HATU (233.4 mg, 992.28 µmol) were dissolved in *N,N*-dimethylformamide (30 mL), and *N*,*N*-diisopropylethylamine (256 mg, 1.98 mmol) was added. The mixture was stirred for 2 h. Water (60 mL) was added to quench the reaction, and the aqueous phase was extracted with ethyl acetate (80 mL × 3). The organic phases were combined, washed with saturated brine (80 mL × 3), dried over anhydrous sodium sulfate and filtered, the filtrate was concentrated under reduced pressure, and the residue was purified by a CombiFlash rapid preparation instrument with eluent system A to give the title compound **6** (40 mg, yield: 22%).

MS m/z (ESI): 549.1 [M+1].

¹H NMR (500 MHz, DMSO-*d*₆): *δ* 7.52-7.49(m, 2H), 7.42-7.35 (m, 4H), 6.71-6.68 (m, 1H), 4.89 (d, 2H), 4.09 (t, 2H), 3.92 (s, 3H), 3.74-3.57 (m, 9H), 3.29 (s, 1H), 2.41 (t, 4H), 0.72-0.61(m, 4H).

### Example 7

### (7-chloro-1-(4-(morpholinomethyl)phenyl)-5,5-dioxido-1,4-dihydrothiochromeno[4,3-c ]pyrazol-3-yl)(4-oxa-7-azaspiro[2.5]octan-7-yl)methanone 7

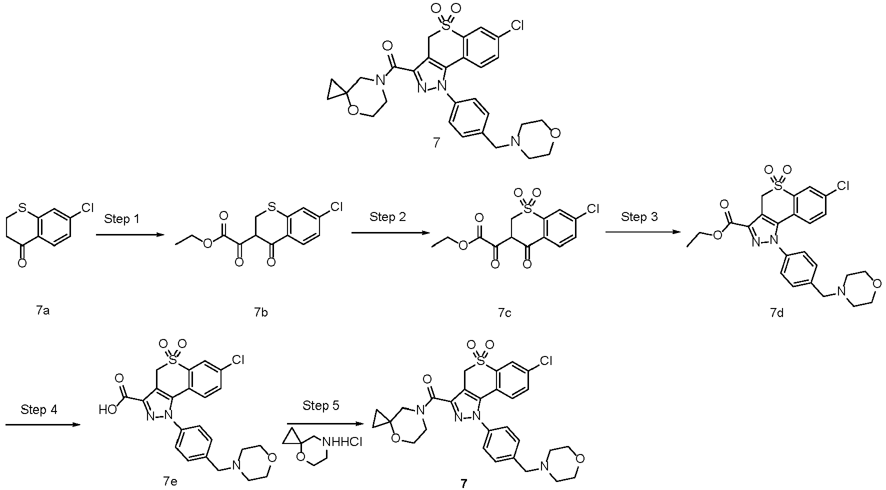

### Step 1

### Ethyl 2-(7-chloro-4-oxothiochroman-3-yl)-2-oxoacetate 7b

Sodium ethoxide (62.0 g, 182.2 mmol, 20% w/w ethanol solution) was added into a 500 mL single-neck flask, and a solution of diethyl oxalate (19.9 g, 136.2 mmol) in toluene (300 mL) was added at 0 °C, followed by addition of 7-chlorothiochroman-4-one **7a** (18.0 g, 90.6 mmol, prepared according to the method disclosed in "Organic Letters, 2020, 22(3), 1155-1159"). The mixture was stirred at room temperature for 17 h. The reaction solution was concentrated under reduced pressure, water (400 mL) was added to the residue, and dichloromethane (200 mL × 2) was added for extraction. The aqueous phase was adjusted to pH of about 2 with 5 M hydrochloric acid solution and extracted with ethyl acetate (200 mL × 3). The organic phases were combined, washed with saturated brine (200 mL × 2), dried over anhydrous sodium sulfate and filtered, and the filtrate was concentrated under reduced pressure to give the title compound **7b** (13.7 g), which was directly used in the next step without purification.

### Step 2

### Ethyl 2-(7-chloro-1,1-dioxido-4-oxothiochroman-3-yl)-2-oxoacetate 7c

Crude compound **7b** (11.80 g, 36.15 mmol) was dissolved in dichloromethane (150 mL), and 3-chloroperoxybenzoic acid (17.2 g, 81.79 mmol) was added. The mixture was stirred at room temperature for 17 h. Then the mixture was concentrated under reduced pressure, and the residue was purified by a CombiFlash rapid preparation instrument with eluent system B to give the title compound 7c (12.3 g, yield: 98.3%).

### Step 3

### Ethyl 7-chloro-1-(4-(morpholinomethyl)phenyl)-1,4-dihydrothiochromeno[4,3-c]pyrazole-3-c arboxylate 5,5-dioxide 7d

Compound **7c** (11.3 g, 33.6 mmol) was dissolved in ethanol (80 mL), and the hydrochloride of compound **2c** (9.0 g) and glacial acetic acid (20 mL) were added. The mixture was stirred at 80 °C for 2 h. Then the mixture was concentrated under reduced pressure, slurried with ethanol and filtered, and the filter cake was dried in vacuum to give the title product **7d** (13.9 g, yield: 83.2%).

### Step 4

### 7-chloro-1-(4-(morpholinomethyl)phenyl)-1,4-dihydrothiochromeno[4,3-c]pyrazole-3-c arboxylic acid 5,5-dioxide 7e

Compound **7d** (13.9 g, 28.3 mmol) was dissolved in tetrahydrofuran (100 mL), and aqueous sodium hydroxide solution (3 M, 5.5 mL) was added. The mixture was stirred at room temperature for 16 h. The reaction solution was adjusted to pH of about 2 with 5.0 M hydrochloric acid solution and concentrated under reduced pressure to give the title product **7e** (16.1 g), which was directly used in the next step without purification.

### Step 5

### (7-chloro-1-(4-(morpholinomethyl)phenyl)-5,5-dioxido-1,4-dihydrothiochromeno[4,3-c ]pyrazol-3-yl)(4-oxa-7-azaspiro[2.5]octan-7-yl)methanone 7

Crude compound **7e** (200 mg, 255.2 µmol), 4-oxa-7-azaspiro[2.5]octane hydrochloride (49.5 mg, 330.77 µmol) and HATU (233.4 mg, 992.28 µmol) were dissolved in N,N-dimethylformamide (10 mL), and *N*,*N*-diisopropylethylamine (256 mg, 1.98 mmol) was added. The mixture was stirred for 3 h. Water (60 mL) was added to quench the reaction, and the aqueous phase was extracted with ethyl acetate (80 mL × 3). The organic phases were combined, washed with saturated brine (80 mL × 3), dried over anhydrous sodium sulfate and filtered, the filtrate was concentrated under reduced pressure, and the residue was purified by a CombiFlash rapid preparation instrument with eluent system A to give the title compound 7 (55 mg, yield: 32.7%).

MS m/z (ESI): 569.1 [M+1].

¹H NMR (500 MHz, DMSO-*d*₆): *δ* 8.00 (s, 1H), 7.72 (d, 1H), 7.61-7.47 (m, 4H), 6.89 (d, 1H), 4.94 (d, 2H), 4.05 (s, 1H), 3.90 (s, 1H), 3.76-3.59 (m, 10H), 2.42 (brs, 4H), 0.68-0.64 (m, 4H).

### Example 8

### (1-(4-(morpholinomethyl)phenyl)-5,5-dioxido-6-(trifluoromethyl)-1,4-dihydrothiochro meno[4,3-c]pyrazol-3-yl)(4-oxa-7-azaspiro[2.5]octan-7-yl)methanone 8

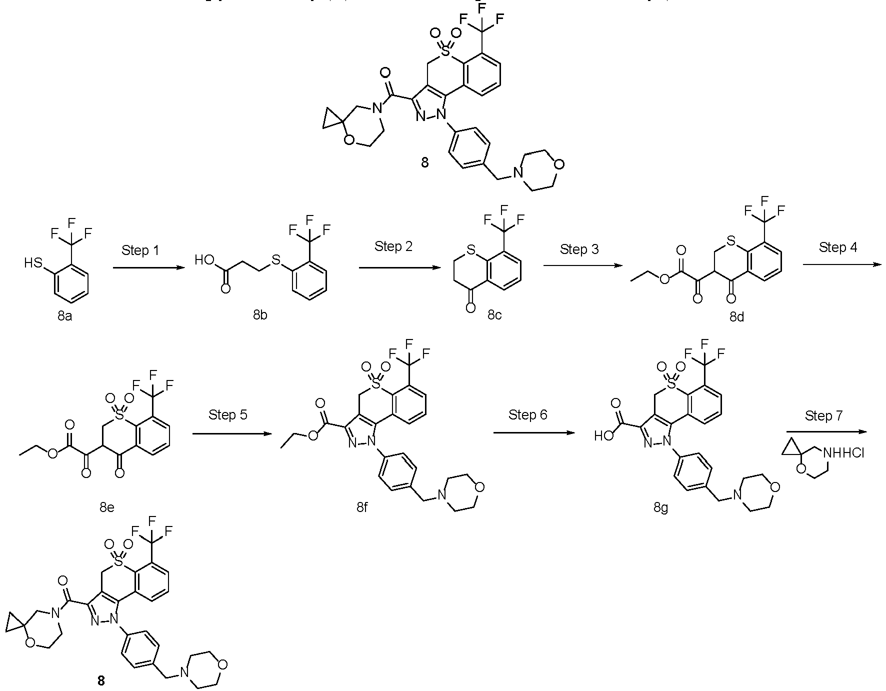

### Step 1

### 3-((2-(trifluoromethyl)phenyl)thio)propanoic acid 8b

Compound **8a** (10.4 g, 58.3 mmol) was dissolved in *N,N*-dimethylformamide (60 mL), and potassium carbonate (16.1 g, 116.5 mmol) was added. The mixture was stirred at 60 °C for 30 min. Then the mixture was cooled, and bromopropionic acid (9.8 g, 64.3 mmol) was added. The resulting mixture was stirred at 60 °C for 3 h. Then the mixture was cooled, poured into water, adjusted to pH of 2 with 2 M hydrochloric acid and extracted three times with ethyl acetate. The organic phases were combined, washed with saturated brine, dried over anhydrous sodium sulfate and filtered, the filtrate was concentrated under reduced pressure, and the residue was purified by a CombiFlash rapid preparation instrument with eluent system B to give the title compound **8b** (11.0 g, yield: 75.3%).

### Step 2

### 8-(trifluoromethyl)thiochroman-4-one 8c

Compound **8b** (11.0 g, 46.5 mmol) was added to concentrated sulfuric acid (150 mL), and the mixture was stirred at room temperature for 3 h. The reaction solution was poured into ice water and stirred well. Then the reaction solution was filtered and washed with water. The solid was dissolved in ethyl acetate, washed with saturated brine, dried over anhydrous sodium sulfate and filtered, the filtrate was concentrated under reduced pressure, and the residue was purified by a CombiFlash rapid preparation instrument with eluent system A to give the title compound 8c (8.0 g, yield: 71.8%).

### Step 3

### Ethyl 2-(8-(trifluoromethyl)-4-oxothiochroman-3-yl)-2-oxoacetate 8d

Sodium ethoxide (23.5 g, 69.1 mmol, 20% w/w ethanol solution) was added to a 500 mL single-neck flask, and a solution of diethyl oxalate (7.6 g, 51.7 mmol) in toluene (200 mL) was added at 0 °C, followed by the addition of compound **8c** (8.0 g, 34.5 mmol). The mixture was stirred at room temperature for 17 h. The reaction solution was concentrated under reduced pressure, water (300 mL) was added to the residue, and dichloromethane (100 mL × 2) was added for extraction. The aqueous phase was adjusted to pH of about 2 with 5 M hydrochloric acid solution and extracted with ethyl acetate (100 mL × 3). The organic phases were combined, washed with saturated brine (200 mL × 2), dried over anhydrous sodium sulfate and filtered, and the filtrate was concentrated under reduced pressure to give crude title compound **8d** (9.1 g), which was directly used in the next step without purification.

### Step 4

### Ethyl 2-(8-(trifluoromethyl)-1,1-dioxido-4-oxothiochroman-3-yl)-2-oxoacetate 8e

Compound **8d** (9.1 g, 27.2 mmol) was dissolved in dichloromethane (150 mL), and 3-chloroperoxybenzoic acid (11.6 g, 57.2 mmol) was added. The mixture was stirred at room temperature for 17 h. Then the mixture was concentrated under reduced pressure, and the residue was purified by a CombiFlash rapid preparation instrument with eluent system B to give the title compound **8e** (9.8 g, yield: 99.3%).

### Step 5

### Ethyl 6-(trifluoromethyl)-1-(4-(morpholinomethyl)phenyl)-1,4-dihydrothiochromeno[4,3-c]py razole-3-carboxylate 5,5-dioxide 8f

Compound **8e** (9.8 g, 26.9 mmol) was dissolved in ethanol (80 mL), and the hydrochloride of compound **2c** (7.0 g) and glacial acetic acid (20 mL) were added. The mixture was stirred at 80 °C for 2 h. Then the mixture was concentrated under reduced pressure, slurried with ethanol and filtered, and the filter cake was dried to give the title product **8f** (8.0 g, yield: 55.5%).

### Step 6

### 6-(trifluoromethyl)-1 -(4-(morpholinomethyl)phenyl)-1,4-dihydrothiochromeno[4,3 -c]py razole-3-carboxylic acid 5,5-dioxide 8g

Compound **8f** (8.0 g, 14.9 mmol) was dissolved in tetrahydrofuran (100 mL), and aqueous sodium hydroxide solution(3 M, 5.5 mL) was added. The mixture was stirred at room temperature for 16 h. The reaction solution was adjusted to pH of about 2 with 5.0 M hydrochloric acid solution and concentrated under reduced pressure to give the title product **8g** (10.5 g, purity: 70%), which was directly used in the next step without purification.

### Step 7

### (1-(4-(morpholinomethyl)phenyl)-5,5-dioxido-6-(trifluoromethyl)-1,4-dihydrothiochro meno[4,3-c]pyrazol-3-yl)(4-oxa-7-azaspiro[2.5]octan-7-yl)methanone 8

Crude compound **8g** (200 mg, 255.2 µmol), 4-oxa-7-azaspiro[2.5]octane hydrochloride (49.5 mg, 330.77 µmol) and HATU (233.4 mg, 992.28 µmol) were dissolved in N,N-dimethylformamide (10 mL), and *N*,*N*-diisopropylethylamine (172.9 mg, 1.34 mmol) was added. The mixture was stirred for 3 h. Water (60 mL) was added to quench the reaction, and the aqueous phase was extracted with ethyl acetate (50 mL × 3). The organic phases were combined, washed with saturated brine (80 mL × 2), dried over anhydrous sodium sulfate and filtered, the filtrate was concentrated under reduced pressure, and the residue was purified by a CombiFlash rapid preparation instrument with eluent system A to give the title compound **8** (60 mg, yield: 37.2%).

MS m/z (ESI): 603.1 [M+1].

¹H NMR (500 MHz, DMSO-*d*₆): *δ* 8.01 (d, 1H), 7.72 (t, 1H), 7.52 (t, 2H), 7.46-7.41 (m, 2H), 7.19 (d, 1H), 5.00 (d, 2H), 4.11 (s, 1H), 3.95 (s, 1H), 3.75-3.52 (m, 10H), 2.41 (brs, 4H), 0.69-0.65 (m, 4H).

### Example 9

(7-methyl-1-(4-(morpholinomethyl)phenyl)-5,5-dioxido-1,4-dihydrothiochromeno[4,3-*c* ]pyrazol-3-yl)(4-oxa-7-azaspiro[2.5]octan-7-yl)methanone **9**

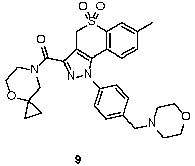

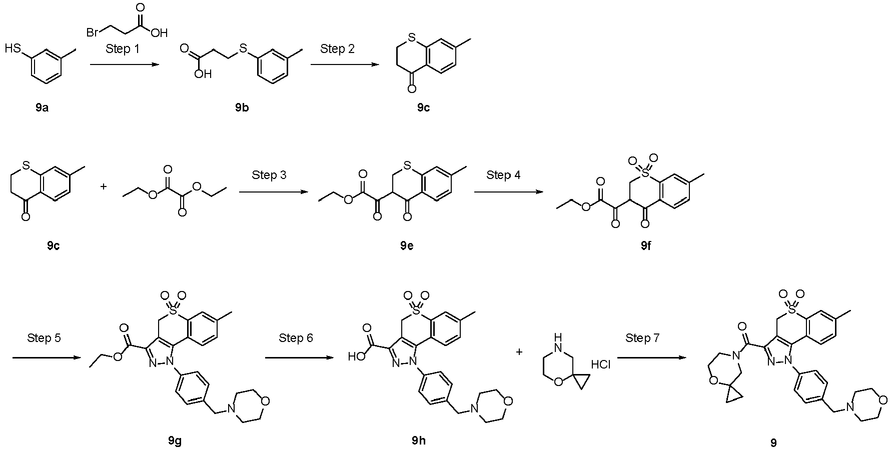

### Step 1

### 3-(m-tolylthio)propionic acid 9b

3-methylbenzenethiol **9a** (10 g, 80.5 mmol) was dissolved in *N,N*-dimethylformamide (100 mL), and potassium carbonate (16 g, 115.7 mmol) was added, followed by addition of 3-bromopropionic acid with stirring at room temperature. The mixture was stirred at room temperature for 2 h. Water (500 mL) was added to quench the reaction, and the aqueous phase was extracted with ethyl acetate (80 mL × 3). The organic phases were combined, dried over anhydrous sodium sulfate and filtered, and the filtrate was concentrated under reduced pressure to give crude title compound **9b** (8.94 g, yield: 56.5%), which was directly used in the next step without purification.

MS m/z(ESI): 195.0 [M-1].

### Step 2

### 7-methylthiochroman-4-one 9c

Crude compound **9b** (8.94 g, 45.5 mmol) was dissolved in concentrated sulfuric acid (100 mL), and the mixture was stirred at room temperature for 3 h. The reaction solution was carefully poured into ice water (500 g) to quench the reaction, and the aqueous phase was extracted with ethyl acetate (80 mL × 3). The organic phases were combined, dried over anhydrous sodium sulfate and filtered, the filtrate was concentrated under reduced pressure, and the residue was purified by column chromatography with developing solvent system B to give the title compound **9c** (5.17 g, yield: 63.7%).

¹H NMR (500 MHz, CDCl₃) *δ* 7.30 (t, 1H), 7.20 (d, 1H), 7.03 (d, 1H), 3.27-3.21 (m, 2H), 2.93-2.85 (m, 2H), 2.49 (s, 3H).

### Step 3

### Ethyl 2-(7-methyl-4-oxothiochroman-3-yl)-2-oxoacetate 9e

Sodium ethoxide (20 g, 58.78 mmol, 20% w/w ethanol solution) was added to a single-neck flask and cooled in an ice bath. Diethyl oxalate (4.5 g, 30.79 mmol, dissolved in 50 mL of toluene) was added, followed by addition of compound **9c** (5.17 g, 29.00 mmol, dissolved in 50 mL of toluene) with stirring. The mixture was stirred at room temperature for 18 h. The reaction solution was concentrated under reduced pressure, and water (200 mL) was added to quench the reaction. The pH was adjusted to neutral with 3 M aqueous hydrochloric acid solution, and the aqueous phase was extracted with ethyl acetate (100 mL × 3). The organic phases were combined, dried over anhydrous sodium sulfate and filtered, and the filtrate was concentrated under reduced pressure to give crude title compound **9e** (7.87 g, yield: 97.5%).

MS m/z(ESI): 279.0 [M+1].

### Step 4

### Ethyl 2-(7-methyl-1,1-dioxido-4-oxothiochroman-3-yl)-2-oxoacetate 9f

Compound **9e** (7.78 g, 28.28 mmol) was dissolved in dichloromethane (240 mL), and 3-chloroperoxybenzoic acid (14 g, 68.96 mmol) was added in portions under an ice bath. The mixture was stirred at room temperature for 3 h. Insoluble substances were filtered off, the filtrate was concentrated under reduced pressure, and the residue was purified by column chromatography with system A to give the title compound **9f** (6.88 g, yield: 78.2%).

MS m/z(ESI): 310.9 [M+1].

### Step 5

### Ethyl 7-methyl-1-(4-(morpholinomethyl)phenyl)-1,4-dihydrothiochromeno[4,3-c]pyrazole-3-c arboxylate 5,5-dioxide 9g

Compound **9f** (6.88 g, 22.17 mmol) and the hydrochloride of compound **2c** (4.5 g, 21.71 mmol) were dissolved in ethanol (150 mL), and glacial acetic acid (2.5 g, 41.63 mmol) was added. The mixture was stirred at reflux for 6 h. After the reaction solution was cooled to room temperature, a yellow solid was filtered off, collected and dried in vacuum to give crude title product **9g** (14.7 g), which was directly used in the next step without purification.

MS m/z(ESI): 482.2 [M+1].

### Step 6

### 7-methyl-1-(4-(morpholinomethyl)phenyl)-1,4-dihydrothiochromeno[4,3-c]pyrazole-3-c arboxylic acid 5,5-dioxide 9h

Crude compound **9g** (5 g, 10.38 mmol) was dissolved in tetrahydrofuran (60 mL), and aqueous sodium hydroxide solution (2.5 M, 10 mL) was added. The mixture was stirred at 60 °C for 1 h. After the reaction solution was cooled to room temperature, the pH was adjusted to neutral with 3 M hydrochloric acid. The organic solvent was removed under reduced pressure and the remaining aqueous phase was lyophilized to give crude title product **9h** (9 g), which was directly used in the next step without purification.

MS m/z(ESI): 452.1 [M-1].

### Step 7

### (7-methyl-1-(4-(morpholinomethyl)phenyl)-5,5-dioxido-1,4-dihydrothiochromeno[4,3-c ]pyrazol-3-yl)(4-oxa-7-azaspiro[2.5]octan-7-yl)methanone 9

Crude compound **9h** (200 mg, 0.22 mmol), 4-oxa-7-azaspiro[2.5]octane hydrochloride (40 mg, 0.27 mmol) and HATU (120 mg, 0.32 mmol) were dissolved in *N,N*-dimethylformamide (5 mL), and *N*,*N*-diisopropylethylamine (100 mg, 0.77 mmol, 0.13 mL) was added. The mixture was stirred for 2 h. Water (150 mL) was added to quench the reaction, and the aqueous phase was extracted with ethyl acetate (50 mL × 3). The organic phases were combined, washed with saturated brine (50 mL × 3), dried over anhydrous sodium sulfate and filtered, the filtrate was concentrated under reduced pressure, and the residue was purified by silica gel column chromatography with eluent system A to give the title product **9** (32 mg, yield: 26.5%).

MS m/z(ESI): 549.1 [M+1].

¹H NMR (500 MHz, DMSO-*d*₆) *δ* 7.84 (s, 1H), 7.54 (t, 2H), 7.49 (d, 1H), 7.45 (d, 1H), 7.38 (d, 1H), 6.73 (d, 1H), 4.84 (d, 2H), 4.10-4.03 (m, 1H), 3.91 (s, 1H), 3.73 (d, 2H), 3.70-3.64 (m, 2H), 3.64-3.56 (m, 6H), 2.44-2.36 (m, 7H), 0.75-0.66 (m, 2H), 0.66-0.58 (m, 2H).

### Control Example 10

### (6-fluoro-1-(4-(morpholinomethyl)phenyl)-5,5-dioxido-1,4-dihydrothiochromeno[4,3-c] pyrazol-3-yl)(7-oxa-4-azaspiro[2.5]octan-4-yl)methanone 10

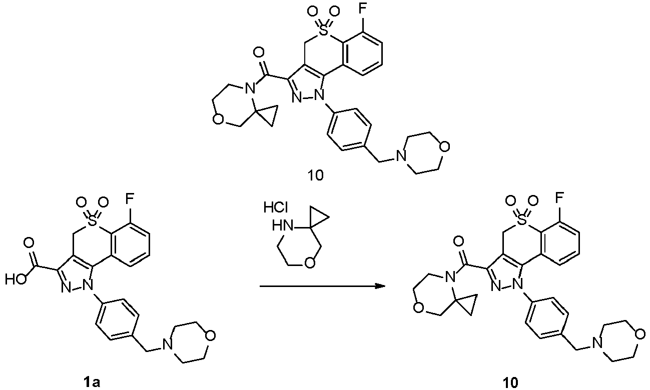

Compound **1a,** 7-oxa-4-azaspiro[2.5]octane hydrochloride (33 mg, 220.77 mmol, Jiangsu Aikon), 1-(3-dimethylaminopropyl)-3-ethylcarbodiimide hydrochloride (251 mg, 651.77 mmol), 1-hydroxybenzotriazole (99 mg, 655.77 mmol), *N,N*-diisopropylethylamine (110 mg, 1.09 mmol) and 4-dimethylaminopyridine (53 mg, 437.18 mmol) were dissolved in dichloromethane (30 mL), and the mixture was stirred at room temperature for 16 h. Water (50 mL) was added, and a mixed solvent (60 mL) of dichloromethane and methanol (V:V = 10:1) was added for extraction. The organic phases were combined, washed successively with water (60 mL) and saturated brine (60 mL), dried over anhydrous sodium sulfate and filtered, the filtrate was concentrated under reduced pressure, and the resulting residue was purified by a CombiFlash rapid preparation instrument with eluent system A to give the title product **10** (25 mg, yield: 20.6%).

MS m/z (ESI): 553.3 [M+1].

¹H NMR (500 MHz, DMSO-*d*₆): *δ* 7.60-7.44 (m, 6H), 6.65 (d, 1H), 4.95 (d, 2H), 3.99-3.56 (m, 9H), 2.41 (s, 4H), 1.27-0.72 (m, 7H).

### Example 11

### (7-chloro-6-fluoro-1-(4-(morpholinomethyl)phenyl)-5,5-dioxido-1,4-dihydrothiochrome no[4,3-c]pyrazol-3-yl)(4-oxa-7-azaspiro[2.5]octan-7-yl)methanone 11

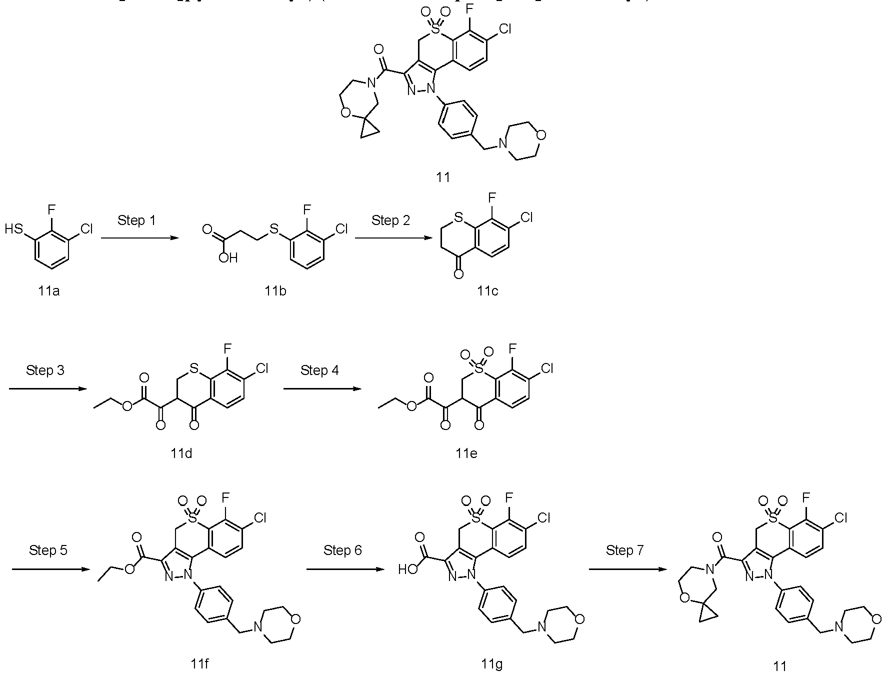

### Step 1

### 3-((3-chloro-2-fluorophenyl)thio)propanoic acid 11b

3-chloro-2- fluorobenzenethiol **11a** (8 g, 49.20 mmol, Wuxi Kehua) was dissolved in N,N-dimethylformamide (100 mL), and potassium carbonate (8.840 g, 63.96 mmol) was added. The mixture was stirred at 60 °C for 30 min, followed by addition of 3-bromopropionic acid (8.279 g, 54.12 mmol). The resulting mixture was stirred at 60 °C for 2 h. Water (500 mL) was added to quench the reaction, and ethyl acetate (200 mL × 1) was added for extraction. The aqueous phase was adjusted to pH of about 3 with concentrated hydrochloric acid and then extracted with ethyl acetate (300 mL × 3). The organic phases were combined, dried over anhydrous sodium sulfate and filtered, and the filtrate was concentrated under reduced pressure to give crude title compound **11b** (11.166 g, yield: 96.7%), which was directly used in the next step without purification.

### Step 2

### 7-chloro-8-fluorothiochroman-4-one 11c

Crude compound **11b** (11.116 g, 47.37 mmol) was dissolved in concentrated sulfuric acid (100 mL), and the mixture was stirred at room temperature for 3 h. The reaction solution was carefully poured into ice water (500 mL) to quench the reaction, and the aqueous phase was extracted with ethyl acetate (200 mL × 3). The organic phases were combined, dried over anhydrous sodium sulfate and filtered, and the filtrate was concentrated under reduced pressure to give the title compound **11c** (8.731 g, yield: 85.1%), which was directly used in the next step without purification.

### Step 3

### Ethyl 2-(7-chloro-8-fluoro-4-oxothiochroman-3-yl)-2-oxoacetate 11d

Sodium ethoxide (27.423 g, 80.60 mmol, 20% w/w ethanol solution, TCI) and toluene (125 mL) were added to a single-neck flask. The mixture was cooled in an ice bath, and diethyl oxalate (8.834 g, 60.45 mmol) was added, followed by addition of crude compound **11c** (8.731 g, 40.30 mmol) with stirring. The resulting mixture was stirred at room temperature for 17 h. The reaction solution was concentrated under reduced pressure, and water (600 mL) was added to quench the reaction. The pH was adjusted to about 3 with concentrated hydrochloric acid, and the aqueous phase was extracted with ethyl acetate (250 mL × 3). The organic phases were combined, dried over anhydrous sodium sulfate and filtered, and the filtrate was concentrated under reduced pressure to give crude title compound **11d** (11.919 g, yield: 93.4%).

MS m/z(ESI): 316.9 [M+1].

### Step 4

### Ethyl 2-(7-chloro-8-fluoro-1,1-dioxido-4-oxothiochroman-3-yl)-2-oxoacetate 11e

Compound **11d** (11.919 g, 37.63 mmol) was dissolved in dichloromethane (130 mL), and 3-chloroperoxybenzoic acid (19.100 g, 94.08 mmol) was added in portions under an ice bath. The mixture was stirred at room temperature for 17 h. Insoluble substances were filtered off, the filtrate was concentrated, and the residue was purified by column chromatography with system A to give the title compound **11e** (11.5 g, yield: 87.6%).

MS m/z(ESI): 347.0 [M-1].

### Step 5

### Ethyl 7-chloro-6-fluoro-1-(4-(morpholinomethyl)phenyl)-1,4-dihydrothiochromeno[4,3-c]pyr azole-3-carboxylate 5,5-dioxide 11f

Compound **11e** (11.5 g, 32.98 mmol) and the hydrochloride of compound **2c** (10.024 g) were dissolved in ethanol (250 mL), and glacial acetic acid (3.961 g, 65.96 mmol) was added. The mixture was stirred at reflux for 3 h. Saturated sodium bicarbonate solution (300 mL) was added to the reaction solution, and ethyl acetate (250 mL × 4) was added for extraction. The organic phases were combined, dried over anhydrous sodium sulfate and filtered, and the filtrate was concentrated under reduced pressure to give crude title product **11f** (16 g, yield: 93.3%), which was directly used in the next step without purification.

MS m/z(ESI): 520.0 [M+1].

### Step 6

### 7-chloro-6-fluoro-1-(4-(morpholinomethyl)phenyl)-1,4-dihydrothiochromeno[4,3-c]pyr azole-3-carboxylic acid 5,5-dioxide 11g

Crude compound **11f** (16 g, 30.77 mmol) was dissolved in tetrahydrofuran (250 mL), and aqueous sodium hydroxide solution (2.5 M, 62 mL) was added. The mixture was stirred at room temperature for 4 h. The reaction solution was adjusted to pH of about 3 with 3 M hydrochloride acid and concentrated under reduced pressure to give crude title product **11g** (15 g, yield: 99.1%), which was directly used in the next step without purification.

MS m/z(ESI): 491.9 [M+1].

### Step 7

### (7-chloro-6-fluoro-1-(4-(morpholinomethyl)phenyl)-5,5-dioxido-1,4-dihydrothiochrome no[4,3-c]pyrazol-3-yl)(4-oxa-7-azaspiro[2.5]octan-7-yl)methanone 11

Crude compound **11g** (506 mg, 0.52 mmol), 4-oxa-7-azaspiro[2.5]octane hydrochloride (80 mg, 0.53 mmol) and HATU (240 mg, 0.63 mmol) were dissolved in N,N-dimethylformamide (25 mL), and *N*,*N*-diisopropylethylamine (340 mg, 2.63 mmol) was added. The mixture was stirred for 17 h. Saturated sodium bicarbonate solution (50 mL) was added to quench the reaction, and the aqueous phase was extracted with ethyl acetate (50 mL × 3). The organic phases were combined, and the filtrate was concentrated under reduced pressure. The residue was purified by silica gel column chromatography with eluent system A to give the title product **11** (72.6 mg, yield: 23.6%).

MS m/z(ESI): 587.0 [M+1].

¹H NMR (500 MHz, DMSO-*d*₆) *δ* 7.85 (s, 1H), 7.53-7.46 (m, 4H), 6.68-6.66 (m, 1H), 5.05-5.03 (m, 2H), 4.06-3.47 (m, 12H), 2.50-2.42 (m, 4H), 0.73-0.60 (m, 4H).

### Example 12

### (9-methyl-1-(4-(morpholinomethyl)phenyl)-5,5-dioxido-1,4-dihydrothiochromeno[4,3-c ]pyrazol-3-yl)(4-oxa-7-azaspiro[2.5]octan-7-yl)methanone 12

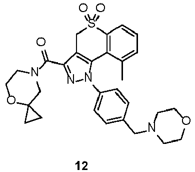

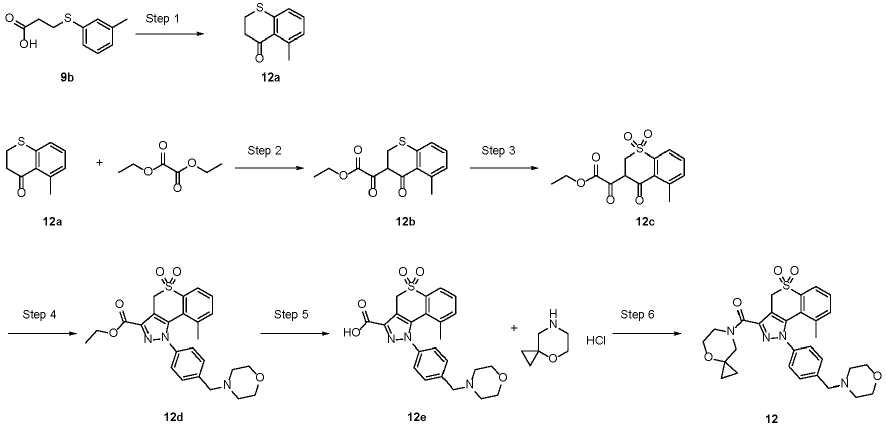

### Step 1

### 5-methylthiochroman-4-one 12a

Crude compound **9b** (8.94 g, 45.5 mmol) was dissolved in concentrated sulfuric acid (100 mL), and the mixture was stirred at room temperature for 3 h. The reaction solution was carefully poured into ice water (500 g) to quench the reaction. Liquid separation was performed, and the aqueous phase was extracted with ethyl acetate (80 mL × 3). The organic phases were combined, dried over anhydrous sodium sulfate and filtered, the filtrate was concentrated under reduced pressure, and the residue was purified by column chromatography with developing solvent system B to give the title compound **12a** (5.17 g, yield: 41.6%).

¹H NMR (500 MHz, CDCl₃) *δ* 7.30 (t, 1H), 7.20 (d, 1H), 7.03 (d, 1H), 3.27-3.21 (m, 2H), 2.93-2.85 (m, 2H), 2.49 (s, 3H).

### Step 2

### Ethyl 2-(5-methyl-4-oxothiochroman-3-yl)-2-oxoacetate 12b

Sodium ethoxide (13 g, 38.21 mmol, 20% w/w ethanol solution) was added to a single-neck flask and cooled in an ice bath. Diethyl oxalate (3.0 g, 20.53 mmol, dissolved in 50 mL of toluene) was added, followed by addition of compound **12a** (3.38 g, 18.93 mmol, dissolved in 50 mL of toluene) with stirring. The mixture was stirred at room temperature for 18 h. The reaction solution was concentrated under reduced pressure, and water (200 mL) was added to quench the reaction. The pH was adjusted to neutral with 3 M aqueous hydrochloric acid solution, and the aqueous phase was extracted with ethyl acetate (100 mL × 3). The organic phases were combined, dried over anhydrous sodium sulfate and filtered, and the filtrate was concentrated under reduced pressure to give crude title compound **12b** (3.66 g, yield: 69.56%), which was directly used in the next step without purification.

MS m/z(ESI): 279.0 [M+1].

### Step 3

### Ethyl 2-(5-methyl-1,1-dioxido-4-oxothiochroman-3-yl)-2-oxoacetate 12c

Compound **12b** (3.66 g, 13.17 mmol) was dissolved in dichloromethane (50 mL), and 3-chloroperoxybenzoic acid (6 g, 29.55 mmol, content: 85%) was added in portions under an ice bath. The mixture was stirred at room temperature for 3 h. Insoluble substances were filtered off, the filtrate was concentrated under reduced pressure, and the residue was purified by column chromatography with system A to give crude title compound **12c** (7.3 g, crude product).

MS m/z(ESI): 310.9 [M+1].

### Step 4

### Ethyl 9-methyl-1-(4-(morpholinomethyl)phenyl)-1,4-dihydrothiochromeno[4,3-c]pyrazole-3-c arboxylate 5,5-dioxide 12d

Crude compound **12c** (7.3 g, 12.94 mmol) and the hydrochloride of compound **2c** (3.6 g) were dissolved in ethanol (50 mL), and glacial acetic acid (1.5 g, 24.98 mmol) was added. The mixture was stirred at reflux for 6 h. After being cooled to room temperature, the reaction solution was filtered, and the filter cake was collected and dried in vacuum to give the title product **12d** (6.8 g), which was directly used in the next step without purification.

MS m/z(ESI): 482.2 [M+1].

### Step 5

### 9-methyl-1-(4-(morpholinomethyl)phenyl)-1,4-dihydrothiochromeno[4,3-c]pyrazole-3-c arboxylic acid 5,5-dioxide 12e

Crude compound **12d** (1.35 g, 1.54 mmol) was dissolved in tetrahydrofuran (15 mL), and aqueous sodium hydroxide solution (2.5 M, 3 mL) was added. The mixture was stirred at 60 °C for 1 h. After the reaction solution was cooled to room temperature, the pH was adjusted to neutral with 3 M hydrochloric acid. The organic solvent was removed under reduced pressure and the remaining aqueous phase was lyophilized to give crude title product **12e** (2.2 g), which was directly used in the next step without purification.

MS m/z(ESI): 452.1 [M-1].

### Step 6

### (9-methyl-1-(4-(morpholinomethyl)phenyl)-5,5-dioxido-1,4-dihydrothiochromeno[4,3-c ]pyrazol-3-yl)(4-oxa-7-azaspiro[2.5]octan-7-yl)methanone 12

Crude compound **12e** (200 mg, 0.22 mmol), 4-oxa-7-azaspiro[2.5]octane hydrochloride (45 mg, 0.30 mmol) and HATU (150 mg, 0.40mmol) were dissolved in N,N-dimethylformamide (5 mL), and *N*,*N*-diisopropylethylamine (150 mg, 1.16 mmol, 0.19 mL) was added. The mixture was stirred for 2 h. Water (150 mL) was added to quench the reaction, and the aqueous phase was extracted with ethyl acetate (50 mL × 3). The organic phases were combined, washed with saturated brine (50 mL × 3), dried over anhydrous sodium sulfate and filtered, the filtrate was concentrated under reduced pressure, and the residue was purified by silica gel column chromatography with eluent system A to give the title product **12** (62 mg, yield: 43.7%).

MS m/z(ESI): 549.1 [M+H].

¹H NMR (500 MHz, DMSO-*d*₆) *δ* 7.89 (d, 1H), 7.64 (t, 1H), 7.53 (d, 1H), 7.45 (t, 2H), 7.27 (d, 2H), 4.79 (s, 2H), 4.06 (t, 1H), 3.93 (s, 1H), 3.79-3.47 (m, 9H), 3.29 (s, 1H), 2.37 (s, 4H), 1.61 (d, 3H), 0.75-0.52 (m, 4H).

### Test Examples:

### Biological Evaluation

### Test Example 1. Test on Inhibitory Activity and Selectivity of Compounds Disclosed Herein on PI3Kδ Enzyme

### I. Experimental objective

This experiment is intended to test the inhibitory effect and selectivity of the compounds on PI3Kδ enzyme activity, and to evaluate the *in vitro* activity of the compounds according to the IC₅₀ values.

### II. Experimental principle

In this experiment, an ADP-Glo^{™} kinase assay kit is used. A substrate is phosphorylated under the action of enzyme, and ADP is generated at the same time. An ADP-Glo Reagent is added to remove unreacted ATP in the reaction system, and the ADP generated by the reaction is detected by a kinase detection reagent. In the presence of a compound, the inhibition rate of the compound is calculated by measuring the signal value.

### III. Experimental materials

### 1. Instruments

| Instrument | Supplier | Model |
|---|---|---|
| Centrifuge | Eppendorf | 5430 |
| Microplate reader | Perkin Elmer | Envision, SN. 1050214 |
| Echo 550 | Labcyte | Echo 550 |

### 2. Reagents and consumables

| Reagent | Supplier | Cat. No. |
|---|---|---|
| PIK3CD/PIK3R1 | Carna | 11-103 |
| PI103 | selleckchem | S1038 |
| DMSO | Sigma | D8418-1L |
| 384-well white plate | PerkinElmer | 6007290 |

### IV Experimental procedures

Test compounds were each subjected to 3-fold dilution from a starting concentration of 10000 nM to obtain 11 concentrations, and duplicate wells were set for the test. Gradient dilution to a corresponding 100-fold final concentration in a 384-well plate was performed to obtain solutions with 11 different concentrations. 50 nL of each solution was transferred to compound wells of a 384-well plate with Echo; negative control wells were added with 50 nL of DMSO. A kinase solution with a 2-fold final concentration was prepared using 1× kinase buffer. 2.5 µL of the kinase solution with a 2-fold final concentration was added to the compound wells; 2.5 µL of the 1× kinase buffer was added to the negative control wells. The mixture was centrifuged at 1000 rpm for 30 s, mixed well by shaking, and then incubated at room temperature for 10 min. A mixed solution of ATP and substrate P1P2 with a 2-fold final concentration was prepared using 1× kinase buffer. The reaction was initiated by adding 2.5 µL of the mixed solution of ATP and substrate with a 2-fold final concentration. The mixture in the 384-well plate was centrifuged at 1000 rpm for 30 s, mixed well by shaking, and then reacted at room temperature for 120 min. 5 µL of ADP-Glo reagent was added, and the mixture was centrifuged at 1000 rpm for 30 s, mixed well by shaking, and then incubated at room temperature for 40 min. 10 µL of kinase detection reagent was added, and the resulting mixture was centrifuged at 1000 rpm for 30 s, mixed well by shaking, and then incubated at room temperature for 30 min. The luminescence RLU was read with an Envision microplate reader.

### V Data analysis

IC₅₀ values for the inhibitory activity of the compounds were calculated using Graphpad Prism software. The results are shown in Table 1 below.

**Table 1. Data on inhibition and selectivity activity of compounds disclosed herein on PI3Kδ enzyme (nM)**

| Example No. | PI3Kδ enzyme IC₅₀ | PI3Kα enzyme IC₅₀ | PI3Kβ enzyme IC₅₀ | PI3Kγ enzyme IC₅₀ |
|---|---|---|---|---|
| 1 | 13 | 467.6 | 1316.0 | 6210.0 |
| 2 | 18.4 | - | - | - |
| 3 | 5.6 | - | - | - |
| 4 | 7.9 | - | - | - |
| 5 | 19 | 2329 | 2791 | >10000 |
| 6 | 68 | 2570 | 7058 | >10000 |
| 7 | 26.5 | 1523 | 1743 | >10000 |
| 9 | 25.0 | 1761 | 1551 | >10000 |
| 11 | 10.5 | 441 | 736 | 8918 |
| 12 | 5.8 | - | - | - |
| 10 (Control Example) | >10000 | - | - | - |

Conclusion: compared with the Control Example, the compounds disclosed herein have stronger inhibitory activity and selectivity on PI3Kδ enzyme.

## Claims

1. A compound of general formula (I) or a tautomer, racemate, enantiomer or diastereomer thereof or a mixture thereof, or a pharmaceutically acceptable salt thereof: 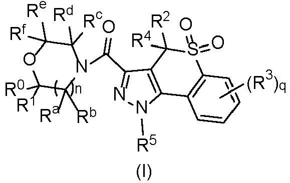 wherein:
R^{a}, R^{b}, R^{c}, R^{d}, R^{e} and R^{f} are identical or different and are each independently selected from the group consisting of a hydrogen atom, alkyl, halogen, alkoxy, haloalkoxy, cyano, hydroxy, hydroxyalkyl, -(CH₂)ₛNR⁷R⁸, cycloalkyl, cycloalkylalkyl, cycloalkyloxy, heterocyclyl, heterocyclylalkyl, heterocyclyloxy, aryl and heteroaryl,
wherein the alkyl, cycloalkyl, heterocyclyl, aryl and heteroaryl are each independently optionally substituted with one or more substituents selected from the group consisting of halogen, alkyl, haloalkyl, cyano, nitro, -(CH₂)_{y}NR^{g}R^{h} and -OR⁹;
R⁰ and R¹, together with the carbon atom to which they are attached, form a spiro ring on the heterocycle to which they are attached, the spiro ring being optionally substituted with one or more R';
R' are identical or different and are each independently selected from the group consisting of a hydrogen atom, alkyl, halogen, alkoxy, haloalkoxy, cyano, hydroxy, hydroxyalkyl, -(CH₂)ₛNR⁷R⁸ and nitro;
R⁵ is selected from the group consisting of a hydrogen atom, alkyl, cycloalkyl, heterocyclyl, aryl and heteroaryl, wherein the alkyl, cycloalkyl, heterocyclyl, aryl and heteroaryl are each independently optionally substituted with one or more substituents selected from the group consisting of halogen, alkyl, haloalkyl, nitro, cyano, hydroxyalkyl, -(CH₂)_{y}NR^{g}R^{h}, -OR⁹, -COR⁹, -COOR⁹, -OS(O)ₜR⁹, -S(O)ₜR⁹, -NR⁶COR⁹, -NR⁶SO₂R⁹ and R;
the R is selected from the group consisting of cycloalkyl, heterocyclyl, aryl, heteroaryl, cycloalkylalkyl, heterocyclylalkyl, arylalkyl and heteroarylalkyl, and the R are each independently optionally substituted with one or more substituents selected from the group consisting of halogen, alkyl, haloalkyl, nitro, cyano, hydroxyalkyl, -(CH₂)_{y}NR^{g}R^{h}, -OR⁹, cycloalkyl, cycloalkylalkyl, heterocyclyl, heterocyclylalkyl, aryl and heteroaryl;
R² and R⁴ are identical or different and are each independently selected from the group consisting of a hydrogen atom, halogen, alkyl, haloalkyl, alkoxy, cycloalkyl, aryl, heterocyclyl, heteroaryl, cycloalkylalkyl, arylalkyl, heterocyclylalkyl and heteroarylalkyl, wherein the alkyl, cycloalkyl, heterocyclyl, aryl and heteroaryl are each independently optionally substituted with one or more substituents selected from the group consisting of alkyl, haloalkyl, halogen, cyano, nitro, -(CH₂)_{y}NR^{g}R^{h}, -OR⁹, -COR⁹, -COOR⁹, -OS(O)ₜR⁹, -S(O)ₜR⁹, -NR⁶COR⁹ and -NR⁶SO₂R⁹;
R³ are identical or different and are each independently selected from the group consisting of a hydrogen atom, halogen, alkyl, haloalkyl, cyano, nitro, -(CH₂)ₛNR⁷R⁸, -ORⁱ, -CORⁱ, -COORⁱ, -OS(O)ₓRⁱ, -S(O)ₓRⁱ, -NR⁶CORⁱ, -NR⁶SO₂Rⁱ, cycloalkyl, heterocyclyl, aryl and heteroaryl, wherein the alkyl, cycloalkyl, heterocyclyl, aryl and heteroaryl are each independently optionally substituted with one or more substituents selected from the group consisting of alkyl, haloalkyl, halogen, cyano, nitro, -(CH₂)_{y}NR^{g}R^{h}, -OR⁹, -COR⁹, -COOR⁹, -OS(O)ₜR⁹, -S(O)ₜR⁹, -NR⁶COR⁹ and -NR⁶SO₂R⁹;
or two adjacent R³, together with the carbon atom to which they are attached, form cycloalkyl, heterocyclyl, aryl or heteroaryl, wherein the cycloalkyl, heterocyclyl, aryl and heteroaryl are each independently optionally substituted with one or more substituents selected from the group consisting of a hydrogen atom, alkyl, halogen, haloalkyl, alkoxy, haloalkoxy, cyano, amino, nitro, hydroxy, hydroxyalkyl, cycloalkyl, heterocyclyl, aryl and heteroaryl;
R⁶ is selected from the group consisting of a hydrogen atom, alkyl, cycloalkyl and aryl, wherein the alkyl, cycloalkyl and aryl are each independently optionally substituted with one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R⁷, R⁸, R^{g} and R^{h} are identical or different and are each independently selected from the group consisting of a hydrogen atom, alkyl, haloalkyl, hydroxyalkyl, cycloalkyl, heterocyclyl, aryl and heteroaryl;
or R⁷ and R⁸, or R^{g} and R^{h}, together with the nitrogen atom to which they are attached, form heterocyclyl, wherein the heterocyclyl is optionally substituted with one or more substituents selected from the group consisting of alkyl, alkoxy, oxo, halogen, amino, cyano, nitro, hydroxy, hydroxyalkyl, cycloalkyl, heterocyclyl, aryl and heteroaryl;
R⁹ and Rⁱ are identical or different and are each independently selected from the group consisting of a hydrogen atom, halogen, alkyl, haloalkyl, hydroxyalkyl, alkoxy, haloalkoxy, -(CH₂)ₛNR⁷R⁸, cycloalkyl, heterocyclyl, aryl and heteroaryl; wherein the alkyl, cycloalkyl, heterocyclyl, aryl and heteroaryl are each independently optionally substituted with one or more substituents selected from the group consisting of halogen, alkyl, alkoxy, haloalkyl, hydroxy, hydroxyalkyl, cyano, amino, nitro, cycloalkyl and heterocyclyl;
n is 1 or 2;
q is 0, 1, 2, 3 or 4;
s and y are identical or different and are each independently selected from the group consisting of 0, 1, 2, 3, 4 and 5; and
t and x are identical or different and are each independently selected from the group consisting of 0, 1 and 2.

2. The compound of general formula (I) or the tautomer, racemate, enantiomer or diastereomer thereof or the mixture thereof, or the pharmaceutically acceptable salt thereof according to claim 1, wherein R⁰ and R¹, together with the carbon to which they are attached, form a spiro 3-6 membered ring on the heterocycle to which they are attached, preferably form a spiro 3-6 membered carbocycle, and more preferably form a spirocyclopropyl.

3. The compound of general formula (I) or the tautomer, racemate, enantiomer or diastereomer thereof or the mixture thereof, or the pharmaceutically acceptable salt thereof according to claim 1 or 2, being a compound of general formula (II) or a tautomer, racemate, enantiomer or diastereomer thereof or a mixture thereof, or a pharmaceutically acceptable salt thereof: 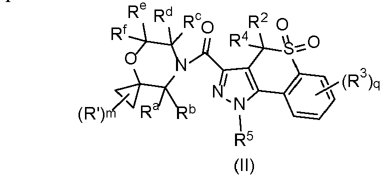
wherein: m is 0, 1, 2 or 3;
R', R^{a}-R^{f}, R²-R⁵ and q are as defined in claim 1.

4. The compound of general formula (I) or the tautomer, racemate, enantiomer or diastereomer thereof or the mixture thereof, or the pharmaceutically acceptable salt thereof according to any one of claims 1-3, wherein R⁵ is aryl or heteroaryl, and the aryl and heteroaryl are each independently optionally substituted with one or more substituents selected from the group consisting of halogen, alkyl, haloalkyl, nitro, cyano, hydroxyalkyl, -(CH₂)_{y}NR^{g}R^{h}, -OR⁹, -COR⁹, -COOR⁹, -OS(O)ₜR⁹, -S(O)ₜR⁹, -NR⁶COR⁹, -NR⁶SO₂R⁹ and R;
the R is selected from the group consisting of cycloalkyl, heterocyclyl, aryl, heteroaryl, cycloalkylalkyl, heterocyclylalkyl, arylalkyl and heteroarylalkyl, and the R are each independently optionally substituted with one or more substituents selected from the group consisting of halogen, alkyl, haloalkyl and-OR⁹;
R⁶, R⁹, R^{g}, R^{h}, y and t are as defined in claim 1.

5. The compound of general formula (I) or the tautomer, racemate, enantiomer or diastereomer thereof or the mixture thereof, or the pharmaceutically acceptable salt thereof according to any one of claims 1-4, wherein R⁵ is aryl or heteroaryl, and the aryl and heteroaryl are each independently optionally substituted with R, wherein the R is selected from the group consisting of cycloalkylalkyl, heterocyclylalkyl, arylalkyl and heteroarylalkyl, and the R are each independently optionally substituted with one or more substituents selected from the group consisting of halogen, alkyl and haloalkyl.

6. The compound of general formula (I) or the tautomer, racemate, enantiomer or diastereomer thereof or the mixture thereof, or the pharmaceutically acceptable salt thereof according to any one of claims 1-5, being a compound of general formula (III) or a tautomer, racemate, enantiomer or diastereomer thereof or a mixture thereof, or a pharmaceutically acceptable salt thereof: 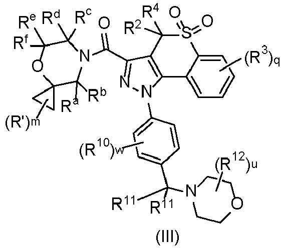 wherein:
R¹⁰ are identical or different and are each independently selected from the group consisting of a hydrogen atom, halogen, alkyl, haloalkyl, nitro, cyano, hydroxyalkyl, -(CH₂)ₛNR⁷R⁸, -OR⁹, cycloalkyl, heterocyclyl, aryl and heteroaryl, wherein the cycloalkyl, heterocyclyl, aryl and heteroaryl are each independently optionally substituted with one or more substituents selected from the group consisting of alkyl, haloalkyl, alkoxy, haloalkoxy, halogen, cyano, nitro and -(CH₂)_{y}NR^{g}R^{h};
R¹¹ are identical or different and are each independently selected from the group consisting of a hydrogen atom, halogen, alkyl, haloalkyl, alkoxy, haloalkoxy, hydroxy, hydroxyalkyl, cyano, nitro, -(CH₂)_{y}NR^{g}R^{h}, cycloalkyl, cycloalkyloxy and cycloalkylalkyl;
each R¹² is identical or different and is each independently selected from the group consisting of a hydrogen atom, halogen, alkyl, haloalkyl, nitro, cyano, hydroxyalkyl, -(CH₂)_{y}NR^{g}R^{h}, -OR⁹, cycloalkyl, cycloalkylalkyl, heterocyclyl, heterocyclylalkyl, aryl and heteroaryl; when u is greater than or equal to 2, two R¹² can form a spiro or bridged ring system on a morpholine ring;
w is 0, 1, 2, 3 or 4;
u is 0, 1, 2, 3, 4, 5 or 6;
R', R^{a}-R^{h}, R²-R⁴, R⁷-R⁹, s, m, y and q are as defined in claim 3.

7. The compound of general formula (I) or the tautomer, racemate, enantiomer or diastereomer thereof or the mixture thereof, or the pharmaceutically acceptable salt thereof according to any one of claims 1-6, wherein R² and R⁴ are identical or different and are each independently selected from the group consisting of a hydrogen atom, halogen and C₁₋₆ alkyl; preferably, R² and R⁴ are both hydrogen atoms.

8. The compound of general formula (I) or the tautomer, racemate, enantiomer or diastereomer thereof or the mixture thereof, or the pharmaceutically acceptable salt thereof according to any one of claims 1-7, wherein R³ are identical or different and are each independently selected from the group consisting of a hydrogen atom, halogen, C₁₋₆ haloalkyl, C₁₋₆ alkoxy and C₁₋₆ alkyl;
preferably, R³ are identical or different and are each independently selected from the group consisting of a hydrogen atom, halogen and C₁₋₆ alkyl.

9. The compound of general formula (I) or the tautomer, racemate, enantiomer or diastereomer thereof or the mixture thereof, or the pharmaceutically acceptable salt thereof according to any one of claims 6-8, wherein R¹⁰ are identical or different and are each independently selected from the group consisting of a hydrogen atom, halogen and C₁₋₆ alkyl; preferably, R¹⁰ is a hydrogen atom.

10. The compound of general formula (I) or the tautomer, racemate, enantiomer or diastereomer thereof or the mixture thereof, or the pharmaceutically acceptable salt thereof according to any one of claims 6-9, wherein R¹¹ are identical or different and are each independently selected from the group consisting of a hydrogen atom, halogen and C₁₋₆ alkyl; preferably, R¹¹ is a hydrogen atom.

11. The compound of general formula (I) or the tautomer, racemate, enantiomer or diastereomer thereof or the mixture thereof, or the pharmaceutically acceptable salt thereof according to any one of claims 6-10, wherein R¹² are identical or different and are each independently selected from the group consisting of a hydrogen atom, halogen and C₁₋₆ alkyl; preferably, R¹² is a hydrogen atom.

12. The compound of general formula (I) or the tautomer, racemate, enantiomer or diastereomer thereof or the mixture thereof, or the pharmaceutically acceptable salt thereof according to any one of claims 1-11, wherein R^{a}-R^{f} are each independently a hydrogen atom.

13. The compound of general formula (I) or the tautomer, racemate, enantiomer or diastereomer thereof or the mixture thereof, or the pharmaceutically acceptable salt thereof according to any one of claims 1-12, selected from the group consisting of the following compounds:

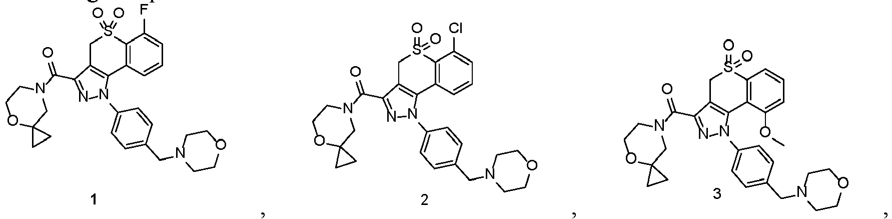

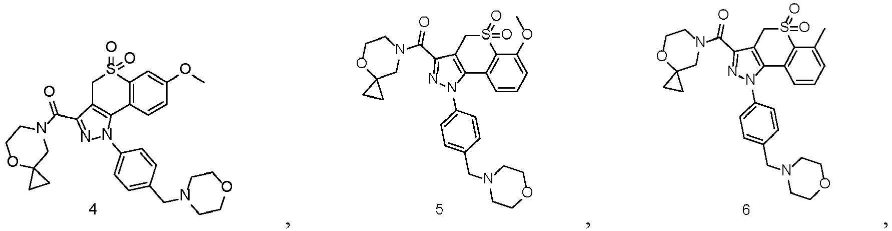

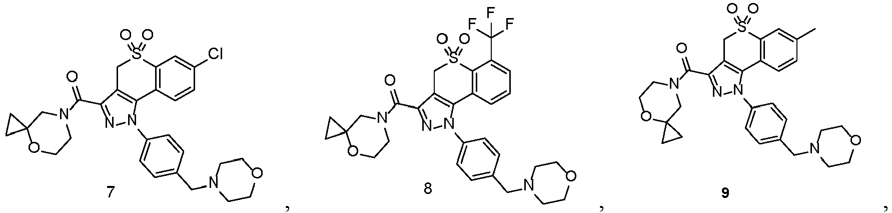

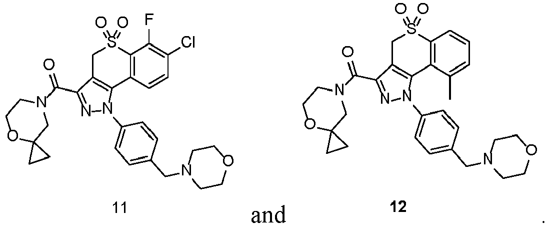

14. A method for preparing the compound of general formula (I) or the tautomer, racemate, enantiomer or diastereomer thereof or the mixture thereof, or the pharmaceutically acceptable salt thereof according to claim 1, comprising the following step: 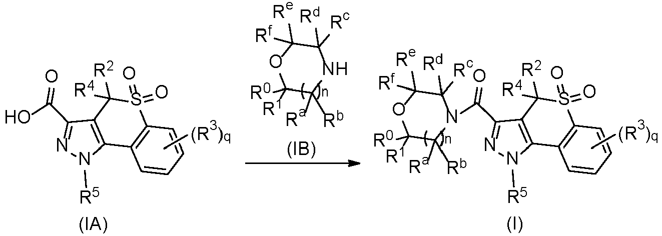
subjecting a compound of general formula (IA) or a tautomer, racemate, enantiomer or diastereomer thereof or a mixture thereof, or a pharmaceutically acceptable salt thereof to a reaction with a compound of general formula (IB) or a pharmaceutically acceptable salt thereof to give the compound of general formula (I) or the tautomer, racemate, enantiomer or diastereomer thereof or the mixture thereof, or the pharmaceutically acceptable salt thereof,
wherein R⁰-R⁵, R^{a}-R^{f}, n and q are as defined in claim 1.

15. A pharmaceutical composition, comprising the compound of general formula (I) or the tautomer, racemate, enantiomer or diastereomer thereof or the mixture thereof, or the pharmaceutically acceptable salt thereof according to any one of claims 1-13, and one or more pharmaceutically acceptable carriers, diluents or excipients.

16. Use of the compound of general formula (I) or the tautomer, racemate, enantiomer or diastereomer thereof or the mixture thereof, or the pharmaceutically acceptable salt thereof according to any one of claims 1-13, or the pharmaceutical composition according to claim 15, in preparing a medicament for inhibiting PI3Kδ.

17. Use of the compound of general formula (I) or the tautomer, racemate, enantiomer or diastereomer thereof or the mixture thereof, or the pharmaceutically acceptable salt thereof according to any one of claims 1-13, or the pharmaceutical composition according to claim 15, in preparing a medicament for treating and/or preventing inflammatory diseases, autoimmune diseases, cancer and related diseases, wherein: the cancer is preferably selected from the group consisting of melanoma, skin cancer, liver cancer, kidney cancer, lung cancer, nasopharyngeal cancer, gastric cancer, esophageal cancer, colorectal cancer, gallbladder cancer, bile duct cancer, chorionic epithelioma, pancreatic cancer, polycythemia vera, pediatric tumors, cervical cancer, ovarian cancer, breast cancer, bladder cancer, urothelial cancer, ureteral tumor, prostate cancer, seminoma, testicular tumor, leukemia, head and neck tumor, endometrial cancer, thyroid cancer, lymphoma, sarcoma, osteoma, neuroturbo chargeoma, neuroblastoma, neuroendocrine carcinoma, brain tumor, CNS cancer, myeloma, astrocytoma, glioblastoma and glioma, the leukemia is preferably selected from the group consisting of chronic lymphocytic leukemia, acute lymphocytic leukemia (ALL), acute myeloid leukemia (AML), chronic myeloid leukemia (CML) and hairy cell leukemia, the lymphoma is preferably selected from the group consisting of small lymphocytic lymphoma, marginal zone lymphoma, follicular lymphoma, mantle cell lymphoma, non-Hodgkin's lymphoma (NHL), lymphoplasmacytic lymphoma, nodal marginal zone lymphoma, T-cell lymphoma, B-cell lymphoma and diffuse large B-cell lymphoma, the lung cancer is preferably non-small cell lung cancer or small cell lung cancer, the myeloma is preferably multiple myeloma (MM), the autoimmune disease is preferably selected from the group consisting of asthma, rheumatoid arthritis, acute disseminated encephalomyelitis (ADEM), Addison's disease, alopecia areata, ankylosing spondylitis, antiphospholipid antibody syndrome (APS), autoimmune hemolytic anemia, autoimmune hepatitis, autoimmune inner ear disease, pemphigus, pemphigoid, Behcet's disease, celiac disease, anti-glutamine transaminase, Chagas' disease, chronic obstructive pulmonary disease, Crohn's disease, dermatomyositis, type 1 diabetes, endometriosis, Goodpasture's syndrome, Graves' disease, Guillain-Barre's syndrome (GBS), Hashimoto's disease, hidradenitis suppurativa, Kawasaki disease, IgA nephropathy, immune thrombocytopenic purpura, idiopathic thrombocytopenic purpura (ITP), interstitial cystitis, lupus, lupus nephritis, membranous nephropathy, mixed connective tissue disease, morphea, multiple sclerosis (MS), myasthenia gravis, narcolepsy, neuromyotonia, pernicious anemia, psoriasis, psoriatic arthritis, polymyositis, primary biliary cirrhosis, schizophrenia, scleroderma, dry eye and mouth syndrome, Sjogren's syndrome, stiffman syndrome, temporal arteritis, ulcerative colitis, vasculitis, vitiligo and Wegener's granulomatosis, the lupus is preferably lupus erythematosus or systemic lupus erythematosus, the pemphigus is preferably pemphigus vulgaris, the liver cancer is preferably hepatocellular carcinoma, the head and neck tumor is preferably head and neck squamous cell carcinoma, the sarcoma is preferably osteosarcoma or soft tissue sarcoma, and the colorectal cancer is preferably colon cancer or rectal cancer.
